(19) Europäisches Patentamt
European Patent Office
Office européen des brevets

(11) EP 0 738 726 B1

(12) **EUROPÄISCHE PATENTSCHRIFT**

(45) Veröffentlichungstag und Bekanntmachung des Hinweises auf die Patenterteilung:
**26.09.2001 Patentblatt 2001/39**

(21) Anmeldenummer: **96105539.9**

(22) Anmeldetag: **09.04.1996**

(51) Int Cl.[7]: **C07D 417/04**, A61K 31/425, C07D 413/04, C07D 263/58, C07D 419/04, C07F 9/6584, C07F 9/6558, C07D 417/14, C07D 413/06

(54) **Heteroatomhaltige Benzocyclopentanoxazolidinone mit antibakteriellen Wirkung**
Heterobenzocyclopentane oxazolidinones having antibacterial activity
Hétérobenzocyclopentane oxazolidinones ayant une activité antibactérienne

(84) Benannte Vertragsstaaten:
**AT BE CH DE DK ES FI FR GB GR IE IT LI LU MC NL PT SE**
Benannte Erstreckungsstaaten:
**LT LV SI**

(30) Priorität: **21.04.1995 DE 19514769**
**27.11.1995 DE 19544106**

(43) Veröffentlichungstag der Anmeldung:
**23.10.1996 Patentblatt 1996/43**

(73) Patentinhaber: **BAYER AG**
**51368 Leverkusen (DE)**

(72) Erfinder:
- **Stolle, Andreas, Dr.**
  **42115 Wuppertal (DE)**
- **Häbich, Dieter, Dr.**
  **42115 Wuppertal (DE)**
- **Bartel, Stephan, Dr.**
  **51465 Bergisch Gladbach (DE)**
- **Riedl, Bernd, Dr.**
  **42115 Wuppertal (DE)**
- **Ruppelt, Martin, Dr.**
  **42115 Wuppertal (DE)**
- **Wild, Hanno, Dr.**
  **Orange, Connecticut 06477 (US)**
- **Endermann, Rainer, Dr.**
  **42113 Wuppertal (DE)**
- **Bremm, Klaus-Dieter, Dr.**
  **45661 Recklinghausen (DE)**
- **Kroll, Hein-Peter, Dr.**
  **42115 Wuppertal (DE)**
- **Labischinski, Harald, Prof. Dr.**
  **42109 Wuppertal (DE)**
- **Schaller, Klaus, Dr.**
  **42109 Wuppertal (DE)**
- **Werling, Hans-Otto, Dr.**
  **42115 Wuppertal (DE)**

(56) Entgegenhaltungen:
**EP-A- 0 311 090** **EP-A- 0 609 441**
**EP-A- 0 609 905**

- **J. MED. CHEM., Bd. 35, 1992, Seiten 1156-1165, XP002009380 PARK ET AL.: "Antibacterials. Synthesis and ..."**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 738 726 B1

Printed by Jouve, 75001 PARIS (FR)

## Beschreibung

Die vorliegende Erfindung betrifft heteroatomhaltige Benzocyclopentanoxazolidinone, Verfahren zu ihrer Herstellung und ihre Verwendung als Arzneimittel, insbesondere als antibakterielle Arzneimittel.

Aus den Publikationen US 5 254 577, US 4 705 799, EP 311 090, US 4 801 600, US 4 921 869, US 4 965 268, EP 312 000 und C.H. Park et al., J. Med. Chem. 35, 1156 (1992) sind N-Aryloxazolidinone mit antibakterieller Wirkung bekannt. Außerdem sind 3-(Stickstoff-substituierte)phenyl-5-beta-amidomethyloxazolidin-2-one aus der EP 609 905 A1 bekannt.

Ferner werden in der PCT 93 08 179 A Oxazolidinonderivate mit einer Monoaminoxidase inhibitorischer Wirkung beschrieben.

Die vorliegende Erfindung betrifft heteroatomhaltige Benzocyclopentanoxazolidinone der allgemeinen Formel (I)

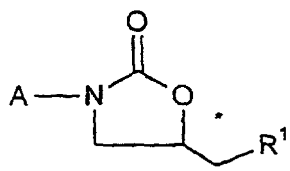

(I)

in welcher

$R^1$ für Azido, Hydroxy oder für eine Gruppe der Formel $-OR^2$, $O-SO_2R^3$ oder $-NR^4R^5$ steht,
worin

- $R^2$ geradkettiges oder verzweigtes Acyl mit bis zu 8 Kohlenstoffatomen oder eine Hydroxyschutzgruppe bedeutet,
- $R^3$ geradkettiges oder verzweigtes Alkyl mit bis zu 4 Kohlenstoffatomen oder Phenyl bedeutet, das gegebenenfalls durch geradkettiges oder verzweigtes Alkyl mit bis zu 4 Kohlenstoffatomen substituiert ist,
- $R^4$ und $R^5$ gleich oder verschieden sind und Cycloalkyl mit 3 bis 6 Kohlenstoffatomen, Wasserstoff, Phenyl oder geradkettiges oder verzweigtes Alkyl oder Alkoxy mit jeweils bis zu 8 Kohlenstoffatomen oder eine Aminoschutzgruppe bedeuten,
oder
- $R^4$ oder $R^5$ eine Gruppe der Formel $-CO-R^6$, $P(O)(OR^7)(OR^8)$ oder $-SO_2-R^9$ bedeutet,
worin

  - $R^6$ Cycloalkyl oder Halogen-substituiertes Cycloalkyl mit jeweils 3 bis 6 Kohlenstoffatomen, Trifluormethyl, geradkettiges oder verzweigtes Alkoxy mit bis zu 8 Kohlenstoffatomen, Phenyl, Benzyloxy oder Wasserstoff bedeutet, oder
  - $R^6$ geradkettiges oder verzweigtes Alkyl oder Alkenyl mit jeweils bis zu 8 Kohlenstoffatomen bedeutet, die gegebenenfalls durch Cyano, Halogen oder Trifluormethyl substituiert sind,
oder
geradkettiges oder verzweigtes Thioalkyl oder Acyl mit jeweils bis zu 6 Kohlenstoffatomen bedeutet,
oder
eine Gruppe der Formel $-NR^{10}R^{11}$ bedeutet,
worin

    - $R^{10}$ und $R^{11}$ gleich oder verschieden sind und Wasserstoff, Phenyl oder geradkettiges oder verzweigtes Alkyl mit bis zu 6 Kohlenstoffatomen bedeuten, oder

$R^6$ einen 5-gliedrigen aromatischen Heterocyclus mit bis zu 3 Heteroatomen aus der Reihe S, N und/oder O bedeutet, der gegebenenfalls durch geradkettiges oder verzweigtes Alkyl mit bis zu 3 Kohlenstoffatomen substituiert ist

$R^7$ und $R^8$ gleich oder verschieden sind und Wasserstoff oder geradkettiges oder verzweigtes Alkyl mit bis zu 4 Kohlenstoffatomen bedeuten,

$R^9$ geradkettiges oder verzweigtes Alkyl mit bis zu 4 Kohlenstoffatomen oder Phenyl bedeutet

A für einen Rest der Formel

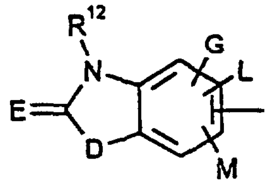

,

steht,
worin

G, L und M gleich oder verschieden sind und für Wasserstoff, Carboxy, Halogen, Cyano, Formyl, Trifluormethyl, Nitro, für geradkettiges oder verzweigtes Alkyl mit bis zu 6 Kohlenstoffatomen oder für eine Gruppe der Formel $-CO-NR^{17}R^{18}$ stehen,
worin

$R^{17}$ und $R^{18}$ gleich oder verschieden sind und Wasserstoff, geradkettiges oder verzweigtes Alkyl mit bis zu 4 Kohlenstoffatomen oder Phenyl bedeuten,

$R^{12}$ Wasserstoff, Cycloalkylcarbonyl oder Cycloalkyl mit jeweils 3 bis 6 Kohlenstoffatomen, oder geradkettiges oder verzweigtes Alkoxycarbonyl mit bis zu 6 Kohlenstoffatomen bedeutet, oder geradkettiges oder verzweigtes Alkyl oder Alkenyl mit jeweils bis zu 10 Kohlenstoffatomen bedeutet, das gegebenenfalls durch Cyano, Azido, Trifluormethyl, Pyridyl, Halogen, Hydroxy, Carboxyl, geradkettiges oder verzweigtes Alkoxycarbonyl mit bis zu 6 Kohlenstoffatomen, Benzyloxycarbonyl, Aryl mit 6 bis 10 Kohlenstoffatomen, Cycloalkyl mit 3 bis 6 Kohlenstoffatomen und/oder durch eine Gruppe der Formel $-(CO)_c-NR^{19}R^{20}$, $R^{21}-N-SO_2-R^{22}$, $R^{23}R^{24}-N-SO_2-$, $R^{25}-S(O)_d-$ oder

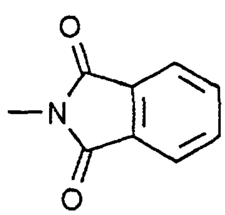

substituiert ist,
worin

c eine Zahl 0 oder 1 bedeutet,

$R^{19}$, $R^{20}$ und $R^{21}$ die oben angegebene Bedeutung von $R^{17}$ und $R^{18}$ haben und mit dieser gleich oder verschieden sind, oder gemeinsam mit dem Stickstoffatom einen 5- bis 6-gliedrigen, gesättigten Heterocyclus mit gegebenenfalls einem weiteren Heteroatom aus der Serie N, S und/oder O bilden, der seinerseits gegebenenfalls, auch an einem weiteren Stickstoffatom, durch geradkettiges oder verzweigtes Alkyl oder Acyl mit bis zu 3 Kohlenstoffatomen substituiert sein kann,

$R^{23}$ und $R^{24}$ die oben angegebene Bedeutung von $R^{17}$ und $R^{18}$ haben und mit dieser gleich oder verschieden sind,

d eine Zahl 0, 1 oder 2 bedeutet,

$R^{22}$ und $R^{25}$ gleich oder verschieden sind und geradkettiges oder verzweigtes Alkyl mit bis zu 4 Kohlenstoffatomen, Benzyl, Phenyl oder Tolyl bedeuten,
oder

$R^{12}$ einen Rest der Formeln

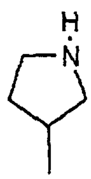 , 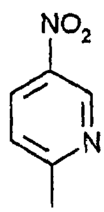 , 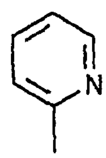 , 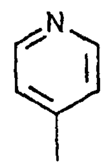 ,

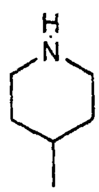 , 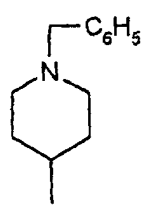 oder 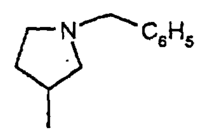

bedeutet oder
eine Gruppe der Formel $-COCCl_3$ oder geradkettiges oder verzweigtes Acyl mit bis zu 6 Kohlenstoffatomen bedeutet, das gegebenenfalls Trifluormethyl, Trichlormethyl oder durch eine Gruppe der Formel $-OR^{26}$ substituiert ist,
worin

$R^{26}$ Wasserstoff oder geradkettiges oder verzweigtes Alkyl mit bis zu 6 Kohlenstoffatomen bedeutet, das gegebenenfalls durch Aryl mit bis zu 10 Kohlenstoffatomen substituiert ist,
oder

$R^{12}$ eine Gruppe der Formel $-(CO)_c-NR^{27}R^{28}$, $-NR^{29}-SO_2R^{30}$, $R^{31}R^{32}-N-SO_2-$ oder $R^{33}-S(O)_f$ bedeutet,
worin

e die oben angegebene Bedeutung von c hat und mit dieser gleich oder verschieden ist,

$R^{27}$ und $R^{28}$ und $R^{29}$ jeweils die oben angegebene Bedeutung von $R^{19}$, $R^{20}$ und $R^{21}$ haben und mit dieser gleich oder verschieden sind,

$R^{31}$ und $R^{32}$ die oben angegebene Bedeutung von $R^{17}$ und $R^{18}$ haben und mit dieser gleich oder verschieden sind,

f die oben angegebene Bedeutung von d hat und mit dieser gleich oder verschieden ist,

$R^{30}$ und $R^{33}$ die jeweils oben angegebene Bedeutungen von $R^{22}$ und $R^{25}$ haben und mit dieser gleich oder verschieden sind,

D ein Sauerstoff oder Schwefelatom bedeutet,

E ein Sauerstoff- oder Schwefelatom oder eine Gruppe der Formel NH bedeutet,

oder im Fall, daß $R^{12}$ nicht für Wasserstoff steht, E eine Gruppe der Formel $NR^{34}$ bedeutet, worin $R^{34}$ mit

Ausnahme von Wasserstoff die oben angegebene Bedeutung von $R^{12}$ hat und mit dieser gleich oder verschieden ist,
oder

$R^{34}$ Cyano oder eine Gruppe der Formel $-CO_2R^{35}$ bedeutet,
worin

$R^{35}$ Benzyl oder Phenyl bedeutet, die gegebenenfalls durch Nitro oder Halogen substituiert sind,

mit der Maßgabe, daß $R^1$ nicht für Hydroxy steht, wenn E NH bedeutet und gleichzeitig $R^{12}$ wasserstoff bedeutet, und deren tautomere Formen, Isomere und Salze.

Tautomerie der erfindungsgemäßen Verbindungen bezieht sich in Abhängigkeit der oben aufgeführten Substituentendefinitionen von E, T, $R^{12}$, $R^{13}$ und $R^{14}$ auf die Möglichkeit der Verlagerung der exocyclischen Doppelbindungen in den 5-gliedrigen Heterocyclus.

Physiologisch unbedenkliche Salze der heteroatomhaltigen Benzocyclopentanoxazolidinone können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Als Salze können Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabiethylamin, 1-Ephenamin oder Methyl-piperidin.

Als Salze können außerdem Reaktionsprodukte mit $C_1$-$C_4$-Alkylhalogenide, insbesondere $C_1$-$C_4$-Alkyljodide fungieren.

Heteroyclus steht im allgemeinen für einen 5- bis 6-gliedrigen, gesättigten oder ungesättigten Ring, der als Heteroatome bis zu 3 Sauerstoff-, Schwefel- und/oder Stickstoffatome enthalten kann. Bevorzugt werden genannt: Thienyl, Furyl, Pyrrolyl, Pyrazolyl, Pyridyl, Pyrimidyl, Pyrazinyl, Pyridazinyl, Thiazolyl, Oxazolyl, Imidazolyl, Pyrrolidinyl, Piperidinyl oder Piperazinyl.

Dazu gehören auch über N-gebundene, 5- bis 6-gliedrige gesättigte Heterocyclen, die außerdem als Heteroatome bis zu 2 Sauerstoff-, Schwefel- und/oder Stickstoffatome enthalten können, wie beispielsweise Piperidyl, Morpholinyl oder Piperazin oder Pyrrolidinyl. Besonders bevorzugt sind Piperidyl, Morphlinyl und Pyrrolidinyl.

Hydroxyschutzgruppe im Rahmen der oben angegebenen Definition steht im allgemeinen für eine Schutzgruppe aus der Reihe: Trimethylsilyl, Triisopropylsilyl, tert. Butyl-dimethylsilyl, Benzyl, Benzyloxycarbonyl, 2-Nitrobenzyl, 4-Nitrobenzyl, tert. Butyloxycarbonyl, Allyloxycarbonyl, 4-Methoxybenzyl, 4-Methoxybenzyloxycarbonyl, Tetrahydropyranyl, Formyl, Acetyl, Trichloracetyl, 2,2,2-Trichlorethoxycarbonyl, Methoxyethoxymethyl, [2-(Trimethylsilyl) ethoxy]methyl, Benzoyl, 4-Methylbenzoyl, 4-Nitrobenzoyl, 4-Fluorbenzoyl, 4-Chlorbenzoyl oder 4-Methoxybenzoyl. Bevorzugt sind Acetyl, tert. Butyldimethylsilyl oder Tetrahydropyranyl.

Aminoschutzgruppe im Rahmen der Erfindung sind die üblichen in der Peptid-Chemie verwendeten Aminoschutzgruppen.

Hierzu gehören bevorzugt: Benzyloxycarbonyl, 2,4-Dimethoxybenzyloxycarbonyl, 4-Methoxybenzyloxycarbonyl, Methoxycarbonyl, Ethoxycarbonyl, tert.Butoxycarbonyl, Allyloxycarbonyl, Phthaloyl, 2,2,2-Trichlorethoxycarbonyl, Fluorenyl-9-methoxycarbonyl, Formyl, Acetyl, 2-Chloracetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl, 4-Nitrophenyl, 4-Methoxyphenyl oder Triphenylmethyl.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Die Racemformen lassen sich ebenso wie die Diastereomeren in bekannter Weise in die stereoisomer einheitlichen Bestandteile trennen

Bevorzugt sind Verbindungen der allgemeinen Formel (I),
in welcher

$R^1$ für Azido, Hydroxy oder für eine Gruppe der Formel $-OR^2$, $O-SO_2R^3$ oder $-NR^4R^5$ steht,
worin

$R^2$ geradkettiges oder verzweigtes Acyl mit bis zu 6 Kohlenstoffatomen oder Benzyl bedeutet,

$R^3$ geradkettiges oder verzweigtes Alkyl mit bis zu 3 Kohlenstoffatomen, Phenyl oder Toluolyl bedeutet

$R^4$ und $R^5$ gleich oder verschieden sind und Cyclopropyl, Cyclobutyl, Cyclopentyl, Cyclohexyl, Wasserstoff, Phenyl oder geradkettiges oder verzweigtes Alkyl oder Alkoxy mit jeweils bis zu 6 Kohlenstoffatomen, tert.Butoxycarbonyl oder Benzyloxycarbonyl bedeuten,
oder

$R^4$ oder $R^5$ eine Gruppe der Formel $-CO-R^6$, $P(O)(OR^7)(OR^8)$ oder $-SO_2-R^9$ bedeutet,
worin

$R^6$ Cyclopropyl, Fluor-substituiertes Cyclpropyl, Cyclobutyl, Cyclopentyl, Cyclohexyl, Trifluormethyl oder geradkettiges oder verzweigtes Alkoxy mit bis zu 6 Kohlenstoffatomen, Phenyl, Benzyloxy oder Wasserstoff bedeutet, oder

$R^6$ geradkettiges oder verzweigtes Alkyl oder Alkenyl mit jeweils bis zu 6 Kohlenstoffatomen bedeutet, die gegebenenfalls durch Cyano, Fluor, Chlor, Brom oder Trifluormethyl substituiert sind, oder
geradkettiges oder verzweigtes Thioalkyl oder Acyl mit jeweils bis zu 5 Kohlenstoffatomen bedeutet, oder
eine Gruppe der Formel $-NR^{10}R^{11}$ bedeutet,
worin

$R^{10}$ und $R^{11}$ gleich oder verschieden sind und Wasserstoff, Phenyl oder geradkettiges oder verzweigtes Alkyl mit bis zu 4 Kohlenstoffatomen bedeuten, oder

$R^6$ Isoxazolyl, Furyl, Thienyl, Pyrryl, Oxazolyl oder Imidazolyl bedeutet, die gegebenenfalls durch Methyl substituiert sind

$R^7$ und $R^8$ gleich oder verschieden sind und Wasserstoff oder geradkettiges oder verzweigtes Alkyl mit bis zu 3 Kohlenstoffatomen bedeuten,

$R^9$ geradkettiges oder verzweigtes Alkyl mit bis zu 3 Kohlenstoffatomen oder Phenyl bedeutet,

A für einen Rest der Formel

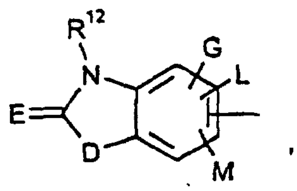

steht,
worin

G, L und M gleich oder verschieden sind und für Wasserstoff, Carboxy, Fluor, Chlor, Brom, Jod, Cyano, Trifluormethyl, Formyl, Nitro, für geradkettiges oder verzweigtes Alkyl mit bis zu 4 Kohlenstoffatomen oder für eine Gruppe der Formel $-CO-NR^{17}R^{18}$ stehen,
worin

$R^{17}$ und $R^{18}$ gleich oder verschieden sind und Wasserstoff, geradkettiges oder verzweigtes Alkyl mit bis zu 3 Kohlenstoffatomen oder Phenyl bedeuten,

$R^{12}$ Wasserstoff, Cyclopropyl carbonyl, Cyclopropyl, Cyclobutyl, Cyclopentyl, Cyclohexyl, oder
geradkettiges oder verzweigtes Alkoxycarbonyl mit bis zu 4 Kohlenstoffatomen, oder geradkettiges oder verzweigtes Alkyl oder Alkenyl mit jeweils bis zu 9 Kohlenstoffatomen bedeutet, die gegebenenfalls durch Cyano, Azido, Trifluormethyl, Pyridyl, Fluor, Chlor, Brom, Hydroxy, Phenyl, Carboxyl, geradkettiges oder verzweigtes Alkoxycarbonyl mit bis zu 5 Kohlenstoffatomen, Benzyloxycarbonyl, Naphthyl, Cyclopropyl, Cyclopentyl, Cyclohexyl und/oder durch eine Gruppe der Formel $-(CO)_c-NR^{19}R^{20}$, $R^{21}-N-SO_2-R^{22}$, $R^{23}R^{24}-N-SO_2-$, $R^{25}-S(O)_d-$ oder

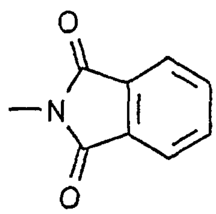

substituiert ist,
worin

c eine Zahl 0 oder 1 bedeutet,

$R^{19}$, $R^{20}$ und $R^{21}$ die oben angegebene Bedeutung von $R^{17}$ und $R^{18}$ haben und mit dieser gleich oder verschieden sind, oder gemeinsam mit dem Stickstoffatom einen Morpholinyl-, Pyrrolidinyl-, Piperazinyl- oder Piperidylring bilden, die gegebenenfalls, auch über die freie N-Funktion, durch Methyl, Ethyl oder Acetyl substituiert sind,

$R^{23}$ und $R^{24}$ die oben angegebene Bedeutung von $R^{17}$ und $R^{18}$ haben und mit dieser gleich oder verschieden sind,

d eine Zahl 0, 1 oder 2 bedeutet,

$R^{22}$ und $R^{25}$ gleich oder verschieden sind und geradkettiges oder verzweigtes Alkyl mit bis zu 3 Kohlenstoffatomen, Benzyl, Phenyl oder Tolyl bedeuten, oder

$R^{12}$ einen Rest der Formeln

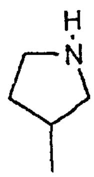 , 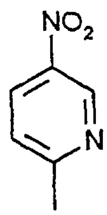 , 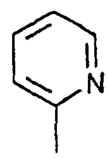 , 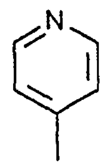 ,

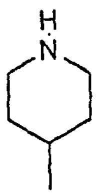 , 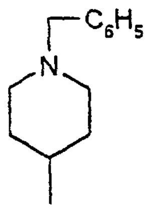 oder 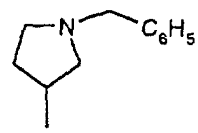

bedeutet oder
eine Gruppe der Formel $-COCCl_3$ oder geradkettiges oder verzweigtes Acyl mit bis zu 5 Kohlenstoffatomen bedeutet, das gegebenenfalls durch Trifluormethyl, Trichlormethyl oder eine Gruppe der Formel $-OR^{26}$ substituiert ist,
worin

$R^{26}$ Wasserstoff oder geradkettiges oder verzweigtes Alkyl mit bis zu 5 Kohlenstoffatomen bedeutet, das gegebenenfalls durch Phenyl oder Naphthyl substituiert ist, oder

$R^{12}$ eine Gruppe der Formel $-(CO)_e-NR^{27}R^{28}$, $-NR^{29}-SO_2R^{30}$, $R^{31}R^{32}-N-SO_2-$ oder $R^{33}-S(O)_f$ bedeutet, worin

e die oben angegebene Bedeutung von c hat und mit dieser gleich oder verschieden ist,

$R^{27}$, $R^{28}$ und $R^{29}$ die jeweils oben angegebene Bedeutung von $R^{19}$, $R^{20}$ und $R^{21}$ haben und mit dieser gleich oder verschieden sind,

$R^{31}$ und $R^{32}$ die oben angegebene Bedeutung von $R^{17}$ und $R^{18}$ haben und mit dieser gleich oder verschieden sind,

f die oben angegebene Bedeutung von d hat und mit dieser gleich oder verschieden ist,

$R^{30}$ und $R^{33}$ die jeweils oben angegebenen Bedeutungen von $R^{22}$ und $R^{25}$ haben und mit dieser gleich oder verschieden sind,

D ein Sauerstoff oder Schwefelatom bedeutet,

E ein Sauerstoff- oder Schwefelatom oder eine Gruppe der Formel NH bedeutet,

oder im Fall, daß $R^{12}$ nicht für Wasserstoff stehet, E eine Gruppe der Formel $NR^{34}$ bedeutet, worin $R^{34}$ mit Ausnahme von Wasserstoff die oben angegebene Bedeutung von $R^{12}$ hat und mit dieser gleich oder verschieden ist, oder

$R^{34}$ Cyano oder eine Gruppe der Formel $-CO_2R^{35}$ bedeutet, worin

$R^{35}$ Benzyl oder Phenyl bedeutet, die gegebenenfalls durch Nitro, Fluor, Chlor oder Brom substituiert sind,

mit der maßgabe, daß $R^1$ nicht für Hydroxy steht, wenn E NH bedeutet und gleichzeitig $R^{12}$ Wasserstoff bedeutet, und deren tautomeren Formen und Salze.

Besonders bevorzugt sind Verbindungen der allgemeinen Formel (I), in welcher

$R^1$ für Azido, Hydroxy oder für eine Gruppe der Formel $-OR^2$, $O-SO_2R^3$ oder $-NR^4R^5$ steht, worin

$R^2$ geradkettiges oder verzweigtes Acyl mit bis zu 5 Kohlenstoffatomen oder Benzyl bedeutet,

$R^3$ Methyl, Ethyl, Phenyl oder Toluolyl bedeutet,

$R^4$ und $R^5$ gleich oder verschieden sind und Cyclopropyl, Cyclobutyl, Cyclopentyl, Cyclohexyl, Wasserstoff, Phenyl oder geradkettiges oder verzweigtes Alkyl oder Alkoxy mit jeweils bis zu 5 Kohlenstoffatomen, tert.Butoxycarbonyl oder Benzyloxycarbonyl bedeuten, oder

$R^4$ oder $R^5$ eine Gruppe der Formel $-CO-R^6$, $P(O)(OR^7)(OR^8)$ oder $-SO_2R^9$ bedeutet, worin

$R^6$ Cyclopropyl, Fluor-substituiertes Cyclopropyl, Cyclobutyl, Cyclopentyl, Cyclohexyl, Trifluormethyl oder geradkettiges oder verzweigtes Alkoxy mit bis zu 5 Kohlenstoffatomen, Phenyl, Benzyloxy oder Wasserstoff bedeutet,

$R^6$ geradkettiges oder verzweigtes Alkyl oder Alkenyl mit jeweils bis zu 5 Kohlenstoffatomen bedeutet, die gegebenenfalls durch Cyano, Fluor, Chlor, Brom oder Trifluormethyl substituiert sind, oder
geradkettiges oder verzweigtes Thioalkyl- oder Acyl mit jeweils bis zu 4 Kohlenstoffatomen bedeutet, oder
eine Gruppe der Formel $-NR^{10}R^{11}$ bedeutet, worin

$R^{10}$ und $R^{11}$ gleich oder verschieden sind und Wasserstoff, Phenyl oder geradkettiges oder verzweigtes Alkyl mit bis zu 3 Kohlenstoffatomen bedeuten, oder

$R^6$ Isoxazolyl, Furyl, Oxazolyl oder Imidazolyl bedeutet, die gegebenenfalls durch Methyl substituiert sind

$R^7$ und $R^8$ gleich oder verschieden sind und Wasserstoff, Methyl oder Ethyl bedeuten,

$R^9$ Methyl oder Phenyl bedeutet,

A für einen Rest der Formel

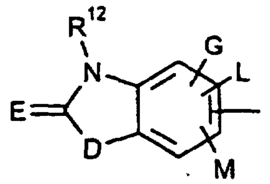

steht,
worin

G, L und M gleich oder verschieden sind und für Wasserstoff, Carboxy, Fluor, Chlor, Brom, Jod, Cyano, Formyl, Nitro, für geradkettiges oder verzweigtes Alkyl mit bis zu 3 Kohlenstoffatomen oder für eine Gruppe $-CO-NH_2$ stehen,

$R^{12}$ Wasserstoff, Cyclopropylcarbonyl, Cyclopropyl, Cyclobutyl, Cyclopentyl, Cyclohexyl, oder
geradkettiges oder verzweigtes Alkoxycarbonyl mit bis zu 3 Kohlenstoffatomen, oder geradkettiges oder verzweigtes Alkyl oder Alkenyl mit jeweils bis zu 8 Kohlenstoffatomen bedeutet, die gegebenenfalls durch Cyano, Azido, Trifluormethyl, Pyridyl, Fluor, Chlor, Brom, Phenyl, Hydroxy, Carboxyl, geradkettiges oder verzweigtes Alkoxycarbonyl mit bis zu 4 Kohlenstoffatomen, Benzyloxycarbonyl, Cyclopropyl, Cyclopentyl, Cyclohexyl und/oder durch eine Gruppe der Formel-$(CO)_c$-$NR^{19}R^{20}$, $R^{21}$-N-$SO_2$-$R^{22}$, $R^{23}R^{24}$-N-$SO_2$-, $R^{25}$-$S(O)_d$- oder

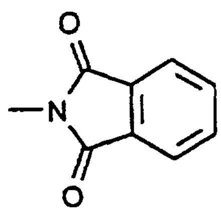

substituiert sind,
worin

c eine Zahl 0 oder 1 bedeutet,

$R^{19}$, $R^{20}$, $R^{21}$, $R^{23}$ und $R^{24}$ gleich oder verschieden sind und Wasserstoff, Methyl oder Ethyl bedeuten,

d eine Zahl 0, 1 oder 2 bedeutet,

$R^{22}$ und $R^{25}$ gleich oder verschieden sind und geradkettiges oder verzweigtes Alkyl mit bis zu 4 Kohlenstoffatomen, Benzyl, Phenyl oder Tolyl bedeuten, oder

$R^{12}$ einen Rest der Formeln

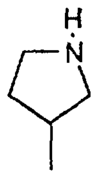 , 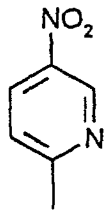 , 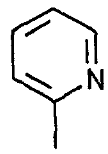 , 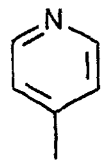 ,

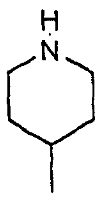 , 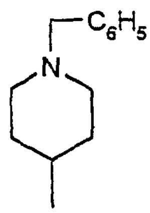 oder 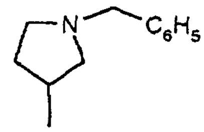

bedeutet oder
eine Gruppe der Formel $-COCCl_3$ oder geradkettiges oder verzweigtes Acyl mit bis zu 4 Kohlenstoffatomen bedeutet, das gegebenenfalls durch Trifluormethyl, Trichlormethyl, eine Gruppe der Formel $-OR^{26}$ substituiert ist,
worin

$R^{26}$ Wasserstoff oder geradkettiges oder verzweigtes Alkyl mit bis zu 4 Kohlenstoffatomen bedeutet, das gegebenenfalls durch Phenyl substituiert ist,
oder

$R^{12}$ eine Gruppe der Formel $-(CO)_e-NR^{27}R^{28}$ oder $R^{33}-S(O)_f$ bedeutet,
worin

e die Zahl 1 bedeutet,

$R^{27}$ und $R^{28}$ gleich oder verschieden sind und Wasserstoff, Methyl oder Ethyl bedeuten,

f die oben angegebene Bedeutung von d hat und mit dieser gleich oder verschieden ist,

$R^{33}$ Methyl, Phenyl, Tolyl oder Benzyl bedeutet,

D ein Sauerstoff oder Schwefelatom bedeutet,

E ein Sauerstoff- oder Schwefelatom oder eine Gruppe der Formel NH bedeutet,

oder im Fall, daß $R^{12}$ nicht für Wasserstoff steht, E eine Gruppe der Formel $NR^{34}$ bedeutet, worin $R^{34}$ mit Ausnahme von Wasserstoff die oben angegebene Bedeutung von $R^{12}$ hat und mit dieser gleich oder verschieden ist, oder

$R^{34}$ Cyano oder eine Gruppe der Formel $-CO_2R^{35}$ bedeutet,
worin

$R^{35}$ Benzyl oder Phenyl bedeutet, die gegebenenfalls durch Nitro substituiert sind,

mit der maßgabe, daß $R^1$ nicht für Hydroxy steht, wenn E NH bedeutet und gleichzeitig $R^{12}$ Wasserstoff bedeutet, und deren tautomeren Formen und Salze.

Ganz besonders bevorzugt sind Verbindungen der allgemeinen Formel (I), in welcher

Außerdem wurden Verfahren zur Herstellung der erfindungsgemäßen Verbindungen der allgemeinen Formel (I) gefunden, dadurch gekennzeichnet, daß man

[A] Verbindungen der allgemeinen Formeln (II) oder (III)

$$A\text{-}N{=}C{=}O \quad (II)$$

oder

$$A\text{-}CO\text{-}N_3 \quad (III)$$

in welchen

A die oben angegebene Bedeutungen hat,

mit Lithiumbromid/$(C_4H_9)_3$ P(O) und Epoxiden der allgemeinen Formel (IV)

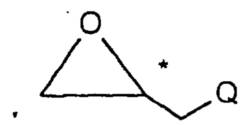

(IV)

in welcher

Q für $C_1$-$C_6$-Acyloxy steht,

in inerten Lösemitteln, gegebenenfalls in Anwesenheit einer Base umsetzt,
und im Fall $R^1$ = OH durch eine typische Esterverseifung oder durch eine typische Umesterung die Hydroxyfunktion freisetzt,
oder

[B] Verbindungen der allgemeinen Formel (V)

$$A\text{-}NH\text{-}CO_2\text{-}X \quad (V)$$

in welcher

A die oben angegebene Bedeutung hat und

X für eine typische Schutzgruppe, vorzugsweise Benzyl steht,

in inerten Lösemitteln und in Anwesenheit einer Base, beispielsweise Lithiumalkylen oder Lithium-N-alkyl- oder Lithium-N-silylalkylamiden, vorzugsweise N-Butyllithium, mit Epoxiden der allgemeinen Formel (IV) umsetzt,
oder

[C] im Fall $R^1$ = OH, zunächst Verbindungen der allgemeinen Formel (III) durch Abspaltung von Stickstoff in Alkoholen in die Verbindungen der allgemeinen Formel (Va)

$$A\text{-}NH\text{-}CO_2\text{-}Y \qquad (Va)$$

in welcher

A die oben angegebene Bedeutung hat und

Y für geradkettiges oder verzweigtes $C_2$-$C_6$-Alkyl, vorzugsweise n-Butyl steht,

überführt,
und in einem zweiten Schritt wie unter [A] beschrieben in inerten Lösemitteln und in Anwesenheit einer Base, vorzugsweise Lithium-N-alkyl-oder N-Silylalkylamiden oder n-Butyllithium und Epoxiden der allgemeinen Formel (IV) umsetzt,
oder

[D] Verbindungen der allgemeinen Formel (VI)

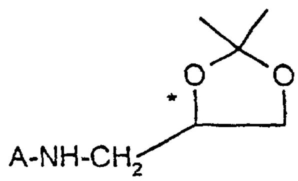

(VI)

in welcher

A die oben angegebene Bedeutung hat,

entweder direkt mit Säuren und Kohlensäurediethylester umsetzt,
oder zunächst durch Umsetzung der Verbindungen der allgemeinen Formel (VI) mit Säuren die Verbindungen der allgemeinen Formel (VII)

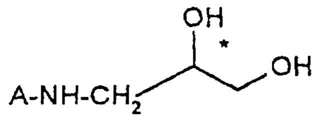

(VII)

in welcher

A die oben angegebene Bedeutung hat,

herstellt,
und anschließend in Anwesenheit eines Hilfsmittels in inerten Lösemitteln cyclisiert,
oder

[E] zunächst Verbindungen der allgemeinen Formel (Ia)

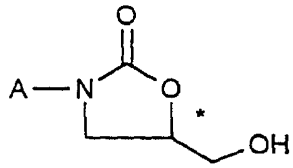

(Ia)

in welcher

A die oben angegebene Bedeutung hat,

durch Umsetzung mit ($C_1$-$C_4$)-Alkyl- oder Phenylsulfonsäurechloriden in inerten Lösemitteln und in Anwesenheit einer Base in die entsprechenden Verbindungen der allgemeinen Formel (Ib)

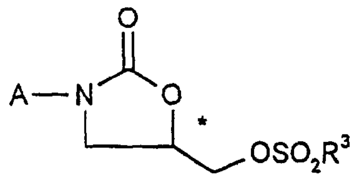

(Ib)

in welcher

A und $R^3$ die oben angegebene Bedeutung haben,

überführt,
anschließend mit Natriumazid in inerten Lösemitteln die Azide der allgemeinen Formel (Ic)

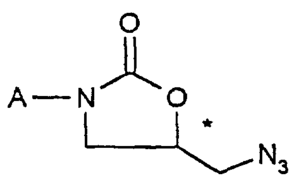

(Ic)

in welcher

A die oben angegebene Bedeutung hat,

herstellt, in einem weiteren Schritt durch Umsetzung mit ($C_1$-$C_4$-O)$_3$-P oder $PPh_3$, vorzugsweise $(CH_3O)_3P$ in inerten Lösemitteln und mit Säuren in die Amine der allgemeinen Formel (Id)

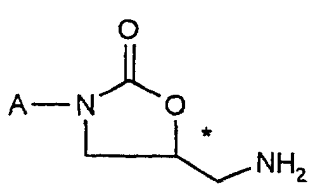

(Id)

in welcher

A die oben angegebene Bedeutung hat,

überführt,
und durch Umsetzung mit Acetanhydrid oder anderen Acylierungsmitteln der allgemeinen Formel (VIII)

$$R^{36}\text{-CO-}R^{6} \qquad \text{(VIII)}$$

in welcher

$R^6$ die oben angegebene Bedeutung hat

und

$R^{36}$ für Halogen, vorzugsweise für Chlor oder für den Rest -OCOR$^6$ steht,

in inerten Lösemitteln die Verbindungen der allgemeinen Formel (Ie)

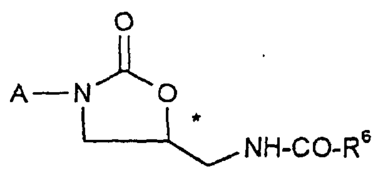

(Ie)

in welcher

A und $R^6$ die oben angegebene Bedeutung haben,

herstellt,
oder

[F] im Fall

A = 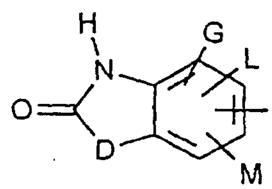 oder 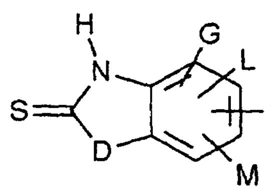

Verbindungen der allgemeinen Formel (IX)

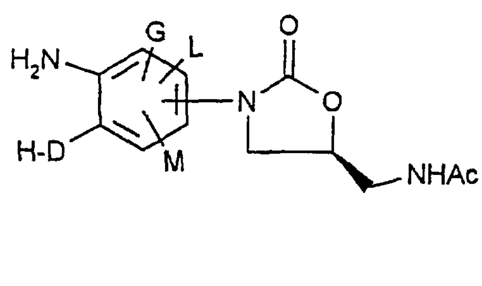

(IX)

in welcher

G, L, M und D die oben angegebene Bedeutung haben,

entweder mit Carbonyldiimidazol bzw. Thiocarbonyldiimidazol in Dimethylformamid oder durch Umsetzung mit KS-

$CO_2$-$C_2H_5$ / $CH_3OH$ und anschließender Zugabe von Wasser cyclisiert, im Fall

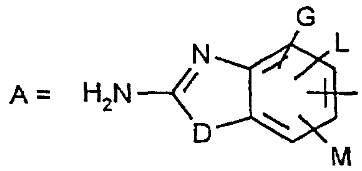

die Verbindungen der allgemeinen Formel (IX) mit BrCN / $H_2O$ / $CH_3OH$ umsetzt, oder

[G] im Fall $R^{12} \neq H$, ausgehend von den Verbindungen mit $R^1$ = NH-$COCH_3$ eine Acylierung oder eine Alkylierung unter Doppelbindungsverschiebung durchführt, oder Verbindungen der allgemeinen Formel (I) mit dem Rest

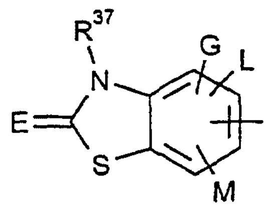

worin

$R^{37}$ $C_1$-$C_{10}$-Alkyl, vorzugsweise $C_1$-$C_3$-Alkyl bedeutet

und E = O, Verbindungen der allgemeinen Formel (X)

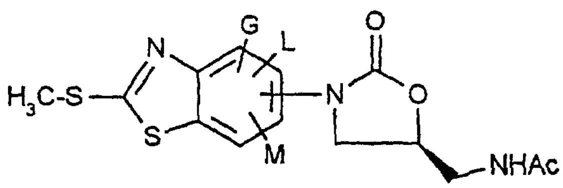

(X)

in welcher

G, L und M die oben angegebene Bedeutung haben,

zunächst durch Umsetzung mit $C_1$-$C_{10}$-Alkylhalogeniden, bevorzugt $C_1$-$C_3$-Alkyl-jodiden, in inerten Lösemitteln in die Salze der Verbindungen der allgemeinen Formel (XI)

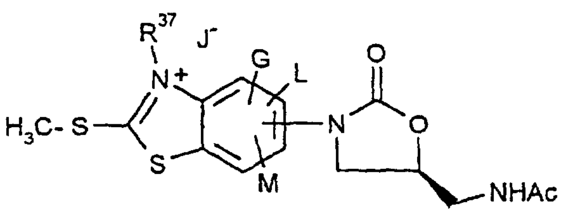

(XI)

in welcher

| | |
|---|---|
| $R^{37}$ | für $C_1$-$C_{10}$-Alkyl, vorzugsweise für $C_1$-$C_3$-Alkyl steht, und |
| G, L und M | die oben angegebene Bedeutung haben, |

überführt,
und in einem letzten Schritt mit Methanol zur Reaktion bringt,
und im Fall E = Verbindungen der allgemeinen Formel (XI) einer Thermolyse unterzieht,
und im Fall der S-Oxide eine Oxidation nach üblicher Methode durchführt,
und gegebenenfalls weitere Substituenten oder bereits vorhandene funktionelle Gruppen nach üblichen Methoden, wie beispielsweise Alkylierung, Redoxreaktionen, Substitutionsreaktionen und/oder Verseifungen oder Ein- und Abbau von Schutzgruppen, einführt bzw. derivatisiert.

Die erfindungsgemäßen Verfahren können durch folgende Formelschemata beispielhaft erläutert werden:

[A]

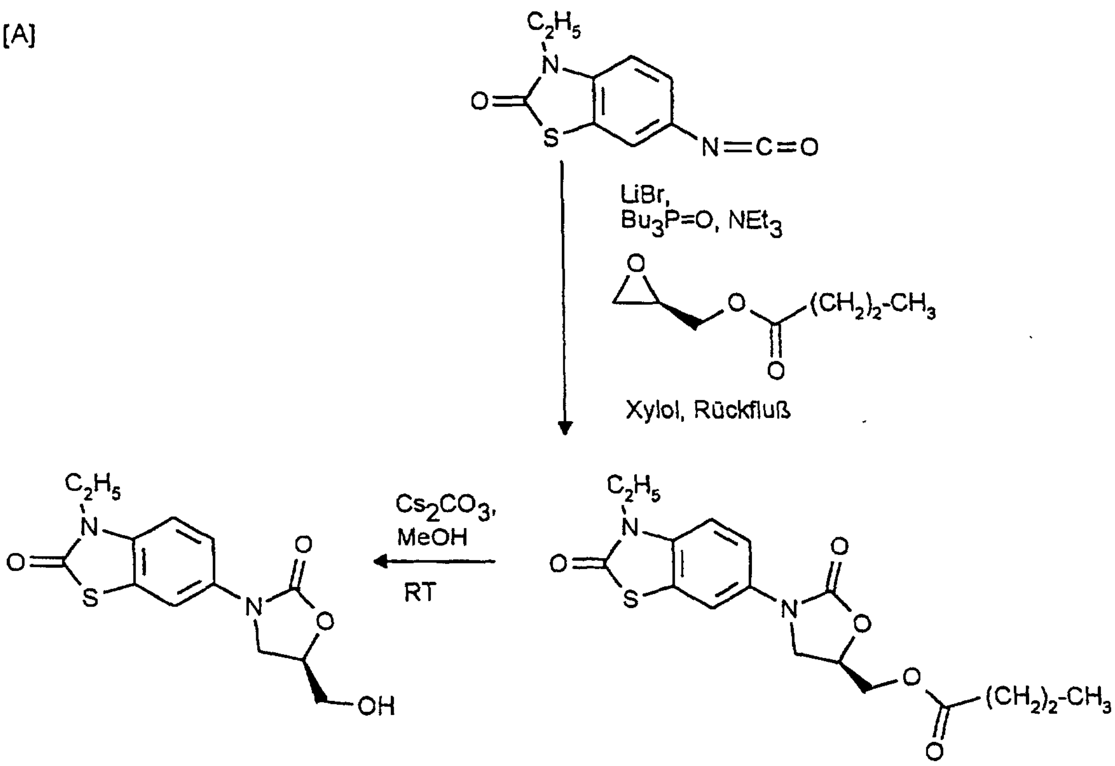

[B]

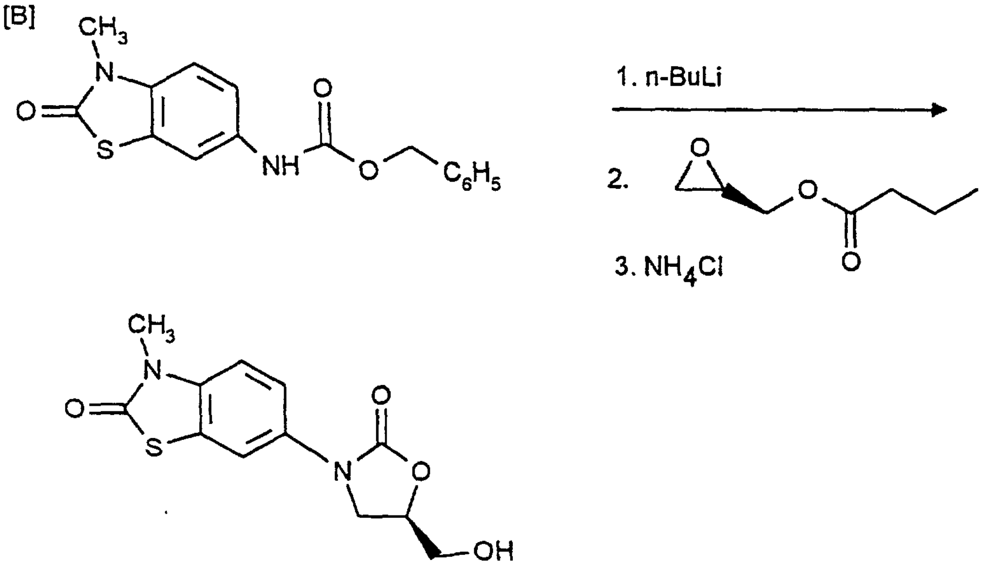


[C]

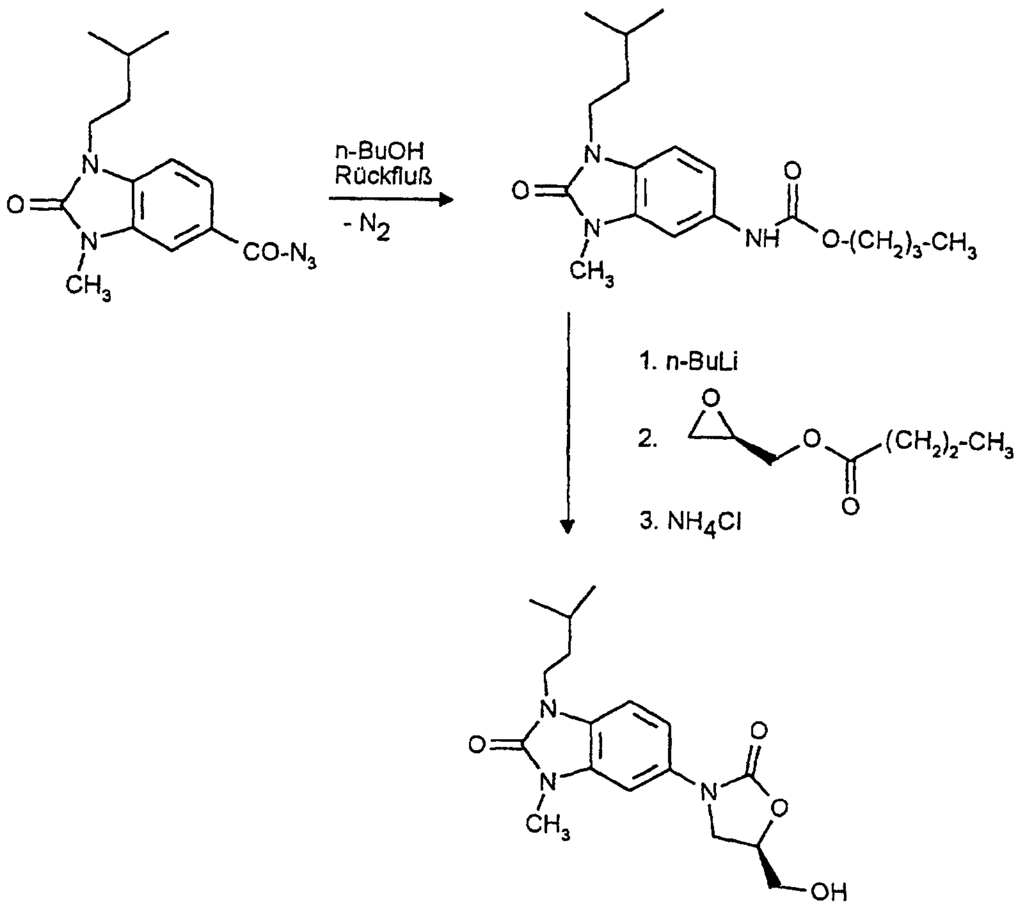

[D]

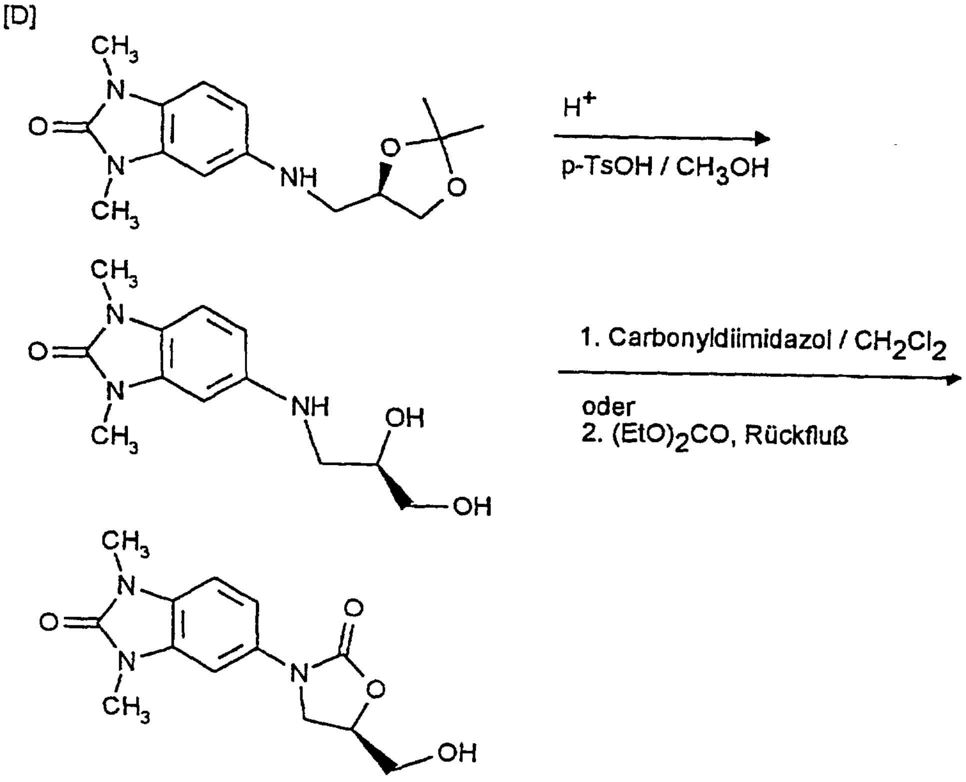
H+
p-TsOH / CH3OH
1. Carbonyldiimidazol / CH2Cl2
oder
2. (EtO)2CO, Rückfluß

[E]

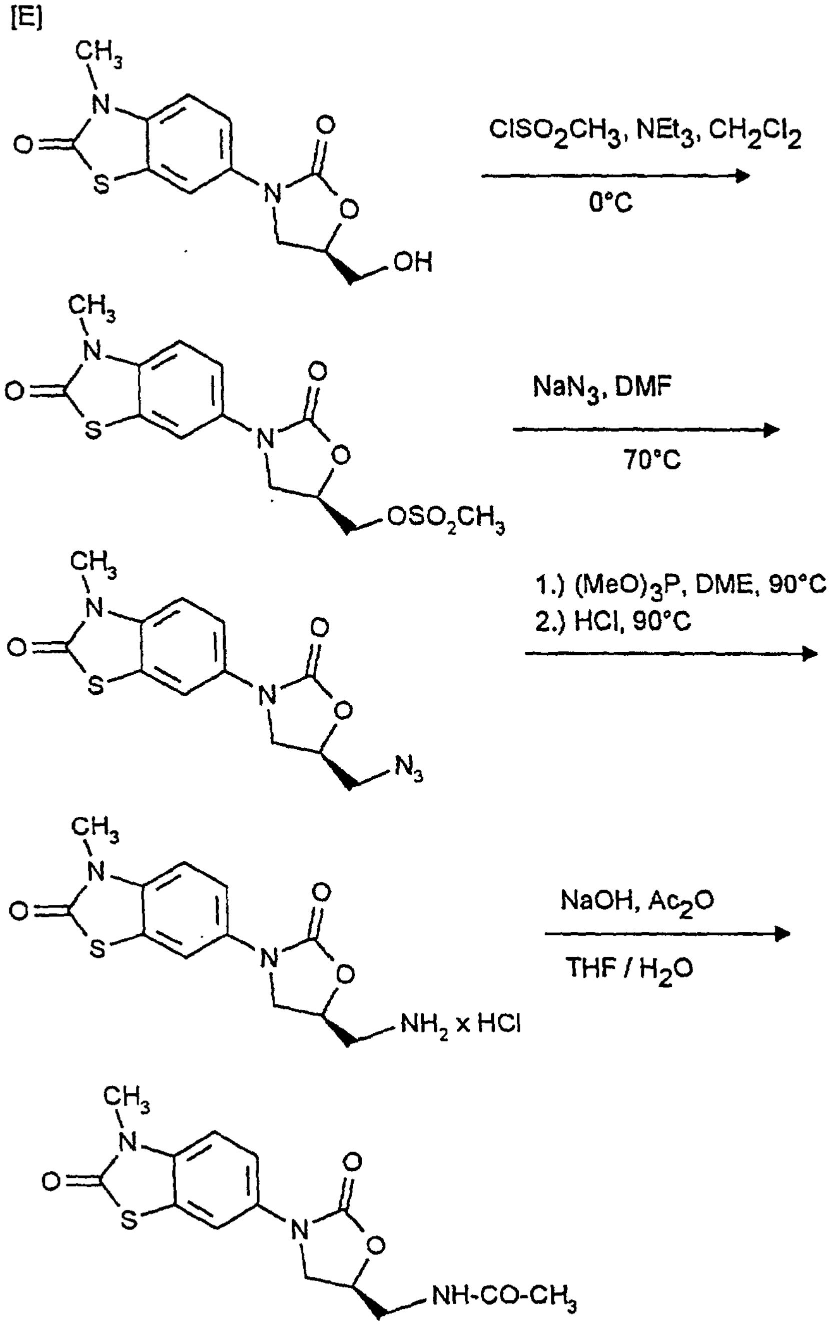

ClSO$_2$CH$_3$, NEt$_3$, CH$_2$Cl$_2$
0°C
NaN$_3$, DMF
70°C
1.) (MeO)$_3$P, DME, 90°C
2.) HCl, 90°C
NaOH, Ac$_2$O
THF / H$_2$O

[F]

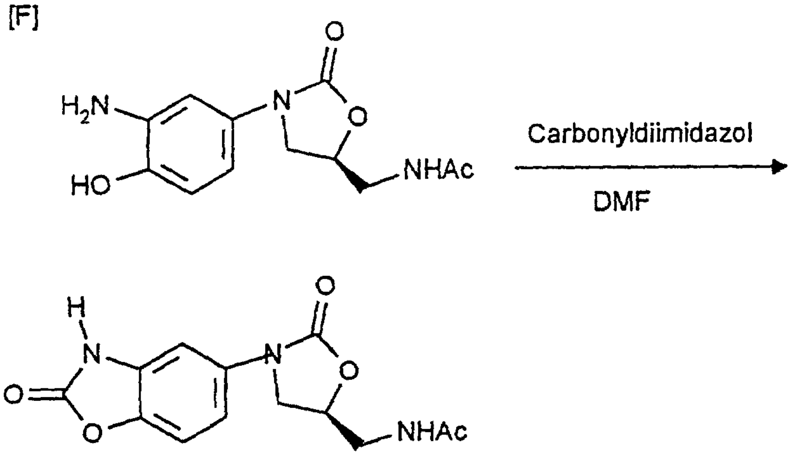


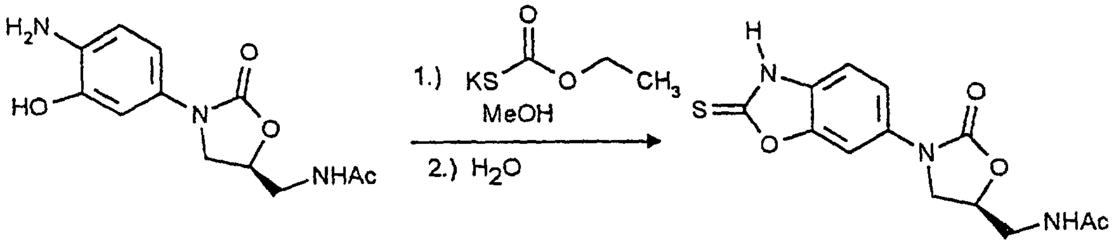


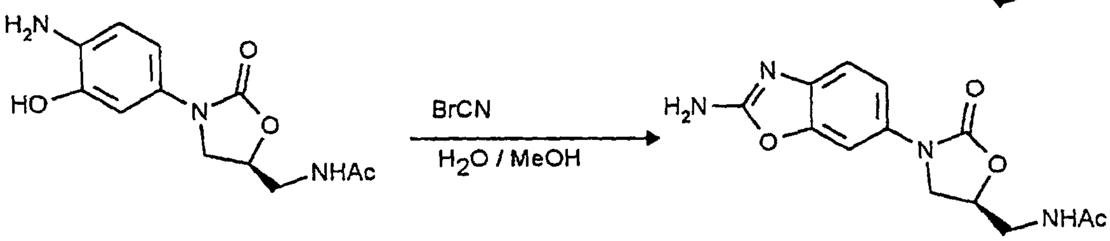

[G]

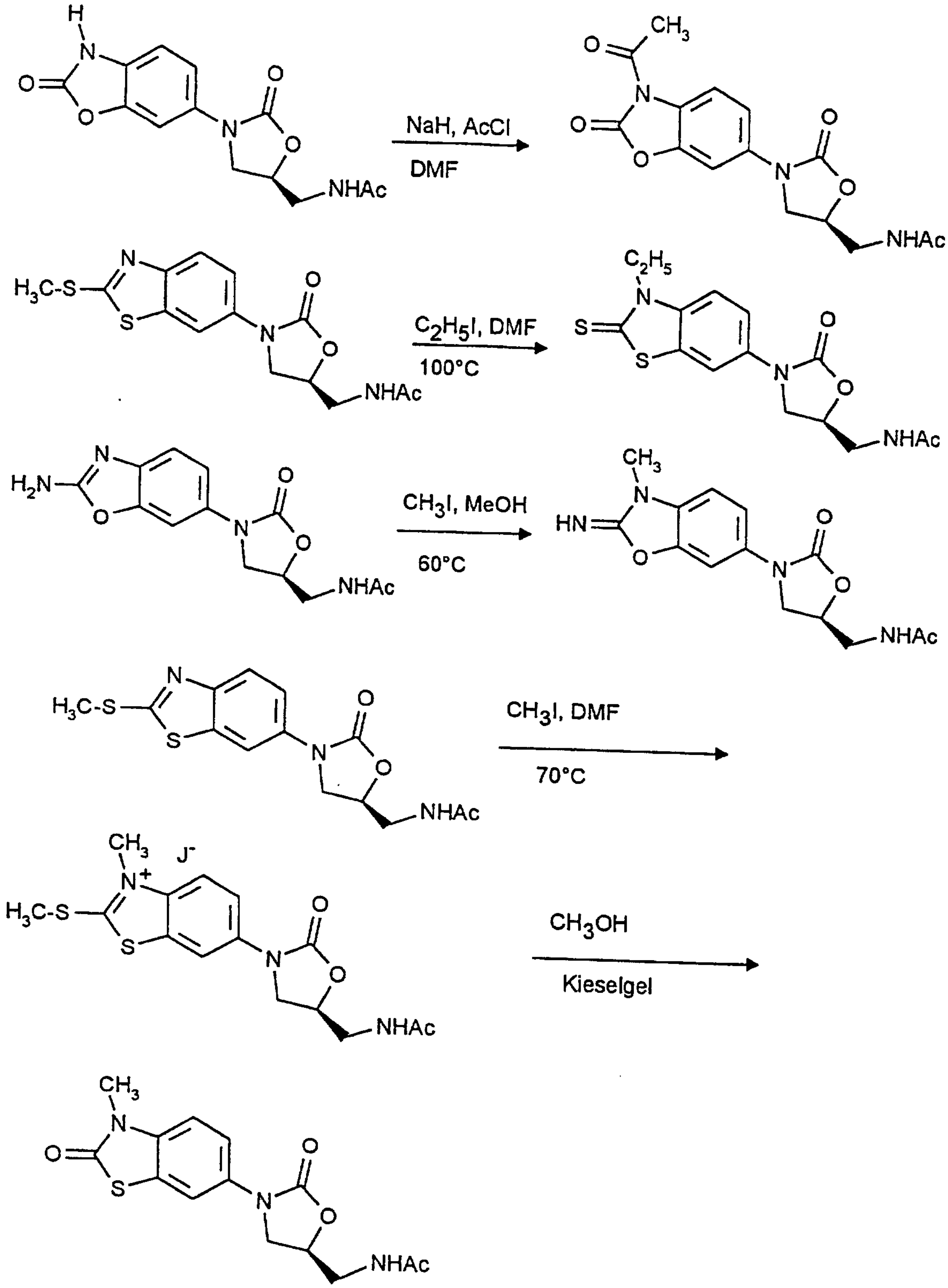
NaH, AcCl
DMF
$C_2H_5I$, DMF
100°C
$CH_3I$, MeOH
60°C
$CH_3I$, DMF
70°C
J⁻
$CH_3OH$
Kieselgel

[G]

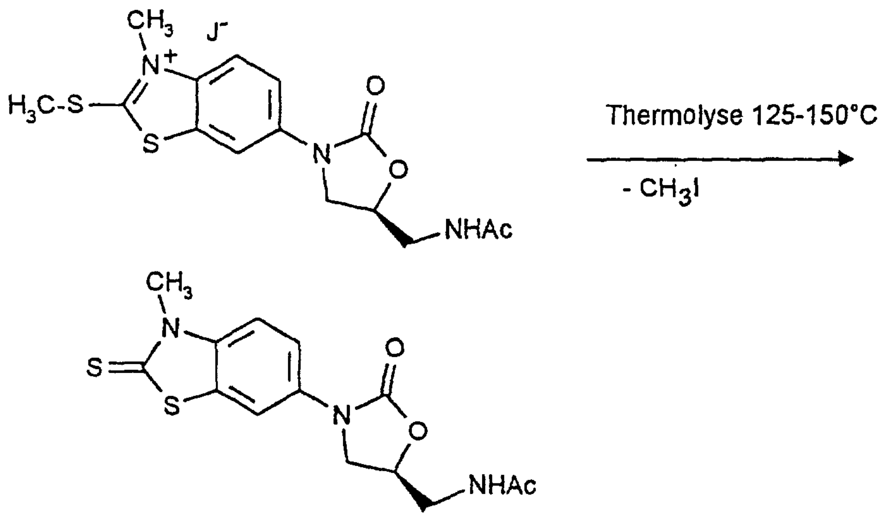


Als Lösemittel eignen sich in Abhängigkeit von den einzelnen Verfahrensschritten die üblichen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, 1,2-Dimethoxyethan, Tetrahydrofuran, Glykoldimethylether oder tert.Butylmethylether, oder Ketone wie Aceton oder Butanon, oder Amide wie Dimethylformamid oder Hexamethyl-phosphorsäuretriamid, oder Kohlenwasserstoffe wie Hexan, Benzol, Dichlorbenzol, Xylol oder Toluol, oder Dimethylsulfoxid, Acetonitril, Essigester, oder Halogenkohlenwasserstoffe wie Methylenchlorid, Chloroform oder Tetrachlorkohlenstoff, oder Pyridin, Picolin oder N-Methylpiperidin. Ebenso können Gemische der genannten Lösemittel verwendet werden.

Als Basen eignen sich in Abhängigkeit von den einzelnen Verfahrensschritten die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Ethyldiisopropylamin, Triethylamin, Picolin, Pyridine oder N-Methylpiperidin, oder Amide wie Natriumamid oder Lithiumdiisopropylamid, oder Lithium-N-silylalkylamide, wie beispielsweise Lithium-N-(bis)triphenylsilylamid oder Lithiumalkyle wie n-Butyllithium.

Die Base wird in einer Menge von 1 mol bis 10 mol, bevorzugt von 1 mol bis 3 mol bezogen auf 1mol der Verbindungen der allgemeinen Formeln (II), (III), (IV) und (Va) eingesetzt.

Alle Umsetzungen werden im allgemeinen bei normalem, erhöhtem oder bei erniedrigtem Druck durchgeführt (z.B. 0,5 bis 5 bar). Im allgemeinen arbeitet man bei Normaldruck.

Das Verfahren [A] erfolgt bevorzugt in Xylol oder Dichlorbenzol, gegebenenfalls in Gegenwart von Triethylamin, unter Rückfluß.

Die basenkatalysierte Umesterung wird mit einem der oben aufgeführten Alkohole, vorzugsweise Methanol, in einem Temperaturbereich von -10°C bis +40°C, vorzugsweise bei Raumtemperatur durchgeführt.

Als Basen eignen sich im allgemeinen Natriumhydrogencarbonat, Natriummethanolat, Hydrazinhydrat, Kaliumcarbonat oder Caesiumcarbonat. Bevorzugt ist Caesiumcarbonat.

Das Verfahren [B] erfolgt in einem der oben aufgeführten Ether mit Lithiumalkylverbindungen oder Lithium-N-silylamiden, wie beispielsweise n-Butyllithium, Lithiumdiisopropylamid oder Lithium-bistrimethylsilylamid, vorzugsweise in Tetrahydrofuran und Lithium-bis-trimethylsilylamid oder n-Butyllithium, in einem Temperaturbereich von -100°C bis +20°C, vorzugsweise von -75°C bis -40°C.

Für das Verfahren [C] eignen sich für den 1. Schritt vorzugsweise die oben aufgeführten Alkohole, im Falle der anschließenden Cyclisierung Tetrahydrofuran.

Als Basen für die Cyclisierung eignen sich vorzugsweise die oben aufgeführten Lithium-N-silylalkylverbindungen oder n-Butyllithium. Besonders bevorzugt ist n-Butyllithium.

Der erste Reaktionsschritt wird bei der Siedetemperatur des entsprechenden Alkohols, die Cyclisierung in einem Temperaturbereich von -70°C bis Raumtemperatur durchgeführt.

Die Cyclisierung [D] wird in Anwesenheit eines Hilfsmittels und/oder Anwesenheit einer Säure durchgeführt.

Als Säuren eignen sich im allgemeinen anorganische Säuren wie beispielsweise Salzsäure oder Schwefelsäure, oder organische Carbonsäuren mit 1-6 C-Atomen, gegebenenfalls substituiert durch Fluor, Chlor und/oder Brom, wie beispielsweise Essigsäure, Trifluoressigsäure, Trichloressigsäure oder Propionsäure, oder Sulfonsäuren mit $C_1$-$C_4$-Alkylresten oder Arylresten wie beispielsweise Methansulfonsäure, Ethansulfonsäure, Benzolsulfonsäure oder Toluolsulfonsäure. Besonders bevorzugt ist Salzsäure.

Die Säure wird in einer Menge von 1 mol bis 10 mol, bevorzugt von 1 mol bis 2 mol, bezogen auf 1 mol der Verbindungen der allgemeinen Formel (VI) eingesetzt.

Als Hilfsmittel eignen sich die üblichen Reagenzien wie Phosgen, Carbonyldiimidazol oder Kohlensäurediethylester oder Chlorameisensäuretrichlormethylester. Bevorzugt sind Carbonyldiimidazol, Kohlensäurediethylester oder Chlorameisensäuretrichlormethylester.

Als Lösemittel eignen sich die oben aufgeführten Halogenkohlenwasserstoffe. Bevorzugt ist Methylenchlorid.

Die Cyclisierungen erfolgen im allgemeinen in einem Temperaturbereich von -20°C bis 100°C, vorzugsweise bei -20°C bis Raumtemperatur.

Die Acylierung [E] erfolgt im allgemeinen in einem der oben aufgeführten Ether oder Halogenkohlenwasserstoffen, vorzugsweise Tetrahydrofuran oder Methylenchlorid, in einem Temperaturbereich von -30°C bis 50°C, bevorzugt von -10°C bis Raumtemperatur.

Die Reduktionen erfolgen im allgemeinen mit Hydriden in inerten Lösemitteln oder mit Boranen, Diboranen oder ihren Komplexverbindungen.

Die Reduktionen können im allgemeinen durch Wasserstoff in Wasser oder in inerten organischen Lösemitteln wie Alkoholen, Ethern oder Halogenkohlenwasserstoffen, oder deren Gemischen, mit Katalysatoren wie Raney-Nickel, Palladium, Palladium auf Tierkohle oder Platin, oder mit Hydriden oder Boranen in inerten Lösemitteln, gegebenenfalls in Anwesenheit eines Katalysators durchgeführt werden.

Bevorzugt werden die Reduktionen mit Hydriden, wie komplexen Borhydriden oder Aluminiumhydriden sowie Boranen durchgeführt. Besonders bevorzugt werden hierbei Natriumborhydrid, Lithiumborhydrid, Natriumcyanoborhydrid, Lithiumaluminiumhydrid, Natrium-bis-(2-methoxyethoxy)aluminiumhydrid oder Boran-Tetrahydrofuran eingesetzt.

Die Reduktion der Azide [E] erfolgt mit $(CH_3O)_3P$ und Salzsäure.

Die Reduktion erfolgt im allgemeinen in einem Temperaturbereich von -50°C bis zum jeweiligen Siedepunkt des Lösemittels, bevorzugt von -20°C bis +90°C.

Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether oder Amide wie Hexamethylphosphorsäuretriamid oder Dimethylformamid, oder Essigsäure. Ebenso ist es möglich, Gemische der genannten Lösemittel zu verwenden.

Die Abspaltung der Hydroxyschutzgruppen erfolgt im allgemeinen nach üblicher Methode, beispielsweise durch hydrogenolytische Spaltung der Benzylether in den oben aufgeführten inerten Lösemitteln in Anwesenheit eines Katalysators mit Wasserstoff-Gas.

Die Abspaltung der Aminoschutzgruppe erfolgt im allgemeinen, ebenfalls nach üblichen Methoden, abspaltet und zwar vorzugsweise Boc mit Salzsäure in Dioxan, Fmoc mit Piperidin und Z mit HBr/HOAc oder durch Hydrogenolyse.

Die oben aufgeführten anderen Derivatisierungsreaktionen erfolgen im allgemeinen nach denen in Compendium of Organic Synthetic Methods, T.T. Harrison und S. Harrison, Wiley Interscience, publizierten Methoden.

Bevorzugt werden Redoxreaktionen, reduktive Aminierung, Umesterung und die Halogenisierung von Methylgruppen mit N-Bromsuccinimid (NBS) oder N-Chlorsuccinimid (NCS) aufgeführt, die im folgenden beispielhaft erläutert werden.

Als Lösemittel für die Alkylierung eignen sich übliche organische Lösemittel, die sich unter den Reaktionsbedingungen nicht verändern. Hierzu gehören bevorzugt Ether wie Diethylether, Dioxan, Tetrahydrofuran, Glykoldimehylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Acetonitril, Aceton oder Nitromethan. Ebenso ist es möglich, Gemische der genannten Lösemittel zu verwenden. Bevorzugt sind Dichlormethan, Dimethylsulfoxid und Dimethylformamid.

Die Alkylierung wird in den oben aufgeführten Lösemitteln bei Temperaturen von 0°C bis +150°C, vorzugsweise bei Raumtemperaturen bis +100°C, bei Normaldruck durchgeführt.

Die Amidierung und die Sulfoamidierung erfolgen im allgemeinen in inerten Lösemitteln in Anwesenheit einer Base und eines Dehydratisierungsmittels.

Als Lösemittel eignen sich hierbei inerte organische Lösemittel, die sich unter den Reaktionsbedingungen nicht verändern. Hierzu gehören Halogenkohlenwasserstoffe wie Dichlormethan, Trichlormethan, Tetrachlormethan, 1,2-Dichlorethan, Trichlorethan, Tetrachlorethan, 1,2-Dichlorethan oder Trichlorethylen, Kohlenwassserstoffe wie Benzol, Xylol, Toluol, Hexan, Cyclohexan, oder Erdölfraktionen, Nitromethan, Dimethylformamid, Acetonitril oder Tetrahydrofuran. Ebenso ist es möglich, Gemische der Lösemittel einzusetzen. Besonders bevorzugt sind Dichlormethan und Tetrahydrofuran.

Als Basen für die Amidierung und die Sulfoamidierung eignen sich die üblichen basischen Verbindungen. Hierzu gehören vorzugsweise Alkali- und Erdalkalihydroxide wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat oder -ethanolat oder Kalium-tert.-buylat, oder organische Amine wie Benzyltrimethylammoniumhydroxid, Tetrabutylammoniumhydroxid, Pyridin, Triethylamin oder N-Methylpiperidin.

Die Amidierung und die Sulfoamidierung werden im allgemeinen in einem Temperaturbereich von 0°C bis 150°C, bevorzugt bei 25°C bis 40°C, durchgeführt.

Die Amidierung und die Sulfoamidierung werden im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Bei der Durchführung der Amidierung und der Sulfoamidierung wird die Base im allgemeinen in einer Menge von 1 bis 3 Mol, bevorzugt von 1 bis 1,5 Mol, bezogen auf 1 Mol der jeweiligen Carbonsäure, eingesetzt.

Als Dehydratisierungsreagenzien eignen sich Carbodiimide wie beispielsweise Diisopropylcarbodiimid, Dicyclohexylcarbodiimid oder N-(3-Dimethylaminopropyl)--N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Isobutylchloroformat oder Benzotriazolyloxy-tris-(dimethylamino)phosphonium-hexyfluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder 4-Dimethylaminopyridin.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 Mol, bevorzugt von 1 bis 1,5 Mol bezogen auf 1 Mol des Esters eingesetzt. Besonders bevorzugt verwendet man molare Mengen der Reaktanden.

Die Veresterung erfolgt im allgemeinen mit den entsprechenden Alkoholen in Anwesenheit von Säuren, vorzugsweise Schwefelsäure, in einem Temperaturbereich von 0°C bis 150°C, vorzugsweise von 50°C bis 100°C und Normaldruck.

Die Verbindungen der allgemeinen Formeln (IV) und (VIII) sind bekannt oder können nach üblichen Methoden hergestellt werden.

Die Verbindungen der allgemeinen Formel (VII) sind größtenteils neu und können beispielsweise wie oben beschrieben hergestellt werden.

Die Verbindungen der allgemeinen Formel (II) sind teilweise bekannt oder neu und können dann beispielsweise hergestellt werden, indem man die entsprechenden Amine mit Chlorameisensäuretrichlorethylester in einem der oben aufgeführten Lösemittel, vorzugsweise Xylol bei Rückflußtemperatur umsetzt.

Die Verbindungen der allgemeinen Formel (III) sind teilweise bekannt oder neu und können dann beispielsweise hergestellt werden, indem man ausgehend von den entsprechenen Carbonsäuren entweder mit Chlorameisensäureisobutylester / Aceton, Natriumazid/Wasser oder mit Diphenylphosphorylazid / Tetrahydrofuran oder mit Xylol oder Methylenchlorid in Gegenwart einer der oben angegebenen Basen, vorzugsweise Triethylamin, bei -10°C bis Raumtemperatur umsetzt.

Die Verbindungen der allgemeinen Formeln (V) und (Va) sind teilweise bekannt oder neu und können entweder durch Abspaltung von Stickstoff aus den entsprechenden Carbonsäureaziden und Umsetzung mit den entsprechenden Alkoholen oder durch Umsetzung der entsprechenden Amine mit Chlorameisensäureestern, vorzugsweise Chlorameisensäurebenzylester in einem der oben aufgeführten Lösemittel, vorzugsweise Tetrahydrofuran oder Dioxan, in einem Temperaturbereich von -10°C bis 200°C, vorzugsweise von 0°C bis 150°C, hergestellt werden.

Die Verbindungen der allgemeinen Formel (Ia) sind neu und können beispielsweise wie unter [A], [B], [D] oder

[E] beschrieben hergestellt werden.

Die Verbindungen der allgemeinen Formel (Ib), (Ic), (Id) und (Ie) sind neu und können wie oben beschrieben hergestellt werden.

Die Verbindungen der allgemeinen Formel (VI) sind größtenteils bekannt oder neu und können beispielsweise hergestellt werden, indem man ausgehend von den freien Aminen (Ia) entweder mit dem Acetonid von Glycerinaldehyd in Methanol und in Anwesenheit von Natriumacetat / Natriumcyanborhydrid oder von Natriumboranat und Methanol in einem Temperaturbereich von -20°C bis +40°C, bevorzugt von -10°C bis 20°C und Normaldruck umsetzt.

Die Umsetzung der Verbindungen der allgemeinen Formel (IX) [F] erfolgt in einem Temperaturbereich von -10°C bis 150°C, vorzugsweise von 10°C bis 60°C und Normaldruck.

Die Verbindungen der allgemeinen Formel (IX) sind vom Bedeutungsumfang der EP 609 905 umfaßt, als konkrete Verbindungen aber neu und können in Analogie zu dem oben aufgeführten Verfahren [E] durch Einsatz von Acetylchlorid hergestellt werden.

Die Acylierungen [G] erfolgen im allgemeinen in einem der oben aufgeführten Lösemitteln, vorzugsweise Dimethylformamid, in Anwesenheit einer Base, vorzugsweise Natriumhydrid, in einem Temperaturbereich von 0°C bis 150°C, vorzugsweise von 20°C bis 80°C und Normaldruck.

Die Alkylierungen unter Doppelbindungsverlagerung erfolgen in Abhängigkeit des Restes A in einem der oben aufgeführten Lösemitteln, vorzugsweise Dimethylformamid oder Methanol, in einem Temperaturbereich von 30°C bis 150°C, vorzugsweise von 50°C bis 110°C und Normaldruck.

Die Umsetzung zu den Verbindungen der allgemeinen Formel (XI) [G] erfolgt in einem der oben aufgeführten Lösemitteln, vorzugsweise Dimethylformamid in einem Temperaturbereich von -10°C bis 150°C, vorzugsweise von 20°C bis 70°C und Normaldruck.

Die Thermolyse [G] erfolgt in einem Temperaturbereich von 80°C bis 200°C, bevorzugt von 125°C bis 150°C.

Die Oxidation zum S-oxid erfolgt im allgemeinen in einem der oben aufgeführten Lösemittel, vorzugsweise in Methylenchlorid mit Oxidationsmitteln wie beispielsweise Metachlorperbenzoesäure, Wasserstoffperoxid, Peressigsäure oder Oxon, vorzugsweise mit Metachlorperbenzoesäure in einem Temperaturbereich von 0°C bis 80°C, bevorzugt von 20°C bis 60°C.

Die Verbindungen der Formel (X) sind als konkrete Verbindungen neu und können in Analogie zu dem oben aufgeführten Verfahren [E] hergestellt werden.

Die Verbindungen der allgemeinen Formel (XI) sind neu und können wie oben beschrieben hergestellt werden.

Die MHK-Werte wurden mit Hilfe der Mikrodilutionsmethode in BH-Medium bestimmt. Jede Prüfsubstanz wurde im Nährmedium gelöst. In der Mikrotiterplatte wurde durch serielle Verdünnung eine Konzentrationsreihe der Prüfsubstanzen angelegt. Zur Inokulation wurden Übernachtkulturen der Erreger verwandt, die zuvor im Nährmedium 1:250 verdünnt wurden. Zu 100 µl der verdünnten, wirkstoffhaltigen Nährlösungen wurden je 100 µl Inokulationslösung gegeben.

Die Mikrotiterplatten wurden bei 37°C bebrütet und nach ca. 20 Stunden (stapho) oder nach 3 bis 5 Tagen (Mycobacterium) abgelesen. Der MHK-Wert (µg/ml) gibt die niedrigste Wirkstoffkonzentration an, bei der kein Wachstum zu erkennen war.

MHK-Werte (µg/ml):

| Bsp.-Nr. | Staph. 133 | Staph. 48N | Staph 25701 | Staph. 9TV | E. coli Neumann | Klebs. 57 USA | Psdm. Bonn |
|---|---|---|---|---|---|---|---|
| 17 | 8 | 8 | 8 | 8 | >64 | >64 | >64 |
| 18 | 0,25 | 0,25 | 0,25 | 0,06 | >64 | >64 | >64 |
| 22 | 1 | 1 | 1 | 0,5 | >64 | >64 | >64 |
| 24 | 8 | 16 | 16 | 16 | >64 | >64 | >64 |
| 37 | 1 | 1 | 1 | 0,5 | 16 | 64 | 64 |
| 38 | 4 | 4 | 4 | 1 | >64 | >64 | >64 |
| 39 | 4 | 4 | 4 | 4 | >64 | >64 | >64 |
| 43 | 0,25 | 0,125 | 0,25 | 0,125 | >32 | >64 | >64 |
| 44 | 0,5 | 0,5 | 0,5 | 0,5 | >64 | >64 | >64 |
| 38 | 4 | 4 | 4 | 1 | >64 | >64 | >64 |
| 47 | 0,5 | 0,5 | 0,5 | 0,25 | 32 | 64 | >64 |
| 56 | 0,5 | 0,5 | 0,5 | 0,25 | 64 | >64 | >64 |
| 70 | 0,5 | 0,5 | 0,5 | 0,5 | >64 | >64 | >64 |

| Bsp.-Nr. | Staph. 133 | Staph. 48N | Staph 25701 | Staph. 9TV | E. coli Neumann | Klebs. 57 USA | Psdm. Bonn |
|---|---|---|---|---|---|---|---|
| 62 | 1 | 1 | 1 | 0,5 | >64 | >64 | >64 |
| 84 | 1 | 1 | 1 | 0,5 | 64 | >64 | >64 |
| 94 | 0,5 | 0,5 | 0,5 | 0,25 | 64 | >64 | >64 |

MHK-Werte (µg/ml)
Keim: Mycobacterium smegmacis

| Bsp.-Nr. | DSM 43061 | DSM 43078 | DSM 43277 | DSM 43299 | DSM 43464 | DSM 43465 |
|---|---|---|---|---|---|---|
| 18 | 0,25 | 0,25 | 0,25 | 1 | 1 | 0,5 |
| 56 | 1 | 1 | 0,25 | 1 | 4 | 0,5 |
| 54 | 4 | 1 | 2 | 4 | 16 | 4 |
| 70 | 0,25 | 0,125 | 0,5 | 2 | 8 | 1 |

Die erfindungsgemäßen Verbindungen der allgemeinen Formeln (I), (Ia), (Ib), (Ic), (Id), (Id) und (Ie) weisen bei geringer Toxizität ein breites antibakterielles Spektrum, speziell gegen gram-positive Bakterien sowie Mycobacterien, Corynebacterien, Haemophilus Influenzae und Anaerobae Keime auf. Diese Eigenschaften ermöglichen ihre Verwendung als chemotherapeutische Wirkstoffe in der Human- und Tiermedizin.

Die erfindungsgemäßen Verbindungen sind gegen ein breites Spektrum von Mikroorganismen wirksam. Mit ihrer Hilfe können gram-positive Bakterien und bakterienähnliche Mikroorganismen, wie Mycoplasmen, bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch solche Erreger hervorgerufen werden.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Der oder die Wirkstoffe können gegebenenfalls in einem oder mehreren der oben angegebenen Trägerstoffe auch in mikroverkapselter Form vorliegen.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30mg/kg Körpergewicht.

Die erfindungsgemäßen Verbindungen können zum Zweck der Erweiterung des Wirkungsspektrums und um eine Wirkungssteigerung zu erreichen auch mit anderen Antibiotika kombiniert werden.

Anhang zum experimentellen Teil

Liste der verwendeten Laufmittelgemische zur Chromatographie:

| | |
|---|---|
| I | Dichlormethan : Methanol |
| II | Toluol : Ethylacetat |
| III | Acetonitril : Wasser |
| IV | Ethylacetat |
| V | Petrolether : Ethylacetat |
| VI | Dichlormethan Ethanol |
| VII | Toluol : Ethanol |
| VIII | Toluol : Ethanol : Triethylamin |

Abkürzungen:

| | |
|---|---|
| Z | Benzyloxycarbonyl |
| Boc | tert.Butoxycarbonyl |
| DMF | Dimethylformamid |
| Ph | Phenyl |
| Me | Methyl |
| THF | Tetrahydrofuran |
| CDI | Carbonyldiimidazol |
| DCE | Dichlorethan |

**Ausgangsverbindungen**

**Beispiel I**

6-(Benzyloxycarbonylamino)-3-methyl-2-benzothiazolinon

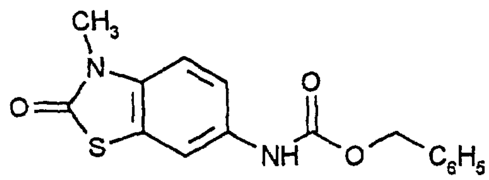

1,76 g (8,12 mmol) 6-Amino-3-methyl-2(3H)-benzothiazolon-hydrochlorid (J. Heterocyclic Chem. 1992, 29, 1069) in 17 ml Wasser, 14 ml THF und 17 ml ges. $NaHCO_3$-Lösung werden bei 0°C tropfenweise mit 1,3 ml (9,10 mmol) Chlorameisensäurebenzylester versetzt. Nach 1 h werden 120 ml Wasser hinzugegeben, das THF im Vakuum abgezogen, der Niederschlag abgesaugt, dreimal mit Wasser, zweimal mit Petrolether gewaschen und bei 60°C getrocknet.
Ausbeute: 2,44 g (96%)
Smp.: 183°C
$R_f$ (II, 7:3) = 0,39
$^1$H-NMR ([$D_6$]DMSO): δ = 7,77 (d, J = 1 Hz, 1H, Benzothiazolinon 7-H); 7,23 - 7,45 (m, 6H, Ph), 7,22 (d, J = 6 Hz, 1H, Benzothiazolinon 4-H); 5,15 (s, 2H); 3,38 (s, 3H-$CH_3$).

Wie für Beispiel I beschrieben erhält man aus den entsprechenden Aminen mit Chlorameisensäurebenzylester die in Tabelle I aufgeführten Verbindungen:

**Tabelle I:**

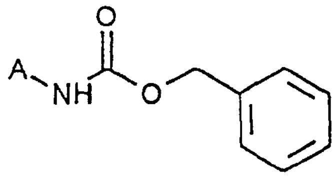

| Bsp.-Nr. | A | Ausbeute (% d.Th.) | Schmp. (°C) | $R_f$ (Laufmittel, Verhältnis) | MS (DCI, $NH_3$) m/z $(M+H)^+$ |
|---|---|---|---|---|---|
| II | 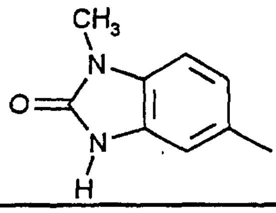 | 96 | 241 | 0,24 (I, 95:5) | 298 |
| III | 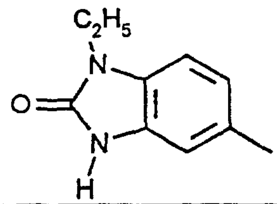 | 99 | 211 | 0,43 (I, 9:1) | 312 |
| IV | 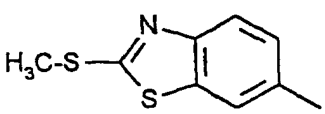 | 96 | 111 | 0,71 (II, 1:4) | 331 |

**Beispiel V**

5-(Benzyloxycarbonylamino)-1,3-dimethyl-2-benzoimidazolinon

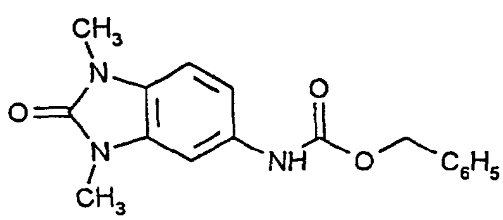

Eine gerührte Suspension von 2,49 g (8,37 mmol) der Verbindung aus Beispiel II, 3,47 g (25,11 mmol) Kaliumcarbonat und 1,90 ml (30,97 mmol) Iodmethan in 50 ml Ethanol wird 1,5 h zum Rückfluß erhitzt. Das Gemisch darf abkühlen, die Feststoffe werden bei einer Temperatur von 30°C durch Filtration abgetrennt und das Filtrat wird im Vakuum eingedampft. Der Rückstand wird in 50 ml Dichlormethan gelöst, mit $MgSO_4$ gut durchgerührt und nach Abdampfen des Lösemittels im Hochvakuum über Sicapent getrocknet. Man erhält 2,28 g (87%) der Titelverbindung als farblose Kristalle.
Schmp.: 176°C
$R_f$ = 0,48 (Dichlormethan : Methanol 95:5)
MS (EI, 70 eV) m/z = 311 $(M)^+$
$^1$H-NMR (200 MHz, $[D_6]$DMSO): $\delta$ = 9,70 (bs, 1H, NHCO); 7,40 (m, 6H, H arom.); 7,01 (s, 2H, H arom.); 5,12 (s, 2H,

$CH_2$); 3,30, 3,31 (2s, 6H, $NCH_3$).

Wie für Beispiel V beschrieben erhält man durch Alkylierung der Verbindungen aus Tabelle I die in Tabelle II aufgeführten Verbindungen:

**Tabelle II:**

| Bsp.-Nr. | Verbindung | Ausbeute (% d.Th.) | Schmp. (°C) | $R_f$ (Laufmittel, Verhältnis) | MS (CI) m/z $(M+H)^+$ |
|---|---|---|---|---|---|
| VI | 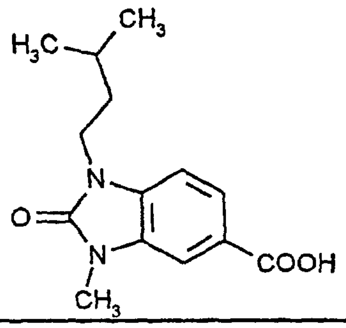 | 77 | / | 0,31 (I, 9:1) | 265 |
| VII | 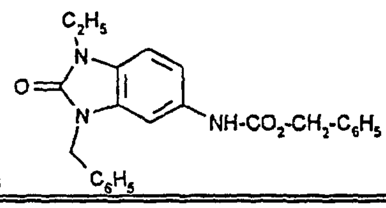 | 75 | 147 | 0,44 (I, 97:3) | 402 |

**Beispiel VIII**

5-Butyloxycarbonylamino-1-(3'-methylbutyl)-2-benzoimidazolinon

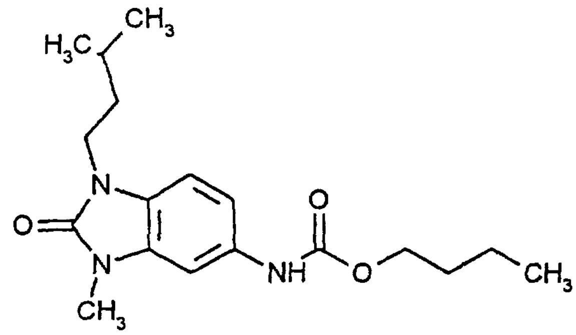

Zu einer auf 0°C gekühlten Lösung von 1,58 g (6,0 mmol) der Verbindung aus Beispiel VI und 1,0 ml (7,21 mmol) Triethylamin in 12 ml Aceton tropft man langsam 1,1 ml (7,8 mmol) Chlorameisensäureisobutylester in 5 ml Aceton. Man rührt 45 min bei 0°C und tropft dann langsam 586 mg (9,02 mmol) Natriumazid in 3 ml Wasser zu. Man rührt 1 Stunde bei 0°C und gibt den Ansatz auf 50 ml Eiswasser. Es wird mit Xylol (3 x 2 ml) extrahiert und die vereinigten organischen Phasen über $MgSO_4$ getrocknet. Diese Lösung wird dann zu 20 ml siedendem n-Butanol langsam zugetropft (heftige Gasentwicklung). Nach beendeter Zugabe wird noch 10 min. unter Rückfluß gekocht, dann auf RT abgekühlt, und das n-Butanol am Rotationsverdampfer abgezogen. Der Rückstand wird an 85 g Kieselgel chromatographiert. Man erhält 448 mg (22%) eines farblosen Öls.
$R_f$ (II, 7:3) = 0,25
MS (CI): m/z = 334 ($M^+$ + H)
$^1$H-NMR ([$D_6$]DMSO): δ = 9,50 (bs, 1H, NH); 7,32 (bs, 1H, Ph); 7,00 (bs, 2H, Ph); 4,10 (t, J = 7 Hz, 2H, $CH_2$); 3,80 (t, J = 6 Hz, 2H, $CH_2$); 3,32 (s, 3H, $NCH_3$); 1,30 - 1,72 (m, 8H); 0,80 - 1,10 (m, 11H).

Wie für Beispiel VIII beschrieben erhält man durch Umsetzung der entsprechenden Säuren die in der Tabelle III aufgeführten Verbindungen:

## Tabelle III:

| Bsp.-Nr. | Verbindung | Ausbeute (% d.Th.) | Schmp. (°C) | $R_f$ (Laufmittel, Verhältnis) | MS (DCI, $NH_3$) m/z $(M+H)^+$ |
|---|---|---|---|---|---|
| IX | 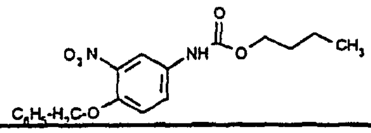 | 78 | 121-122 | 0,67 (VII, 95:5) | 345 |
| X | 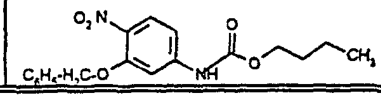 | 63 | 133 | 0,51 (VII, 95:5) | 345 |

**Beispiel XI**

(5R)-3-(4-Benzyloxy-3-nitrophenyl)-5-(hydroxymethyl)-oxazolidin-2-on

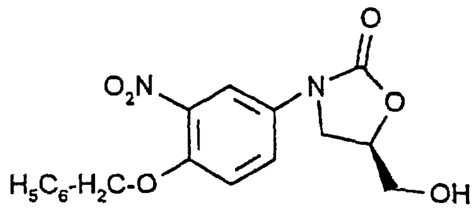

23,0 g (66,7 mmol) der Verbindung aus Beispiel IX werden in 200 ml THF gelöst und auf 0°C gekühlt. Nun werden langsam ca. 68 ml 1,0 M LiHMDS-Lösung in THF zugetropft. Anschließend werden 9,5 ml (68 mmol) (R)-Glycidylbutyrat zugetropft. Man läßt auf RT kommen, versetzt mit gesättigter Ammoniumchloridlösung und zieht im Vakuum das THF ab. Der entstandene Niederschlag wird abgesaugt, mit Wasser und Ether gewaschen und im Hochvakuum getrocknet.
Ausbeute: 20,85 g (91%)
Smp.: 128-130°C
$R_f$ (II, 1:1) = 0,21
MS (FAB): m/z = 345 ($M^+$)
$^1$H-NMR ([$D_6$] DMSO): δ = 8,0 (d, 1H, Ph), 7,62 (d, 1H, Ph), 7.30 - 7,50 (m, 6H, Ph), 5,30 (s, 2H, $CH_2$); 5,25 (t, 1H, OH); 4,68 - 4,80 (m, 1H,5-H); 4,15 (t, 1H, 4-H); 3,90 (dd, 1H, 4-H); 3,55 - 3,75 (m, 2H, $CH_2O$).

In Analogie zur Vorschrift des Beispiels XI werden die in der Tabelle IV aufgeführten Verbindungen hergestellt:

## Tabelle IV

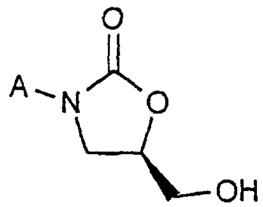

| Bsp.-Nr. | A | Ausbeute (% d.Th.) | Schmp. (°C) | Rf (Laufmittel, Verhältnis) | [α]20D (DMSO) | MS (FAB) m/z (M⁺ + H) |
|---|---|---|---|---|---|---|
| XII | 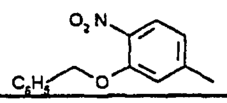 | 73 | 137-139 | 0,28 (II, 1:1) | -38,1 (c=0,985) | 345 |
| XIII | 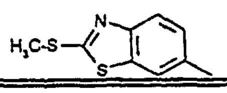 | 67 | 156 | 0,24 (II, 1:4) | | 297 |

**Beispiel XIV**

(5R)-3-(4-Benzyloxy-3-nitrophenyl)-5-(methylsulfonyloxymethyl)oxazolidin-2-on

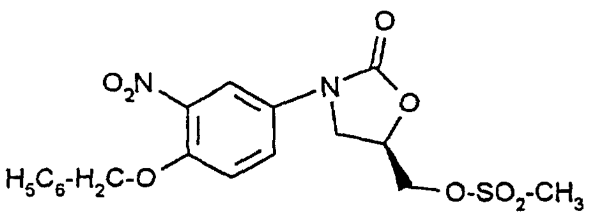

Eine auf 0°C gekühlte Lösung von 71,5 g (208 mmol) der Verbindung aus Beispiel XI und 35 ml (250 mmol) Triethylamin in 650 ml wasserfreiem THF wird langsam mit 23,6 ml (230 mmol) Methansulfonsäurechlorid versetzt. Man rührt 3 h bei 0°C und gibt auf Eiswasser. Der entstandene Niederschlag wird abgesaugt, mit Wasser und Toluol gewaschen und im Hochvakuum getrocknet. Ausbeute: 65,8 g (75%)

Smp.: 149-150°C

$R_f$ (VII, 5:1) = 0,36

MS (FAB): m/z = 423 ($M^+$)

$^1$H-NMR ([$D_6$]DMSO): δ = 8,12 (d, J = 1 Hz, 1H, Ph); 7,75 (dd, J = 6 Hz, J = 1 Hz, 1H, Ph); 7,35 - 7,55 (m, 6H, Ph); 5,30 (s, 2H, $CH_2$); 4,40 - 4,60 (m, 2H, $CH_2O$); 4,22 (t, J = 9 Hz, 1H, 4-H); 3,85 (dd, J = 9 Hz, J = 5 Hz, 1H, 4-H); 3,25 (s, 3H, $SO_2CH_3$).

In Analogie zur Vorschrift des Beispiels XIV werden die in der Tabelle V aufgeführten Verbindungen hergestellt:

**Tabelle V:**

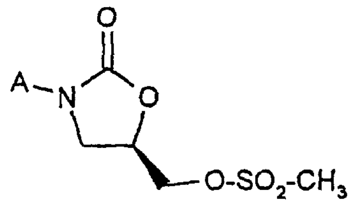

| Bsp.-Nr. | A | Ausbeute (% d.Th.) | Schmp. (°C) | $R_f$ (Laufmittel, Verhältnis) | $[\alpha]^{20}_D$ (DMSO) | MS (FAB) m/z ($M^+$ +H) |
|---|---|---|---|---|---|---|
| XV | 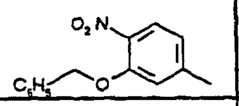 | 92 | 140-142 | 0,34 (VII, 5:1) | -48,8 (c=1,01) | 423 |
| XVI | 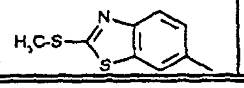 | 82 | 106 | 0,41 (I, 95.5) | / | 375 |

**Beispiel XVII**

(5R)-3-(4-Benzyloxy-3-nitrophenyl)-5-(azidomethyl)oxazolidin-2-on

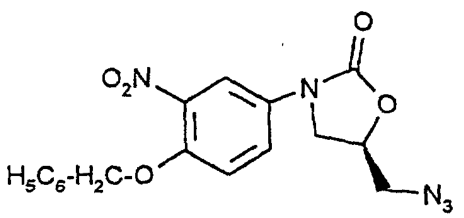

Eine Lösung von 25,7 g (60,8 mmol) der Verbindung aus Beispiel XI in 200 ml wasserfreiem DMF wird mit 4,4 g (66,9 mmol) Natriumazid versetzt und 12 h bei 70°C gerührt. Man läßt auf Raumtemperatur abkühlen und rührt 200 ml Eiswasser ein. Der entstandene Niederschlag wird abfiltriert, mit Wasser und Petrolether gewaschen und im Vakuum getrocknet.
Ausbeute: 21,4 g (95%)
Smp.: 158-160°C
$R_f$ (VII, 5:1) = 0,48
MS (EI) : m/z = 370 ($M^+$)
$^1$H-NMR -([$D_6$]DMSO): δ = 8,05 (d, 1H, J = 8 Hz, Ph); 7,25 - 7,50 (m, 7H, Ph); 5,30 (s, 2H, $CH_2$); 4,85 - 5,05 (m, 1H, 5-H); 4,23 (t, J= 9 Hz, 1H, 4-H); 3,55 - 3,90 (m, 3H, 4-H, $CH_2N_3$).

In Analogie zur Vorschrift des Beispiels XVII werden die in Tabelle VII aufgeführten Verbindungen hergestellt:

**Tabelle VII:**

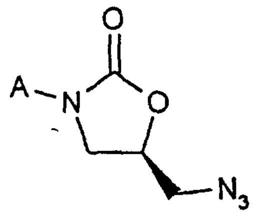

| Bsp.-Nr. | A | Ausbeute (% d.Th.) | Schmp. (°C) | $R_f$ (Laufmittel, Verhältnis) | $[\alpha]^{20}_{d}$ (DMSO) | MS (FAB) m/z ($M^+$ + H) |
|---|---|---|---|---|---|---|
| XVIII | 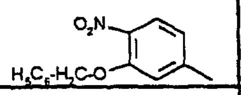 | 92 | 138-140 | 0,26 (VII, 5:1) | -119,4° (c=1,1) | 370 |
| XIX | 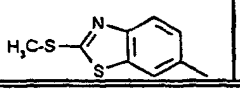 | 95 | 136 | 0,59 (I, 95:5) | / | 322 |

**Beispiel XX**

(5S)-3-(4-Benzyloxy-3-nitrophenyl)-5-(aminomethyl)oxazolidin-2-on

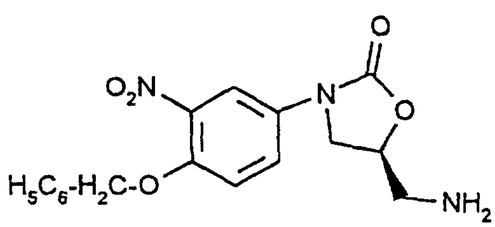

Eine Lösung von 53,1 g (144 mmol) der Verbindung aus Beispiel XVII in 160 ml 1,2-Dimethoxyethan wird auf 50°C erwärmt. Man tropft langsam 20,4 ml (173 mmol) Trimethylphosphit zu (Gasentwicklung) und rührt nach beendeter Zugabe 2 h bei 90°C. Nun tropft man 36 ml 6 N HCl zu und rührt nochmals 22 h bei 90°C. Man läßt auf Raumtemperatur abkühlen, gibt 810 ml 0,1 N HCl hinzu, wäscht die wäßrige Phase mit Ether (3x 320 ml) und stellt anschließend auf pH = 9. Die wäßrige Phase wird mit Essigester (3 x 650 ml) extrahiert (2 x 300 ml), die vereinigten organischen Phasen mit ges. NaCl-Lösung gewaschen (1 x 100 ml) und getrocknet ($Na_2SO_4$). Die Lösemittel werden im Vakuum abgezogen und im Hochvakuum getrocknet.
Ausbeute: 47,2 g (96%)
Smp.: 135-136°C
$R_f$ (VIII, 85:10:5) = 0,05
MS (EI): m/z = 344 ($M^+$)
$^1$H-NMR ([$D_6$]DMSO): δ = 8,3 - 9,1 (bs, 3H, $NH_3$); 8,15 (d, 1H, Ph); 7,3 - 7,8 (m, 7H, Ph); 5,30 (v, 2H, $CH_2$); 4,9 - 5,1 (m, 1H, 4-H); 4,20 (m, 1H, 5-H); 4,00 (m, 1H, 5-H); 3,10 - 3,40 (m, 2H, $CH_2N$).

In Analogie zur Vorschrift des Beispiels XX werden die in der Tabelle VIII aufgeführten Verbindungen hergestellt:

## Tabelle VIII:

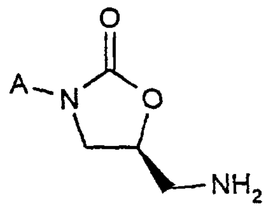

| Bsp.-Nr. | D | Ausbeute (% d.Th.) | Schmp. (°C) | $R_f$ (Laufmittel, Verhältnis) | MS (FAB) m/z ($M^+$) |
|---|---|---|---|---|---|
| XXI | 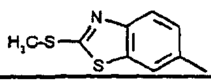 | 83 | 110 | 0,09 (III, 9:1) | |
| XXII | 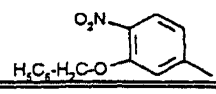 | 89 | 132-134 | 0,08 (VIII, 85:10:5) | 344 |

**Beispiel XXIII**

(5S)-3-(4-Benzyloxy-3-nitrophenyl)-5-(acetylaminomethyl)oxazolidin-2-on

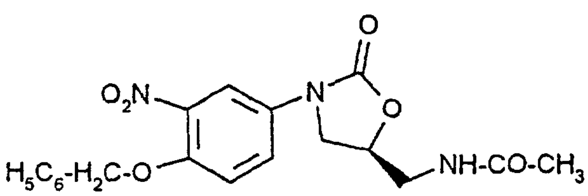

Zu einer auf 0°C gekühlten Lösung von 47,2 g (137 mmol) der Verbindung aus Beispiel XX und 29,04 ml (212 mmol) Triethylamin in 500 ml wasserfreiem THF tropft man langsam 14,6 ml (205 mmol) Acetylchlorid. Man rührt 2 h bei 0°C nach und gibt auf Eiswasser. Der Niederschlag wird abgesaugt, mit Wasser und Ether gewaschen, und im Hochvakuum über $P_2O_5$ getrocknet.
Ausbeute: 48,9 g (93%)
Smp.: 177-178°C
$R_f$ (VII, 1:1) = 0,51
MS (FAB) m/z = 386 $(M+H)^+$
$^1$H-NMR ([$D_6$]DMSO): δ = 8,24 (t, J = 4 Hz, 1H, NH); 8,10 (d, J = 1 Hz, 1H, Ph); 7,75 (dd, J = 6 Hz, J = 1 Hz, 1H, Ph); 7,20 - 7,50 (m, 6H, Ph); 5,30 (s, 2H, $CH_2$); 4,70 - 4,80 (m, 1H, 5-H); 4,15 (t, J = 9 Hz, 1H, 4-H); 3,70 (dd, J = 9 Hz, J = 5 Hz, 1H, H-4); 3,35 - 3,50 (m, 5H, $CH_2N$, $NCH_3$); 1,83 (s, 3H, $COCH_3$).

In Analogie zur Vorschrift des Beispiels XXIII werden die in Tabelle IX aufgeführten Verbindungen hergestellt:

**Tabelle IX:**

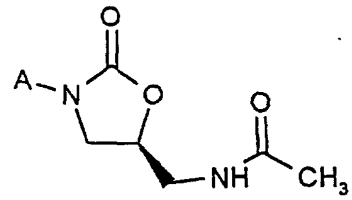

| Bsp.-Nr. | A | Ausbeute (% d.Th.) | Schmp. (°C) | $R_f$ (Laufmittel, Verhältnis) | $[\alpha]^{20}_D$ (DMSO) | MS (FAB) m/z (M+H) |
|---|---|---|---|---|---|---|
| XXIV | 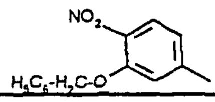 | 86 | 155-156 | 0,62 (VII, 1:1) | -23,6° (c=1,05) | 386 |
| XXV | 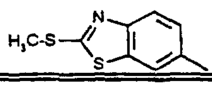 | 83 | 136 | 0,15 (I, 95:5) | | 338 |

**Beispiel XXVI**

(5S)-3-(2-Methylthio-3-methyl-benzothiazol-6-yl )-5-(acetylaminomethyl)-oxazolidin-2-on Iodid

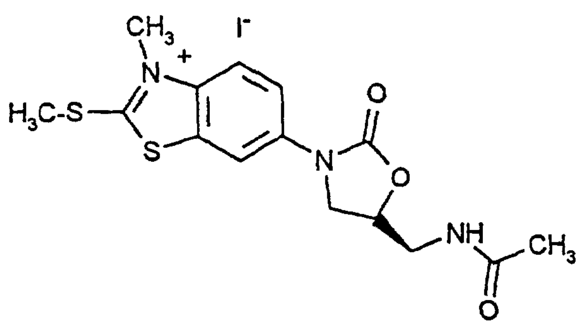

Eine gerührte Lösung von 1,35 g (4,00 mmol) der Verbindung aus Beispiel XXV in 6 ml wasserfreiem DMF wird mit 2,6 ml (40,00 mmol) Iodmethan versetzt und 23 h auf 70°C erhitzt. Danach darf die Reaktionsmischung abkühlen, man gibt 80 ml Ether zu und trennt den entstandenen Niederschlag durch Filtration ab. Nach Verrühren in 50 ml Ethanol, erneuter Filtration und Trocknen des Produkts im Hochvakuum über Sicapent erhält man 1,17 g (61%) der Titelverbindung als farblose Kristalle.
Schmp.: 149°C (Z)
MS (FAB) m/z = 352 (Kation $M^+$)
$^1$H-NMR (250 MHz, [$D_6$]DMSO): δ = 8,60 (d, J = 1 Hz, 1H, Benzothiazol H-7); 8,28 (m, 1H, NHCO); 8,20 (d, J = 10 Hz, 1H, Benzothiazol H-4); 8,02 (dd, J = 1 Hz, J = 10 Hz, 1H, Benzothiazol H-5); 4,82 (m, 1H, H-5); 4,20 (t, J = 10 Hz, 1H, H-4 cis); 4,10 (s, 3H, $NCH_3$); 3,85 (dd, J = 7 Hz, J = 10 Hz, 1H, H-4 trans); 3,46 (m, 2H, $CH_2N$); 3,12 (s, 3H, $SCH_3$); 1,85 (s, 3H, $COCH_3$).

**Beispiel XXVII**

(5S)-3-(3-Amino-4-hydroxyphenyl)-5-(acetylaminomethyl)-oxazolidin-2-on

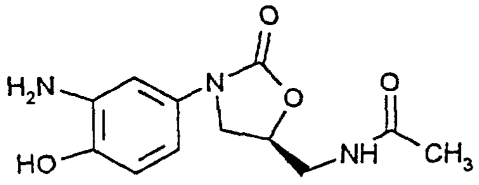

3,58 g (9,28 mmol) der Verbindung aus Beispiel XXIII und 350 mg Pd-C (10%) werden in 100 ml Methanol und 100 ml THF 3 h unter Wasserstoff (1 atm) gerührt. Es wird vom Katalysator abfiltriert, das Lösemittel abgezogen und getrocknet.
Ausbeute: 2,5 g (quant.)
$R_f$ (VII, 1:1) = 0,42
MS (CI): m/z = 265 ($M^+$)
$[\alpha]^{20}_D$ = -110,45 (c=1,0, DMSO)
$^1$H-NMR ([$D_6$]DMSO): δ = 9,0 - 9,5 (bs, 1H, OH); 8,20 (t, J = 4 Hz, 1H, NHCO); 7,05 (bs, 1H, Ph); 6,55 (bs, 2H, Ph); 4,55 - 4,70 (m, 1H, 5-H); 4,30 - 4,52 (bs, 2H, $NH_2$); 3,95 (t, J = 6 Hz, 1H, 4-H); 3,60 (dd, J= Hz, J = 4 Hz, 1H, 4-H); 3,40 (t, J = 4 Hz, 2H, $CH_2N$); 1,73 (s, 3H, $COCH_3$).
Wie für Beispiel XXVII beschrieben erhält man aus den entsprechenden Ausgangsverbindungen die in Tabelle X aufgeführten Verbindungen:

**Tabelle X:**

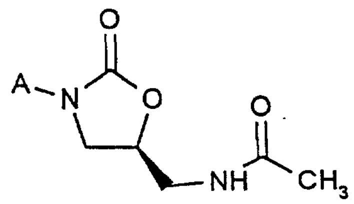

| Bsp.-Nr. | D | Ausbeute (% d.Th.) | Schmp. (°C) | $R_f$ (Laufmittel, Verhältnis) | $[\alpha]^{20}_D$ (DMSO) | MS (CDI, $NH_3$) m/z $(M+H)^+$ |
|---|---|---|---|---|---|---|
| XXVIII | 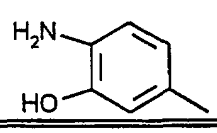 | quant. | 221-222 | 0,31 (VII, 1:1) | -19,89 (c=1,0) | 265 |

**Beispiel XXIX**

(5S)-3-(3-Hydroxy-4-(N-iso-propylamino)phenyl)-5-(acetylaminomethyl)oxazolidin-2-on

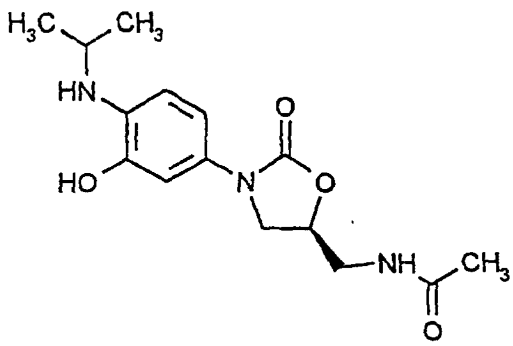

Zu einer Mischung aus 1,06 g (4,0 mmol) der Verbindung aus Beispiel XXVIII, 600 µl (8,0 mmol) Aceton und 50 ml THF werden bei 0°C 4,4 ml (4,4 mmol) einer 1 M Boran-Tetrahydrofuran-Komplex-Lösung in THF gegeben und weitere 24 h bei Raumtemperatur gerührt. Die entstandene Lösung wird mit 4 ml 1 M Natriumhydroxidlösung versetzt, getrocknet ($Na_2SO_4$) und das Lösemittel abgezogen.
Ausbeute: 1,23 g (quant.)
$R_f$ (I, 10:1) = 0,29
MS (EI):m/z = 307 ($M^+$)
$^1$H-NMR ([$D_6$]DMSO): δ = 9,50 (bs, 1H, OH), 8,25 (t, 1H, NHCO), 7,10 (d, 1H, Ar-2-H), 6,62 (dd, 1H, Ar-6-H), 6,45 (d, 1H, Ar-5-H), 4,65 (m, 1H, 5-H), 3,90-4,10 (m, 2H, ArNH, 4-H), 3,50-3,70 (m, 2H, CHN, 4-H), 3,40 (t, 2H, $CH_2N$), 1,70 (s, 3H, $COCH_3$), 1,10 (d, 6H, $CH_3$).

## Tabelle XI:

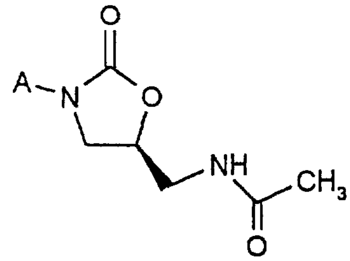

| Bsp.-Nr. | A | Ausbeute | $R_f$ (Laufmittel Verhältnis) | MS (DCI, $NH_3$) m/z ($M^+$+H) |
|---|---|---|---|---|
| XXX | 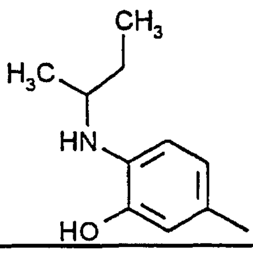 | quant. | 0,69 (I, 5:1) | 322 |
| XXXI | 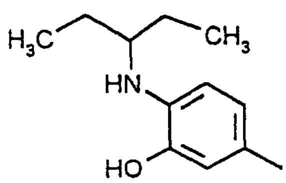 | quant. | 0,33 (I, 10:1) | 336 |
| XXXII | 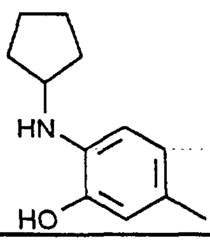 | quant. | 0,23 (I, 10:1) | 334 |
| XXXIII | 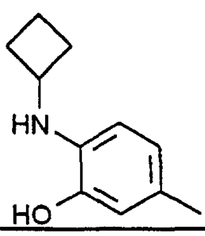 | quant. | 0,28 (I, 10:1) | 320 |
| XXXIV | 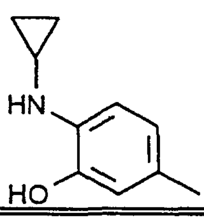 | 8 | 0,25 (I, 10:1) | 305 |

**Beispiel XXXV**

(5S)-3-(2-Imino-3-methyl-2,3-dihydrobenzoxazol-6-yl)-5-acetylaminomethyl)-oxazolidin-2-on

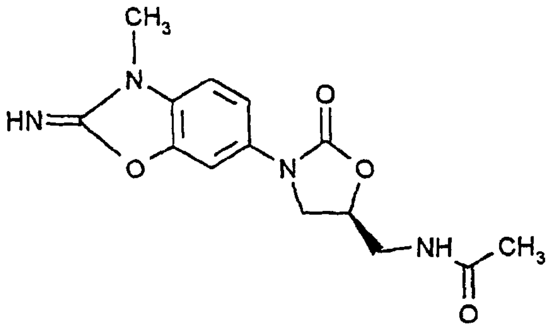

Eine Lösung aus 2 g (6,89 mmol) der Verbindung aus Beispiel XXVII in 30 ml Dimethylformamid wird mit 4,3 ml (69 mmol) Iodmethan versetzt und die Mischung 2 h bei 100°C gerührt. Das Lösemittel wird im Vakuum abgezogen, der Rückstand in Dichlormethan verrührt, abgesaugt und getrocknet.
Ausbeute: 2,32 g (78 %)
$R_f$ (VII, 1:1) = 0,10
MS (DCI):m/z = 305 ($M^+$+H)
$^1$H-NMR ([$D_6$]DMSO): δ = 10,0-10,5 (bs, 1H, HN=C), 8,25 (bt, 1H, NHCO), 7,95 (d, 1H, Ar-7-H), 7,62 (d, 1H, Ar-4-H), 7,55 (dd, 1H, Ar-5-H), 4,75 (m, 1H, 5-H), 4,18 (t, 1H, 4-H), 3,78 (dd, 1H, 4-H), 3,61 (s; 3H, $NCH_3$), 3,30-3,40 (m, 2H, $CH_2N$), 1,82 (s, 3H, $NCOCH_3$).

**Beispiel XXXVI**

3-Isopropyl-6-nitrobenzothiazol-2-on

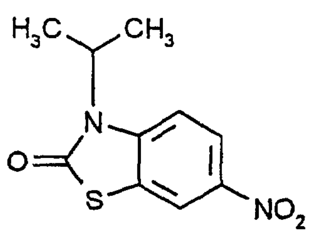

6-Nitrobenzothiazol-2-on (35,1 ml, 0,18 mol), Kaliumcarbonat (24,3 g, 0,18 mol) und 2-Iodopropan (153 g, 0,9 mol) in 2-Propanol (1 l) werden 24 Stunden unter Rückfluß erhitzt. Die abgekühlte Reaktionsmischung wird filtriert, das Lösungsmittel im Vakuum abgezogen, der Rückstand in Dichlormethan aufgenommen und mit Wasser gewaschen. Die organische Phase wird getrocknet ($Na_2SO_4$) und das Dichlormethan im Vakuum abgezogen. Das Rohprodukt wird durch Chromatographie (Kieselgel, Dichlormethan/Petrolether 2:1) gereinigt.
Ausbeute: 8,7 g (20 %)
Schmp.: 138 bis 142°C
$R_f$ (Dichlormethan) = 0,47
MS (CI): m/z = 256 (M+$NH_4$+)

**Beispiel XXXVII**

6-Amino-3-isopropylbenzothiazol-2-on

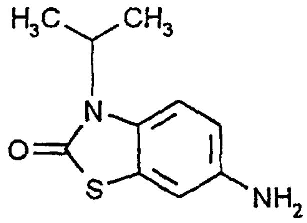

Die Verbindung aus Beispiel XXXVI (3,2 g, 138 mmol) wird in einer Mischung aus Ethanol (90 ml), Wasser (24 ml) und $CaCl_2$ (0,96 g, 8,65 mmol) suspendiert.

Das Reaktionsgemisch wird unter Rückfluß erhitzt, Zink-Staub (28,8 g, 0,42 mol) werden zugegeben und weitere 30 Minuten unter Rückfluß gerührt. Die Mischung wird heiß filtriert, der Rückstand gut mit Wasser gewaschen, das Filtrat eingeengt und der Rückstand in Ether kristallisiert.
Ausbeute: 2,8 g (97 %)
Schmp.: 138 bis 140°C
$R_f$ (Dichlormethan) = 0,19

In Analogie zur Vorschrift des Beispiels XXXVII werden die in Tabelle XII aufgeführten Verbindungen dargestellt:

Tabelle XII:

$A\text{-}NH_2$

| Bsp.-Nr. | A | Ausbeute (% d. Th.) | $R_f$ (Laufmittel, Verhältnis) |
|---|---|---|---|
| XXXVIII | 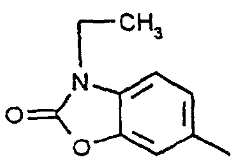 | quant. | 0,90 (VI, 10:1) |

In Analogie zur Vorschrift des Beispiels I werden die in der Tabelle XIII aufgeführten Verbindungen dargestellt:

Tabelle XIII:

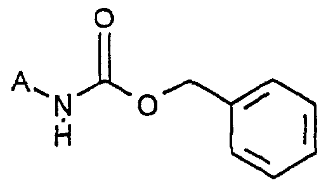

| Bsp.-Nr. | A | Ausbeute (% d. Th.) | Schmp. (°C) | $R_f$ (Laufmittel, Verhältnis) |
|---|---|---|---|---|
| XXXIX | 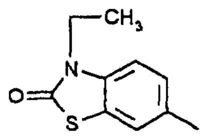 | 87 | 163 | 0,15 ($CH_2Cl_2$) |
| XL | 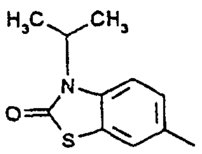 | quant. | 160 | 0,70 ($CH_2Cl_2$) |
| XLI | 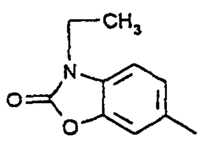 | 85 | - | 0,70 (VII, 95:5) |

In Analogie zur Vorschrift des Beispiels XXIII werden die in Tabelle XIV aufgeführten Verbindungen dargestellt:

Tabelle XIV:

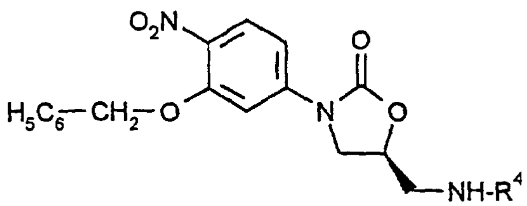

| Bsp.-Nr. | $R^4$ | Alkylierungs-mittel | Ausbeute (% d. Th.) | $R_f$ (Laufmittel, Verhältnis) | MS (CI) m/z ($M+NH_4^+$) |
|---|---|---|---|---|---|
| XLII | 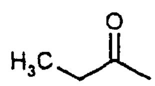 | 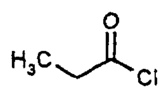 | 99 | 0,36 (I, 10:1) | 417 |
| XLIII | 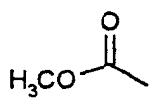 | 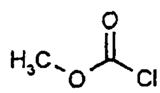 | 46 | 0,63 (I, 10:1) | 419 |
| XLIV | 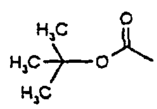 | $BOC_2O$ | 95 | 0,80 (I, 10:1) | 461 |

In Analogie zur Vorschrift des Beispiels XXVII werden die in der Tabelle XV aufgeführten Verbindungen dargestellt:

Tabelle XV:

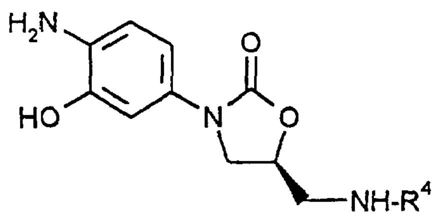

| Bsp.-Nr. | $R^4$ | Ausbeute (% d. Th.) | $R_f$ (Laufmittel, Verhältnis) | MS m/z |
|---|---|---|---|---|
| XLV | 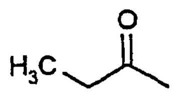 | quant. | 0,26 (I, 10:1) | 297 ($M+NH_4^+$) |
| XLVI | 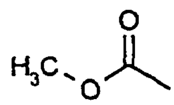 | 97 | 0,40 (I, 10:1) | 299 ($M+NH_4^+$) |
| XLVII | 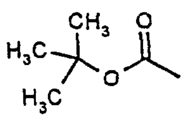 | quant. | 0,28 (I, 10:1) | 323 ($M^+$) |

In Analogie zur Vorschrift des Beispiels XXIX werden die in der Tabelle XVI aufgeführten Verbindungen dargestellt:

Tabelle XVI:

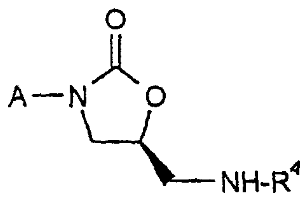

| Bsp.-Nr. | A | $R^4$ | Ausbeute (% d. Th.) | $R_f$ (Laufmittel, Verhältnis) | MS m/z |
|---|---|---|---|---|---|
| XLVIII | 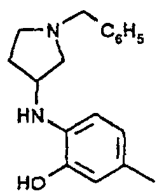 | 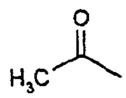 | 31 | - | - |
| XLIX | 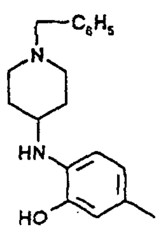 | 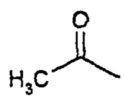 | 58 | 0,07 (I, 10:1) | 439 ($M+H^+$) |
| L | 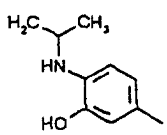 | 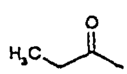 | 63 | 0,35 (I, 10:1) | 322 ($M+H^+$) |
| LI | 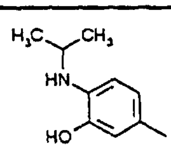 | 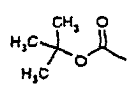 | 97 | 0,57 (I, 10:1) | - |
| LII | 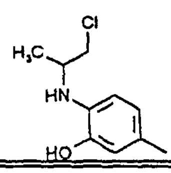 | 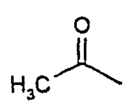 | 94 | 0,22 (I, 10:1) | 342 ($M+H^+$) |

**Herstellungsbeispiele**

**Beispiel 1**

(5R)-3-[3-Methyl-2-benzothiazolinon-6-yl]-5-(hydroxymethyl)-oxazolidin-2-on

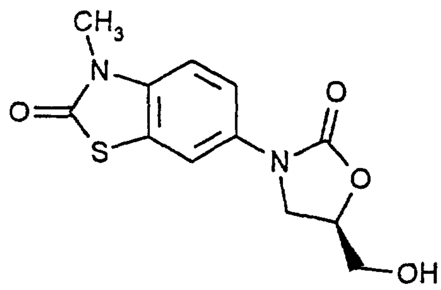

Methode A

26,76 g (85,12 mmol) der Verbindung aus Beispiel I werden in 400 ml THF gelöst, mit 10 mg 1,10-Phenanthrolin-Hydrat versetzt und auf -70°C gekühlt. Nun werden langsam ca. 34 ml 2,5 N n-Butyllithium-Lösung in Hexan bis zum Farbumschlag nach rot zugetropft. Anschließend werden 12 ml (85,12 mmol) (R)-Glycidylbutyrat zugetropft. Man läßt auf RT kommen, versetzt mit gesättigter Ammoniumchloridlösung und zieht im Vakuum das THF ab. Der entstandene Niederschlag wird abgesaugt, mit Wasser und Ether gewaschen und im Hochvakuum getrocknet.
Ausbeute: 17,93 g (75%)
Smp.: 166°C
$R_f$ (II, 1:1) = 0,09
MS (EI): m/z = 280 ($M^+$)
$^1$H-NMR ([$D_6$]DMSO): $\delta$ = 7,80 (d, J = 1 Hz, 1H, Benzothiazolinon 7-H); 7,60 (dd, J = 6, J = 1 Hz, 1H, Benzothiazolinon 5-H); 7,32 (d, J = 6 Hz, 1H, Benzthiazolinon 4-H); 5,23 (t, J = 6 Hz, 1H, OH); 4,62 - 4,80 (m, 1H, 5-H); 4,10 (t, J = 9 Hz, 1H, 4-H); 3,85 (dd, J = 9, J = 5 Hz, 1H, 4-H); 3,48 - 3,75 (m, 2H, $CH_2O$); 3,40 (s, 3H, $CH_3$).

Methode B

9,3 g (0,03 mol) der Verbindung aus Beispiel I werden in 150 ml THF gelöst und auf -70°C gekühlt. Anschließend werden 4 ml (0,01 mol) 2,5 M n-Butyllithiumlösung in Hexan zugetropft. Danach werden gleichzeitig langsam nochmals 8 ml (0,02 mol) n-Butyllithium und 4,23 ml (0,03 mol) (R)-Glycidylbutyrat zugetropft.
Man läßt auf Raumtemperatur kommen und rührt drei Stunden nach. Die Aufarbeitung erfolgt wie für Methode A beschrieben. Ausbeute: 6 g (72 %).
Wie für Beispiel 1, Methode A, beschrieben erhält man aus den entsprechenden Carbamaten die in Tabelle 1 aufgeführten Verbindungen:

**Tabelle 1:**

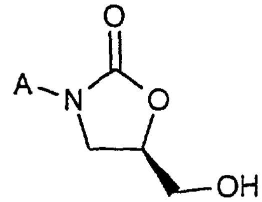

| Bsp.-Nr. | A | Ausbeute (% d.Th.) | Schmp. (°C) | Rf (Laufmittel, Verhältnis) | MS (CI) m/z $(M^+ + H)$ |
|---|---|---|---|---|---|
| 2 | 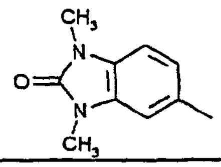 | 55 | 197 | 0,15 (I, 95:5) | 277 |
| 3 | 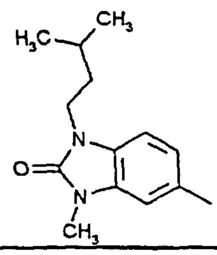 | 43 | 122 | 0,19 (I, 95:5) | 333 |
| 4 | 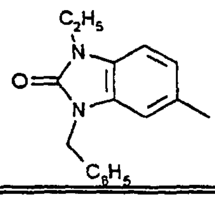 | 72 | 149 | 0,15 (I, 95:5) | 368 |

**Beispiel 5**

(5R)-3-(3-Methyl-2-benzothiazolinon-6-yl)-5-(methansulfonyloxymethyl)oxazolidin-2-on

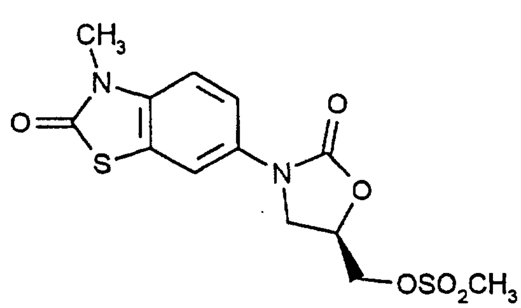

Eine auf 0°C gekühlte, gerührte Lösung von 18,72 g (66,78 mmol) der Verbindung aus Beispiel I und 13 ml (93,5 mmol) Triethylamin in 180 ml wasserfreiem Dichlormethan wird langsam mit 6,7 ml (86,82 mmol) Methansulfonsäurechlorid versetzt. Man rührt 20 min bei 0°C, weitere 5 h bei Raumtemperatur, saugt den entstandenen Niederschlag

ab, wäscht mit Wasser und Ether und trocknet im Hochvakuum.

Ausbeute: 21,45 g (89%)

Smp.: 172°C

$R_f$ (I, 95:5) = 0,27

MS (FAB): m/z = 359 ($M^+$)

$^1$H-NMR ([$D_6$]DMSO): δ = 7,78 (d, J = 1 Hz, 1H, Benzothiazolinon 7-H); 7,68 (dd, J = 6 Hz, J = 1 Hz, 1H, Benzothiazolinon 5-H); 7,35 (d, J = 6 Hz, 1H, Benzothiazolinon 4-H); 4,90 - 5,10 (m, 1H, 5-H); 4,40 - 4,60 (m, 2H, $CH_2O$); 4,20 (t, J = 9 Hz, 1H, 4-H); 3,85 (dd, J = 9 Hz, J = 5 Hz, 1H, 4-H); 3,40 (s, 3H, 4-$NCH_3$); 3,20 (s, 3H, $SO_2CH_3$).

Wie für Beispiel 5 beschrieben, erhält man aus den entsprechenden Alkoholen die in der Tabelle 2 aufgeführten Methansulfonate.

**Tabelle 2:**

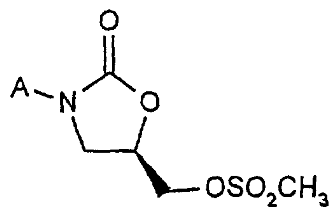

| Bsp.-Nr. | A | Ausbeute (% d.Th.) | Schmp. (°C) | $R_f$ (Laufmittel, Verhältnis) | MS (FAB) m/z ($M^+$ + H) |
|---|---|---|---|---|---|
| 6 | 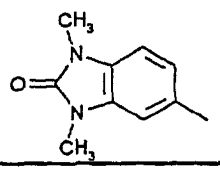 | 78 | 188 | 0,25 (I, 95:5) | 355 a) |
| 7 | 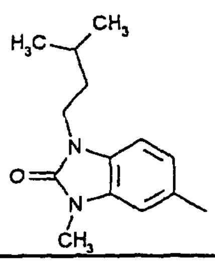 | 76 | | 0,32 (I, 95:5) | 411 a) |
| 8 | 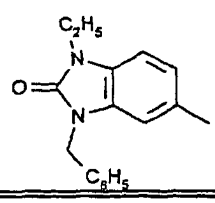 | 67 | 187 | 0,16 (II, 1:1) | 446 |

a) MS (EI), m/z (M)

**Beispiel 9**

(5R)-3-(3-Methyl-2-benzothiazolinon-6-yl)-5-(azidomethyl)-oxazolidin-2-on

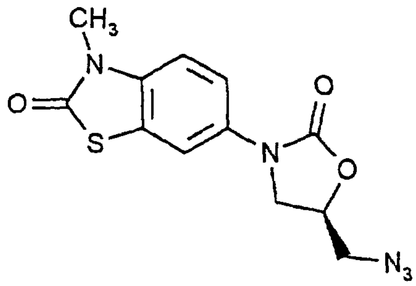

Eine Lösung von 17,03 g (47,51 mmol) der Verbindung aus Beispiel 5 in 58 ml wasserfreiem DMF wird mit 4,02 g (61,77 mmol) Natriumazid versetzt und 5 h bei 70°C gerührt. Man läßt auf Raumtemperatur abkühlen und rührt 100 ml Eiswasser ein. Der entstandene Niederschlag wird abfiltriert, mit Wasser und Petrolether gewaschen und im Vakuum getrocknet.
Ausbeute: 12,8 g (88%)
Smp.: 129°C
$R_f$ (I, 95:5) = 0,40
MS (EI): m/z = 305 ($M^+$)
$^1$H-NMR ([$D_6$]DMSO): δ = 7,85 (d, J = 1 Hz, 1H, Benzothiazolinon 7-H); 7,57 (dd, J = 6 Hz, J = 1 Hz, Benzothiazolinon 5-H); 7,34 (d, J = 6 Hz, 1H, Benzothiazolinon 4-H); 4,82 - 5,00 (m, 1H, 5-H); 4,15 (t, J = 9 Hz, 1H, 4-H); 3,65 - 3,77 (m, 3H, 4-H, $CH_2N_3$); 3,41 (s, 3H, $NCH_3$).

Wie für Beispiel 9 beschrieben erhält man aus den entsprechenden Methansulfonaten die in der Tabelle 3 aufgeführten Azide:

**Tabelle 3:**

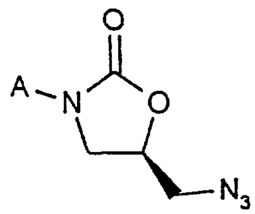

| Bsp.-Nr. | A | Ausbeute (% d.Th.) | Schmp. (°C) | $R_f$ (Laufmittel, Verhältnis) | MS (FAB) m/z (M+ + H) |
|---|---|---|---|---|---|
| 10 | 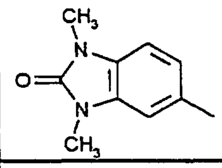 | 87 | 179 | 0,33 (I, 95:5) | 302 [a)] |
| 11 | 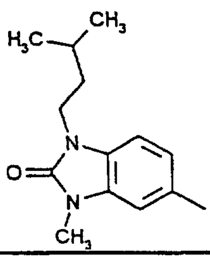 | 78 | | 0,36 (I, 97:3) | 358 [a)] |
| 12 | 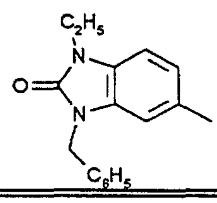 | 90 | 120 | 0,51 (IV) | 393 |

a) MS (EI), m/z ($M^+$)

**Beispiel 13**

(5S)-3-(3-Methyl-2-benzothiazolinon-6-yl)-5-(aminomethyl)-oxazolidin-2-on Hydrochlorid

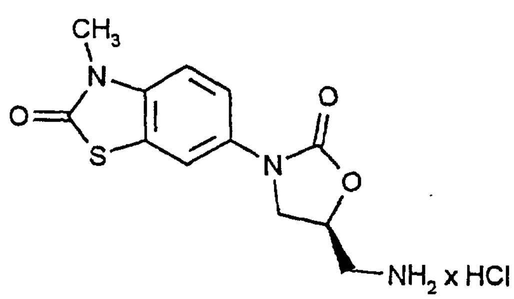

Eine gerührte Lösung von 12,75 g (41,76 mmol) der Verbindung aus Beispiel 9 in 30 ml 1,2-Dimethoxyethan wird auf 50°C erwärmt. Man tropft langsam 5,7 ml (50,11 mmol) Trimethylphosphit zu (Gasentwicklung) und rührt nach

beendeter Zugabe 2 h bei 90°C nach. Nun tropft man 8,4 ml 6 N HCl zu und rührt nochmals 3 h bei 90°C nach. Man läßt auf Raumtemperatur abkühlen, trennt den Niederschlag durch Filtration ab, wäscht mit 1,2-Dimethoxyethan und trocknet im Hochvakuum über $P_2O_5$.
Ausbeute: 8,86 g (75%)
Smp.: 259°C (Zers.)
$R_f$ (III, 95:5) = 0,09
MS (EI): m/z = 279 ($M^+$)
$^1$H-NMR ([$D_6$]DMSO): δ = 8,5 (bs, 3H, NH); 7,85 (d, J = 1 Hz, 1H, Benzothiazolinon 7H); 7,65 (dd, J = 6 Hz, J = 1 Hz, 1H, Benzothiazolinon 5-H); 7,34 (d, J = 6 Hz, 1H, Benzothiazolinon 4-H); 4,90 - 5,10 (m, 1H, 5-H); 4,23 (t, J = 9 Hz, 1H, 4-H); 4,42 (dd, J = Hz, J = 5 Hz, 1H, 4-H); 3,40 (s, 3H, $NCH_3$); 3,15 - 3,35 (m, 2H, $CH_2N$).

Wie für Beispiel 13 beschrieben erhält man aus den entsprechenden Aziden die in der Tabelle 4 aufgeführten Aminhydrochloride:

## Tabelle 4:

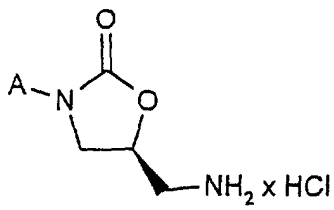

| Bsp.-Nr. | A | Ausbeute (% d.Th.) | Schmp. (°C) | $R_f$ (Laufmittel, Verhältnis) | MS (FAB) m/z ($M^+$ + H) |
|---|---|---|---|---|---|
| 14 | 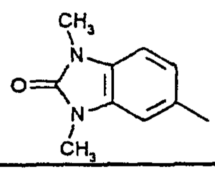 | 92 | 272 (Z) | 0,33 (III, 8:2) | 276 a) |
| 15 | 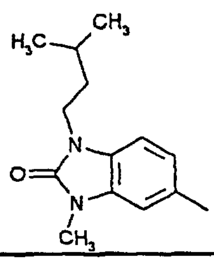 | 83 | (öl) | 0,12 (III, 9:1) | 333 |
| 16 | 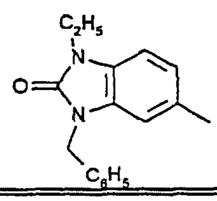 | 95 | (Öl) | 0,5 (III, 8:2) | - |

a) MS (EI), m/z ($M^+$)

**Beispiel 17**

(5R)-3-[3-Methyl-2-benzothiazolinon-6-yl)-5-(dimethoxyphosphonaminomethyl)oxazolidin-2-on

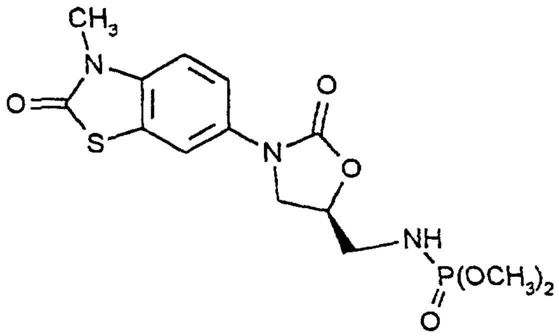

Eine Lösung aus 164 mg (0,5 mmol) der Verbindung aus Beispiel 9 in 3 ml 1,2-Dimethoxyethan wird auf 50°C erwärmt und langsam 0,7 ml (0,55 mmol) Trimethylphosphit zugetropft. Nach beendeter Zugabe rührt man weitere 2 h bei 90°C, zieht anschließend die Lösungsmittel ab und kristallisiert den Rückstand zweimal aus Ethanol.
Ausbeute: 32 mg (20%)
Smp.: 169°C
$R_f$ (I, 95.5) =0,15
MS (FAB): m/z = 388 ($M^+$+H)
$^1$H-NMR ([$d_6$]DMSO): δ = 7,82 (d, J = 1 Hz, 1H, Benzthiazolinon 7-H); 7,57 (dd, J = 6 Hz, J = 1 Hz, 1H, Benzthiazolinon 5-H); 7,35 (d, J = 6 Hz, 1H, Benzthiazolinon 4-H); 5,30 - 5,50 (m, 1H, PNH); 4,60 - 4,80 (m, 1H, 5-H); 4,10 (t, J = 7 Hz, 1H, 4-H); 3,90 (dd, J = 7 Hz, J = 4 Hz, 1H, 4-H); 3,60 (d, J = 11 Hz, 3H, $POCH_3$); 3,55 (d, J = 11 Hz, 3H, $POCH_3$); 3,40 (s, 3H, $NCH_3$).

**Beispiel 18**

(5S)-3-(3-Methyl-2-benzothiazolinon-6-yl)-5-(acetylaminomethyl)oxazolidin-2-on

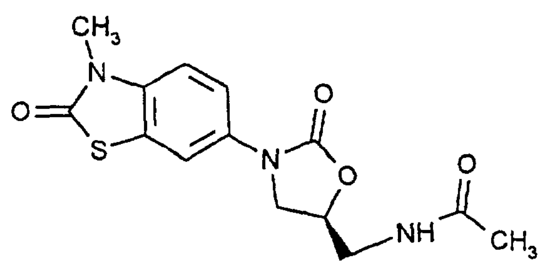

Methode A:

Eine gerührte Lösung von 8,55 g (27,07 mmol) der Verbindung aus Beispiel 13 in 80 ml THF wird in einer Lösung von 1,09 g (27,34 mmol) Natriumhydroxid in 8 ml Wasser versetzt. Dazu tropft man bei 0-5°C langsam 2,81 ml (29,78 mmol) Acetanhydrid in 6 ml THF und hält pH = 9 durch gleichzeitige Zugabe einer 5 N wäßrigen NaOH-Lösung. Man rührt 1 h bei 0°C nach und dampft das THF im Vakuum ab. Der Niederschlag wird abgesaugt, mit Wasser und Ether gewaschen, und im Hochvakuum über $P_2O_5$ getrocknet.
Ausbeute: 8,39 g (96%)
Smp.: 208°C
$R_f$ (I, 95:5) = 0,21

MS (DCI, $NH_3$) m/z = 322 $(M+H)^+$
$^1$H-NMR ([$D_6$]DMSO): δ = 8,24 (t, J = 4 Hz, 1H, NH); 7,85 (d, J = 1 Hz, 1H, Benzthiazolinon 7-H); 7,55 (dd, J = 6 Hz, J = 1 Hz, 1H); Benzthiazolinon 5-H); 7,32 (d, J = 6 Hz, 1H, Benzthiazolinon 4-H); 4,55 - 4,80 (m, 1H, 5-H); 4,15 (t, J = 9 Hz, 1H, 4-H); 3,67 (dd, J = 9 Hz, J = 5 Hz, 1H, H-4); 3,35 - 3,50 (m, 5H, $CH_2N$, $NCH_3$); 1,83 (s, 3H, $COCH_3$).

Methode B:

1,10 g (2,30 mmol) (5S)-3-(2-Methylthio-3-methyl-benzo[4,5-d]-thiazol-6-yl)-5-acetylaminomethyl-oxazolidin-2-on Iodid (Beispiel XXVI) werden in 24 ml eines Gemisches aus Dichlormethan : Methanol 4:1 gelöst. Man gibt 1,5 g Kieselgel zu und rührt 1 h bei Raumtemperatur nach. Dann gibt man 6 ml Methanol zu und dampft das Lösemittel im Vakuum ab. Der Rückstand wird auf eine Säule mit 100 g Kieselgel gegeben und mit Dichlormethan : Methanol 95:5 eluiert. Die produkthaltigen Fraktionen werden gesammelt, das Lösemittel wird im Vakuum abgedampft und der Rückstand aus Ethanol umkristallisiert. Man erhält 343 mg (46%) der Titelverbindung. Die physikalischen Daten sind identisch mit der nach Methode A erhaltenen Verbindung.

In Analogie zum Beispiel 18 erhält man die in der Tabelle 5 aufgeführten Acetamide.

## Tabelle 5:

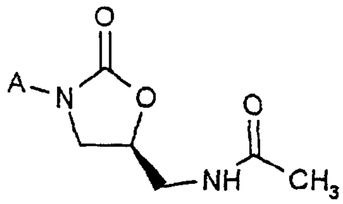

| Bsp.-Nr. | A | Methode | Ausbeute (% d.Th.) | Schmp. (°C) | $R_f$ (Laufmittel, Verhältnis) | MS (FAB) m/z ($M^+$ + H) |
|---|---|---|---|---|---|---|
| 19 | 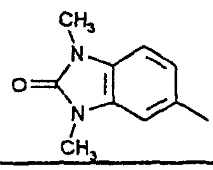 | A | 49 | 224 (Z) | 0,36 (I, 95:5) | 318 a) |
| 20 | 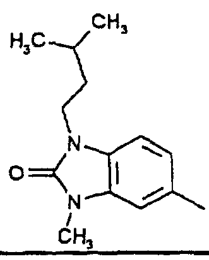 | A | 37 | 142 | 0,41 (I, 9:1) | 374 a) |
| 21 | 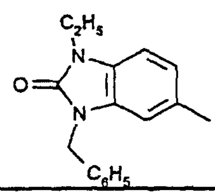 | A | 57 | 129-132 | 0,69 (III, 8:2) | 409 |
| 22 | 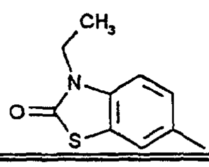 | B | 5 | / | 0,12 (I, 95:5) | 335 a) |

a) MS (EI), m/z ($M^+$)

**Beispiel 23**

(5S)-3-(1-Ethyl-2-benzimidazolon-6-yl)-5-(acetylaminomethyl)-2-oxazolidinon

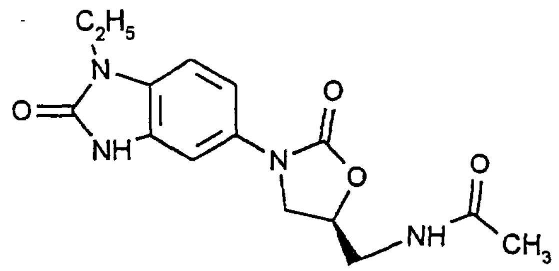

3 g (7,35 mmol) der Verbindung aus Beispiel 21 werden in 60 ml $NH_3$ bei -40°C vorgelegt. Man gibt unter DC-Kontrolle ca. 360 mg (15 mmol) Natrium zu. Bei vollständigem Umsatz wird gesättigte Ammoniumchloridlösung zugegeben und der Ammoniak über Nacht abgedampft. Das erhaltene Rohprodukt wird an Kieselgel chromatographiert.

| | |
|---|---|
| Ausbeute | 1,5 g (64 % der Theorie) |
| Fp | 80 bis 85°C |
| FAB | 319 |
| $R_F$ | 0,35 (I, 9:1) |

**Beispiel 24**

(5S)-3-(2-Benzoxazolinon-6-yl)-5-(acetylaminomethyl)-oxazolidin-2-on

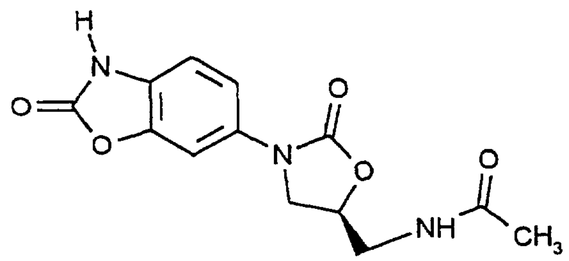

1 g (3,76 mmol) der Verbindung aus Beispiel XXVII und 0,67 g (4,14 mmol) Carbonyldiimidazol in 10 ml wasserfreiem DMF werden 8 h bei Raumtemperatur gerührt. Das Lösemittel wird im Vakuum abgezogen, der Rückstand in Dichlormethan verrührt, abgesaugt und getrocknet.
Ausbeute: 0,86 g (78%)
Smp.: 219-220°C (Z)
$R_f$ (VII; 1:1) = 0,53
$[\alpha]_D^{20}$ = -23,213 (c = 1,0, DMSO)
MS (FAB): m/z = 292 ($M^+$+H)
$^1$H-NMR ([$D_6$]DMSO): δ = 11,4 - 11,8 (bs, 1H, NH); 8,23 (t, J = 4 Hz, 1H, NHCO); 7,55 (d, J = 1 Hz, 1H, Benzoxazolinon 7-H); 7,20 (dd, J = 6 Hz, J = 1 Hz, 1H, Benzoxazolinon); 7,10 (d, J = 6 Hz, 1H, Benzoxazolinon 4-H); 4,60 - 4,80 (m, 1H, 5-H); 4,10 (t, J = 6 Hz, 1H, 4-H); 3,72 (dd, J= 7 Hz, J = 4 Hz,, 1H, 4-H); 3,40 (t, J = 3 Hz, 2H, $H_2CN$); 1,82 (s, 3H, $COCH_3$).

In Analogie zur Vorschrift des Beispiels 24 werden die in der Tabelle 6 aufgeführten Verbindungen hergestellt:

**Tabelle 6:**

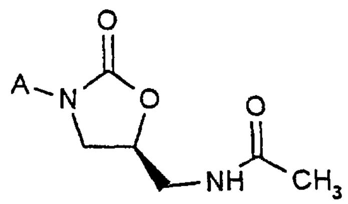

| Bsp.-Nr. | A | Ausbeute (% d.Th.) | Schmp. (°C) | $R_f$ (Laufmittel, Verhältnis) | $[\alpha]^{20}_D$ (DMSO) | MS (FAB) m/z ($M^+$ + H) |
|---|---|---|---|---|---|---|
| 25 | 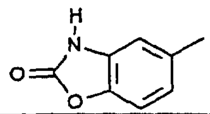 | 22 | 169 (Z) | 0,33 (VII, 2:1) | -16.4 (c=1) | 292 |

**Beispiel 26**

(5S)-3-(2-Mercaptobenzoxazol-6-yl)-5-(acetylaminomethyl)oxazolidin-2-on

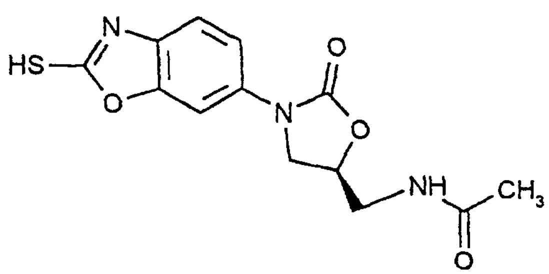

276 mg (1,04 mmol) der Verbindung aus Beispiel XXVIII und 184 mg (1,14 mmol) Kalium-O-ethyldithiocarbonat in 6 ml Ethanol werden 8 h bei 70°C gerührt. Anschließend werden 30 ml Wasser und 30 ml Essigester hinzugegeben, die organische Phase abgetrennt, die wäßrige Phase mit Essigester extrahiert, die vereinigten organischen Phasen mit ges. NaCl-Lösung gewaschen, getrocknet ($Na_2SO_4$) und die Lösemittel abgezogen. Der Rückstand wird aus Methanol umkristallisiert.
Ausbeute: 102 mg (31%)
Smp.: 239°C (Z)
$R_f$ (VIII, 1:1) = 0,41
$[\alpha]^{20}_D$ = -25,15 (c=1,0, DMSO)
MS (CI): m/z = 307 ($M^+$)
$^1$H-NMR ([$D_6$]DMSO): δ = 8,25 (t, 1H, J = 4 Hz, NHCO); 7,75 (d, J = 1 Hz, 1H, Benzoxazol 7-H); 7,45 (dd, J = 6 Hz, J = 1 Hz, 1H, Benzoxazol 5-H); 7,25 (d, J = 6 Hz, 1H, Benzoxazol 4-H); 4,65 - 4,82 (m, 1H, 5-H); 4,15 (t, J = 6 Hz, 1H, 4-H); 3,75 (dd, J = 7 Hz, 4 Hz, 1H, 4-H); 3,45 (t, J = 4 Hz, 2H, $H_2CN$); 3,10 - 3,40 (bs, 1H); 1,85 (s, 3H, $COCH_3$).

In Analogie zur Vorschrift des Beispiels 26 werden die in der Tabelle 7 aufgeführten Verbindungen hergestellt:

Tabelle 7:

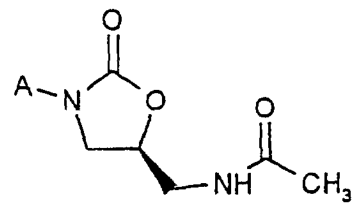

| Bsp.-Nr. | A | Ausbeute (% d.Th.) | Schmp. (°C) | $R_f$ (Laufmittel, Verhältnis) | MS (CI) m/z ($M^+$ + H) |
|---|---|---|---|---|---|
| 27 | 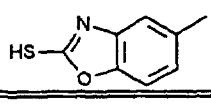 | 33 | >250 | 0,55 (VII, 1:1) | 307 |

**Beispiel 28**

(5S)-3-(2-Mercapto-benzothiazol-6-yl)5-(acetylaminomethyl)oxazolidin-2on

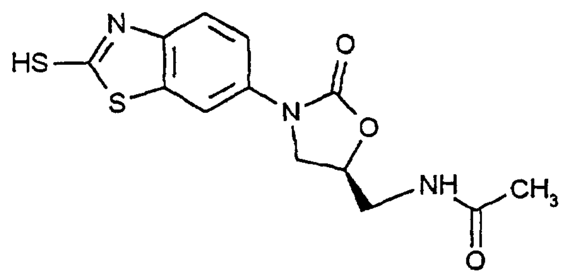

Eine Lösung von 500 mg (1,21 mmol) (5S)-3-(2-Benzylthio-benzo[4,5-d]thiazol-6-yl)-5-acetylaminomethyl-oxazolidin-2-on in 2,5 ml Trifluoressigsäure und 0,56 ml Thioanisol wird 42 h auf 60°C erwärmt. Das Gemisch darf abkühlen, man versetzt mit 25 ml Ether, trennt den Niederschlag durch Filtration ab, wäscht mit 5 ml Ether und trocknet im Hochvakuum. Man erhält 59 mg (15%) der Titelverbindung als Feststoff.
Schmp.: 161°C
$R_f$ = 0,24 (Dichlormethan : Methanol 92:8)
MS (DCI, $NH_3$): m/z = 324 $(M+H)^+$
$^1$H-NMR (250 MHz, $D_6$-DMSO): $\delta$ = 13,73 (bs, 1H, 5H); 8,24 (m, 1H, NH); 7,86 (d, J = 1 Hz, 1H, Benzothiazol H-7); 7,63 (dd, J = 1, 10 HZ, 1H, Benzothiazol H-5); 7,30 (d, j = 10 Hz, 1H, Benzothiazol H-4); 4,74 (m, 1H, H-5); 4,11 (dd, J = 9, 9 Hz, 1H, H-4 cis); 3,76 (dd, J = 7, 9 Hz, 1H, H-4 trans); 3,42 (t, J = 6 Hz, 2H, $CH_2N$); 1,84 (s, 3H, $COCH_3$).

**Beispiel 29**

(5S)-3-(2-Aminobenzoxazol-6-yl)-5-(acetylaminomethyl)oxazolidin-2-on

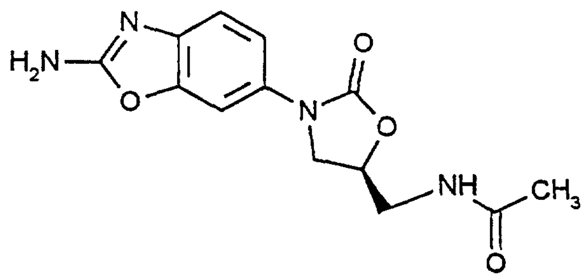

Zu einer Lösung aus 253 mg (2,41 mmol) Bromcyan in 2,5 ml Methanol und 2,5 ml Wasser werden 553 mg (2,19 mmol) der Verbindung aus Beispiel XXVIII in 10 ml Methanol gegeben und das Reaktionsgemisch 20 h bei Raumtemperatur gerührt. Das Methanol wird im Vakuum abgezogen, der ausgefallene Niederschlag abfiltriert, mit Wasser gewaschen und im Hochvakuum getrocknet.
Ausbeute: 393 mg (62%)
Smp.: 237°C
$R_f$ (VII, 1:1) = 0,4
MS (EI): m/z = 290 ($M^+$)
$^1$H-NMR ([$D_6$]DMSO): $\delta$ = 8,25 (t, J = 4 Hz, 1H, NHCO); 7,62 (bs, 1H, Ph); 7,50 (bs, 2H, $NH_2$); 7,30 (bs, 1H, Ph); 7,15 (bs, 1H, 7H); 4,60 - 4,78 (m, 1H, 5-H); 4,12 (Z, J= 7 Hz, 1H, 4-H); 3,70 (dd, J = 7 Hz, J = 4 Hz, 1H, 4-H); 3,35 - 3,45 (m, 2H, $CH_2N$); 1,80 (s, 3H, $CH_3CO$).
Analog Beispiel 29 werden die in Tabelle 8 aufgeführten Verbindungen hergestellt:

**Tabelle 8:**

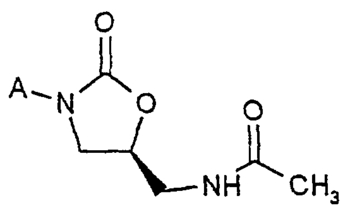

| Bsp.-Nr. | A | Ausbeute (% d.Th.) | Schmp. (°C) | $R_f$ (Laufmittel, Verhältnis) | MS (EI) m/z ($M^+$ -CI) |
|---|---|---|---|---|---|
| 30 | 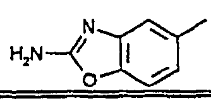 | 56 | 219-220 | 0,42 (II, 1:1) | 290 |

**Beispiel 31**

(5S)-3-(2-Aminobenzoxazol-6-yl)-5-(acetylaminomethyl)oxazolidin-2-on Hydrochlorid

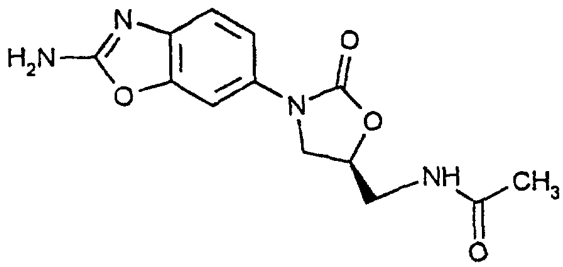

x HCl

Zu einer Lösung aus 150 mg (0,52 mmol) der Verbindung aus Beispiel 29 in 35 ml Methanol werden 2,58 ml (2,58 mmol) 1 N HCl in Ether und anschließend 130 ml Ether gegeben. Der ausgefallene Niederschlag wird abgesaugt, mit Ether gewaschen und im Hochvakuum getrocknet.
Ausbeute: 170 mg (89%)
Schmp.: 226-227°C
$^1$H-NMR ([$D_6$]-DMSO) $\delta$ = 8,8 - 9,2 (bs, 1H, NH); 8,28 (t, J = 4 Hz, 1H, NHCO); 7,80 (s, 1H, Ph); 7,20 - 7,35 (m, 2H, Ph); 4,5 - 5,0 (m, 3H, 5-H, $NH_2$); 4,15 (t, J = 7 Hz, 1H, 4-H); 3,73 (dd, J = 7 Hz, J = 4 Hz, 1H, 4-H); 3,40 (t, J = 4 Hz, 2H, $CH_2N$); 1,80 (s, 3H, $CH_3CO$).

Analog Beispiel 31 werden die in Tabelle 9 aufgeführten Verbindungen dargestellt:

**Tabelle 9:**

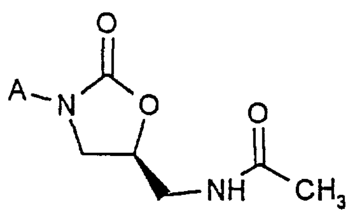

x HCl

| Bsp.-Nr. | A | Ausbeute (% d.Th.) | Schmp. (°C) |
|---|---|---|---|
| 32 | 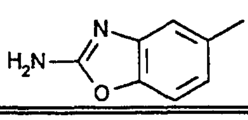 | 47 | 220 |

**Beispiel 33**

(5S)-3-(3-Acetyl-2-benzoxazolinon-6-yl)-5-(acetylaminomethyl)-oxazolidin-2-on

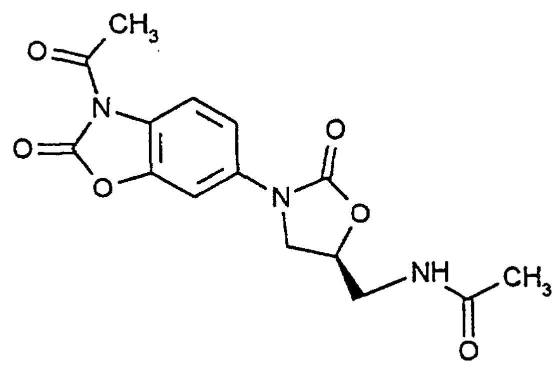

Zu einer Lösung aus 200 mg (0,68 mmol) der Verbindung aus Beispiel 23 in 10 ml DMF werden 16,5 ml (0,68 mmol) Natriumhydrid (80% in Paraffin) gegeben und das Reaktionsgemisch 15 min bei Raumtemperatur gerührt. Bei 0°C werden anschließend 50 µl (54 mg, 0,68 mmol) Acetylchlorid zugetropft und weitere 15 h bei 0°C gerührt. Zur Aufarbeitung wird das Lösemittel im Vakuum abgezogen, das Rohprodukt in Essigester und Wasser aufgenommen, die wäßrige Phase dreimal mit Essigester extrahiert, die vereinigten organischen Phasen mit gesättigter NaCl-Lösung gewaschen, getrocknet ($Na_2SO_4$), eingeengt und aus Methanol umkristallisiert.
Ausbeute: 54 mg (23%)
$R_f$ (VII, 1:1= = 0,62
MS (EI): m/z = 333 ($M^+$)
$^1$H-NMR ([$D_6$]DMSO): δ = 8,25 (t, J = 4 Hz, 1H, NHCO); 7,90 (d, J = 6 Hz, 1H, Benzoxazolin 4-H); 7,72 (d, J = 1 Hz, 1H, Benzoxazolin 7-H); 7,33 (dd, J = 6 Hz, J = 1 Hz, Benzoxazolin 5-H); 4,60 - 4,80 (m, 1H, 5-H); 4,15 (t, J= 6 Hz, 1H, 4-H); 3,73 (dd, J = 7 Hz, 4 Hz, 1H, 4-H); 3,55 (t, J = Hz, 2H, $CH_2N$)); 2,60 (s, 3-H, $CH_3CO$); 1,80 (s, 3H, $CH_3CON$).

In Analogie zur Vorschrift des Beispiels 33 werden die in Tabelle 10 aufgeführten Verbindungen hergestellt:

## Tabelle 10:

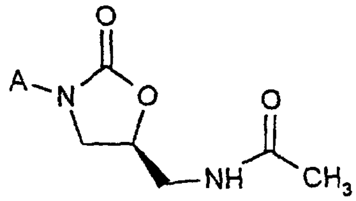

| Bsp.-Nr. | A | Ausbeute (% d.Th.) | Schmp. (°C) | $R_f$ (Laufmittel, Verhältnis) | $[\alpha]^{20}_D$ (DMSO) | MS (FAB) m/z ($M^+$ + H) |
|---|---|---|---|---|---|---|
| 34 | 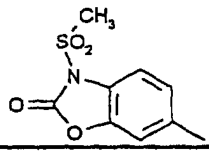 | 49 | 224-225 | 0,25 (VII, 5:1) | -17.7° (c=0,5) | 370 |
| 35 | 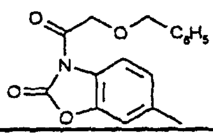 | 54 | 220-222 | 0,26 (VII, 5:1) | -23,2° (c=0,5) | 440 |
| 36 | 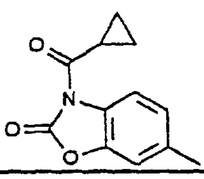 | 82 | 223-224 | 0,36 (VII, 5:1) | -22,6° (c=0,5) | 359 a) |
| 37 | 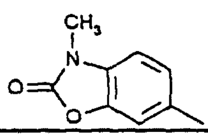 | 53 | 243-244 | 0,20 (VII, 5:1) | -30,6° (c=0,5) | 306 |
| 38 | 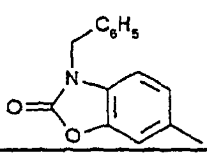 | 77 | 247-248 | 0,29 (VII, 1:1) | -19,9° (c=0,5) | 381 a) |
| 39 | 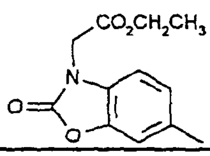 | 57 | 197-198 | 0,22 (VII, 5:1) | -23,0° (c=0,5) | 378 |

| Bsp.-Nr. | A | Ausbeute (% d.Th.) | Schmp. (°C) | $R_f$ (Laufmittel, Verhältnis) | $[\alpha]^{20}_D$ (DMSO) | MS (FAB) m/z ($M^+$ + H) |
|---|---|---|---|---|---|---|
| 40 | 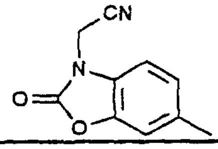 | 29 | 210-212 | 0,25 (VII, 5:1) | -19,2° (c=1,0) | 331 |
| 41 | 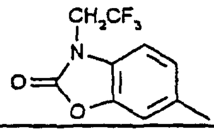 | 82 | - | 0,60 (VII, 1:1) | - | 374 |
| 42 | 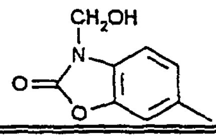 | 85 | 230-231 (Z) | 0,53 (VII, 1:1) | -20,7° (c=1,0) | 322 |

a) MS (EI), m/z ($M^+$)

**Beispiel 43**

(5S)-3-(3-Methyl-2-benzoxazolinthion-6-yl)-5-acetylaminomethyl)-oxazolidin-2-on

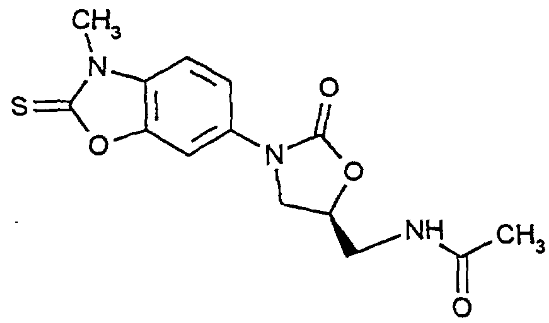

767,9 mg (2,9 mmol) der Verbindung aus Beispiel XXVIII und 511 mg (3,2 mmol) Kalium-O-ethyldithiocarbonat in 15 ml Ethanol werden 8 h bei 70°C gerührt. Anschließend wird das Lösemittel abgezogen, der Rückstand mit 20 ml DMF und 410 mg (28,9 mmol) Methyliodid versetzt und 20 h bei 150°C gerührt. Nach dem Abkühlen gibt man 40 ml $CH_2Cl_2$ hinzu, saugt den Niederschlag ab, wäscht mit $CH_2Cl_2$ und verkocht anschließend mit Methanol. Der Rückstand wird im Hochvakuum getrocknet.
Ausbeute: 602 mg (65%)
$R_f$ (VII, 1:1) = 0,44
MS (CI): m/z = 322 ($M^+$)
$^1H$-NMR ($[D_6]$DMSO: $\delta$ = 8,25 (t, J = 4 Hz, 1H, NHCO); 7,82 (s, 1H, Ph); 7,50 (s, 2H, Ph); 4,65 - 4,85 (m, 1H, 5-H); 4,15 (t, J = Hz, 1H, 4-H); 3,25 (dd, J = 7 Hz, J = 4 Hz, 1H, 4-H); 3,14 (s, 3H, $NCH_3$); 3,40 - 3,50 (m, 2H, $CH_2N$); 1,82 (s, 3H, $CH_3CO$).

**Beispiel 44**

(5S)-3-(3-Ethyl-benzothioazolinthion-6-yl)-5-(acetylaminomethyl)oxazolidin-2-on

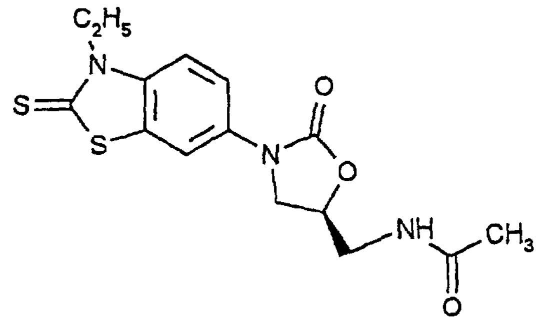

Eine gerührte Lösung von 303 mg (0,90 mmol) (5S)-3-(2-Methylthio-benzo[4,5-d]thiazol-6-yl)-5-(acetylaminomethyl)-oxazolidin-2-on (Beispiel XXVI) in 3 ml wasserfreiem DMF wird mit 0,72 ml (9,00 mmol) Iodethan versetzt und 23 h auf 100°C (Badtemperatur) erhitzt. Das Reaktionsgemisch darf abkühlen, man gibt 30 ml Ether zu und trennt den entstandenen honigartigen Niederschlag durch Dekandieren ab. Nach chromatographischer Reinigung an 58 g Kieselgel (Dichlormethan : Methanol 95:5) erhält man 74 mg (25%) der Titelverbindung als Kristalle.
Schmp.: 224°C
$R_f$ = 0,15 (Dichlormethan : Methanol 95:5)
MS (EI): m/z = 351(M)$^+$
$^1$H-NMR ([$D_6$]DMSO): δ = 8,23 (m, 1H, NHCO); 7,96 (d, J = 1 Hz, 1H, Benzothiazolon H-7); 7,73 (dd, J = 1, 9 Hz, 1H, Benzothiazolon H-5); 7,63 (d, J = 9 Hz, 1H, Benzothiazolon H-4); 4,76 (m, 1H, H-5); 4,46 (q, J = Hz, 2H, $CH_3\underline{CH_2}$); 4,17 (dd, J = 10, 10 Hz, 1H, H-4 cis); 3,80 (m, 1H, H-4 cis); 3,46 (m, 2H, $CH_2N$); 1,83 (s, 3H, $COCH_3$); 1,28 (t, J = 7 Hz, 3H, $\underline{CH_3}CH_2$).

Wie für Beispiel 44 beschrieben erhält man analog die in Tabelle 11 aufgeführten Verbindungen:

**Tabelle 11:**

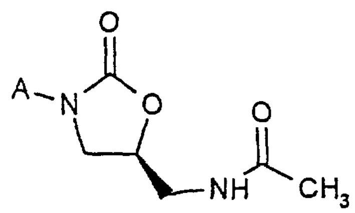

| Bsp.-Nr. | A | Ausbeute (% d.Th.) | Schmp. (°C) | $R_f$ (Laufmittel, Verhältnis) | MS (EI) m/z (M + H)$^+$ |
|---|---|---|---|---|---|
| 45 | 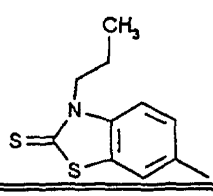 | 16 | 197 | 0,14 (I, 95:5) | 366 |

**Beispiel 46**

(5S)-3-(3-Methyl-benzothiazolinthion-6-yl)-5-(acetylaminomethyl)oxazolidin-2-on

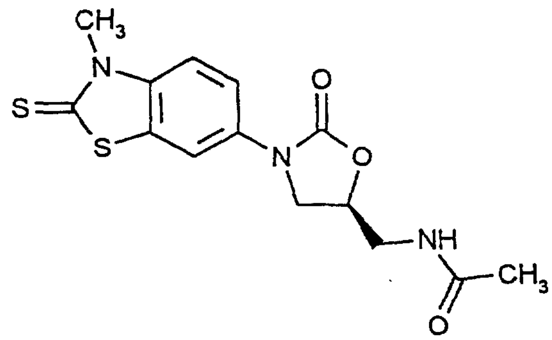

83 mg (0,17 mmol) der Verbindung aus Beispiel XXVI werden unter Rühren im Vakuum (1 mm) in Sustanz innerhalb von 1,5 h von 125°C auf 150°C erhitzt. Der Rückstand darf abkühlen und wird gut mit 250 ml Wasser gewaschen, mit 15 ml Ethylacetat und 5 ml Ethanol gut gerührt und über Sicapent im Hochvakuum getrocknet. Man erhält 48 mg (83%) der Titelverbindung als farblose Kristalle.
Schmp.: 253°C (Z)
$R_f$ = 0,10 (Dichlormethan : Methanol 95:5)
MS (FAB): m/z = 338 $(M+H)^+$
$^1$H-NMR (200 MHz, $D_6$-DMSO): δ = 8,28 (m, 1H, NHCO); 7,91 (d, J = 1Hz, 1H, Benzothiazolinthion H-7); 7,72 (dd, J = 1, 9 Hz, 1H, Benzothiazolinthion H-5); 7,56 (d, J = 9 Hz, 1H, Benzothiazolinthion H-4); 4,78 (m, 1H, H-5); 4,15 (dd, J= 10, 10 Hz, 1H, H-4 cis); 3,75 (m, 4H, $CH_3$, H-4 trans); 3,43 (m, 2H, $CH_2N$); 1,85 (s, 3H, $COCH_3$).

In Analogie zur Vorschrift des Beispiels 24 werden die in der Tabelle aufgeführten Verbindungen dargestellt.

Tabelle 12

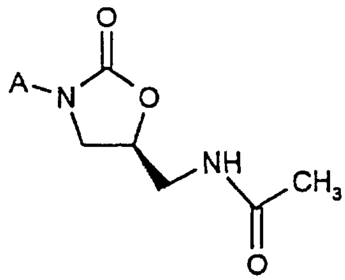

| Bsp.-Nr. | A | Ausbeute | $R_f$ Laufmittel, Verhältnis | MS (DCl) m/z ($M^+$+H) |
|---|---|---|---|---|
| 47 | 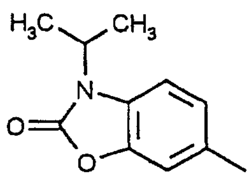 | quant. | 0,44 (I, 10:1) | 324 |
| 48 | 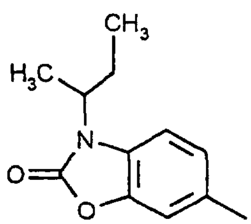 | 78 | 0,71 (I, 5:1) | 348 |
| 49 | 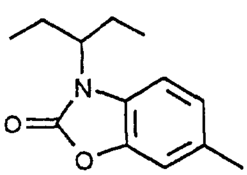 | 73 | 0,16 (I, 10:1) | 362 |
| 50 | 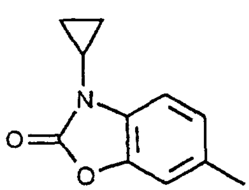 | 62 | 0,38 (I-10:1) | 322 |

| Bsp.-Nr. | A | Ausbeute | $R_f$ Laufmittel, Verhältnis | MS (DCl) m/z ($M^+$+H) |
| --- | --- | --- | --- | --- |
| 51 | 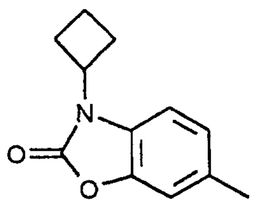 | 77 | 0,23 (I, 10:1) | 346 |
| 52 | 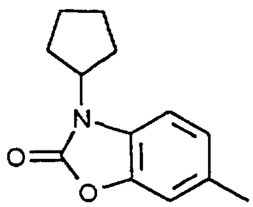 | 86 | 0,22 (I, 10:1) | 360 |

In Analogie zur Vorschrift des Beispiels 33 werden die in der Tabelle aufgeführten Verbindungen dargestellt.

## Tabelle 13

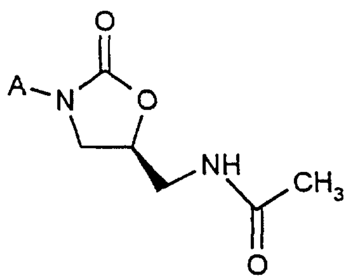

| Bsp.-Nr. | A | Ausbeute (% d.Th) | $R_f$ Laufmittel, Verhältnis | MS (DCl) m/z ($M^+$+H) |
| --- | --- | --- | --- | --- |
| 53 | 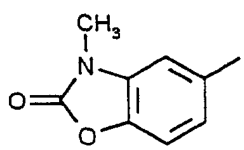 | 50 | 0,14 (VII, 5:1) | 306 |

**Beispiel 54**

(5S)-3-(3-Methyl-2-benzothiazolinon-6-yl)-5-(cyclopropylcarbonylaminomethyl)-oxazolidin-2-on

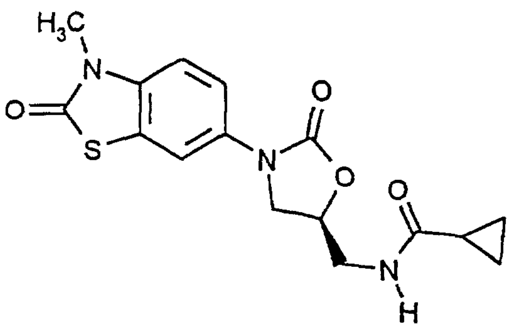

4,74 g (0,015 mol) (5S)-3-(3-Methyl-2-benzothiazolinon-6-yl)-5-(aminomethyl)-oxazolidin-2-on Hydrochlorid (Beispiel 13) werden in 150 ml Dichlormethan bei ca. 5°C unter Argon vorgelegt. Nacheinander werden 4,5 ml (0,033 mol) Triethylamin und 1,35 ml (0,015 mol) Cyclopropancarbonsäurechlorid zugetropft. Man rührt eine Stunde bei Raumtemperatur, versetzt mit Wasser, trennt die organische Phase ab und zieht das Lösemittel ab. Das erhaltene Rohprodukt wird an Kieselgel (Laufmittel:Dichlormethan/Methanol 100:2) gereinigt und anschließend mit Dichlormethan/Petrolether verrieben.
Ausbeute: 5,1 g (98 %)
Schmelzpunkt: 190-192°C
$R_f$ (I, 100:2) = 0,15
MS (DCI, $NH_3$):m/z = 348 $(M+H)^+$

In Analogie zur Vorschrift des Beispiels 54 werden die in der Tabelle 14 aufgeführten Verbindungen dargestellt.

Tabelle 14

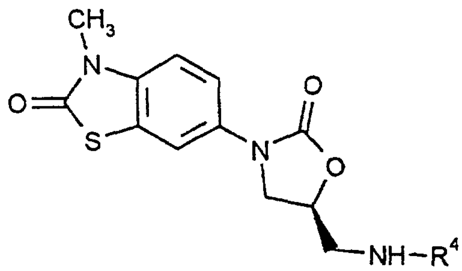

| Bsp.-Nr. | $R^4$ | Acylierungs-mittel | Ausbeute (% d.Th.) | Schmp. (°C | $R_f$(Lauf-mittel, Verhält-nis | MS(Cl) m/z ($M^+$+H) |
|---|---|---|---|---|---|---|
| 55 | 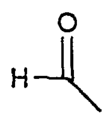 | 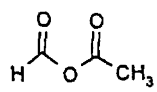 | 26 | 182-184 | 0,13 (I, 100:5) | 325[a] |
| 56 | 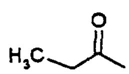 | 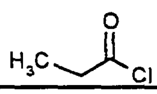 | 58 | 186-188 | 0,12 (I,100:2) | 336 |
| 57 | 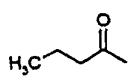 | 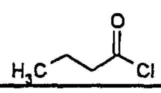 | 76 | 185-188 | 0,25 (I, 100:5) | 350 |
| 58 | 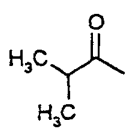 | 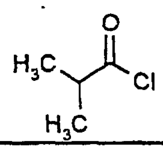 | 79 | 218-220 | 0,29 (I, 100:5) | 350 |
| 59 | 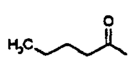 | 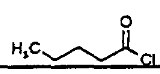 | 68 | 190-192 | 0,24 (I,100:5) | 364 |
| 60 | 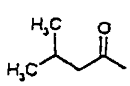 | 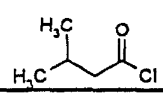 | 63 | 213-215 | 0,28 (I,100:5) | 364 |
| 61 | 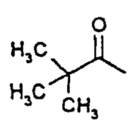 | 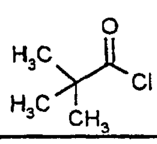 | 69 | - | 0,15 (I, 100:5) | 364 |
| 62 | 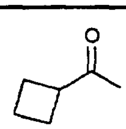 | 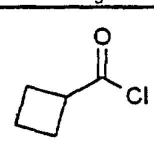 | 74 | 195-197 | 0,20 (I, 100:5) | 379[a] |
| 63 | 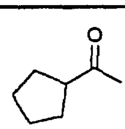 | 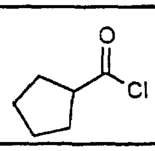 | 74 | 211-213 | 0,39 (I, 100:5) | 376 |
| 64 | 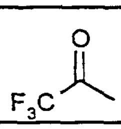 | $(F_3CCO)_2O$ | 50 | 198-200 | 0,14 (I, 100:2) | 375[b] |

| Bsp.-Nr. | $R^4$ | Acylierungs-mittel | Ausbeute (% d.Th.) | Schmp. (°C | $R_f$(Lauf-mittel, Verhält-nis | MS(Cl) m/z $(M^+ +H)$ |
|---|---|---|---|---|---|---|
| 65 | 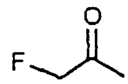 | 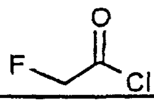 | 52 | 208-210 | 0,40 (I, 100:5) | 339[b] |
| 66 | 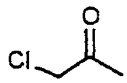 | 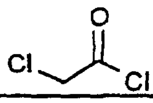 | 45 | 192-194 | 0,48 (I, 100:5) | 373[a] |
| 67 | 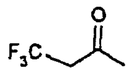 | 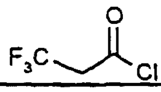 | 37 | 106-108 | 0,37 (I, 100:5) | 407[a] |
| 68 | 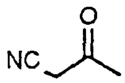 | 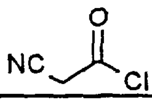 | 29 | 113-115 | 0,10 (I, 100:2) | - |
| 69 | 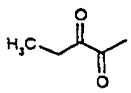 | 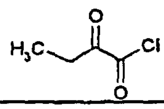 | 26 | 230-232 | 0,26 (I, 100:5) | 397[a] |
| 70 | 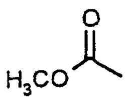 | 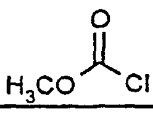 | 48 | 173-175 | 0,13 (I, 100:2) | 337[b] |
| 71 | 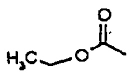 | 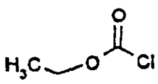 | 51 | 143-145 | 0,40 (I,100:5) | 369[a] |
| 72 | 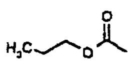 | 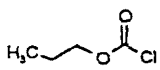 | 41 | 148-150 | 0,33 (I, 100:5) | 383[a] |
| 73 | 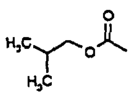 | 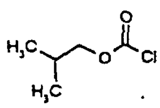 | 46 | 168-170 | 0,40 (I,100:5) | 397[a] |
| 74 | 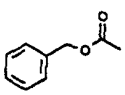 | 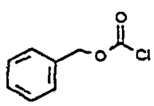 | 25 | 183-185 | 0,59 (I, 100:5) | 414 |
| 75 | 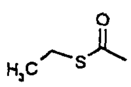 | 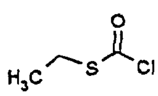 | 41 | 181-183 | 0,56 (I, 100:5) | 385[a] |
| 76 | 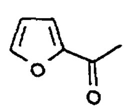 | 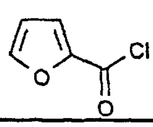 | 31 | 187-189 | 0,13 (I, 100:2) | 391[a] |
| 77 | 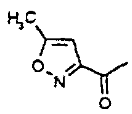 | 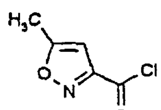 | 49 | 228-230 | 0,25 (I, 100:2) | 406[a] |

| Bsp.-Nr. | $R^4$ | Acylierungsmittel | Ausbeute (% d.Th.) | Schmp. (°C | $R_f$(Laufmittel, Verhältnis | MS(CI) m/z ($M^+$+H) |
|---|---|---|---|---|---|---|
| 78 | 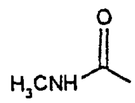 | $H_3C$-NCO | 85 | 188-191 | 0,39 (I, 9:1) | 337 |
| 79 | $H_3C$-$SO_2$- | $H_3C$-$SO_2$Cl | 35 | 188-190 | 0,27 (I, 100:5) | 375[a] |

[a] MS (Cl, $NH_3$):m/z ($M+NH_4^+$)
[b] MS (EI):m/z ($M^+$)

**Beispiel 80**

(5S)-3-(3-Allyl-2-benzoxazolinon-6-yl)-5-(acetylaminomethyl)-oxazolidin-2-on

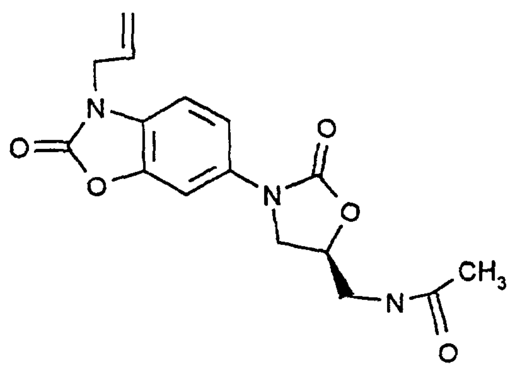

Eine Lösung aus 174 mg (0,6 mmol) der Verbindung aus Beispiel 23 und 140 µl (0,9 mmol) Diazobicycloundecen (DBU) in 10 ml DMF wird 1 h bei 40 bis 50°C gerührt. Anschließend werden 50 µl (0,6 mmol) Allylbromid hinzugegeben und die Mischung weitere 14 h bei 100°C gerührt. Das Lösemittel wird im Vakuum abgezogen und der Rückstand durch Chromatographie gereinigt.
Ausbeute: 155 mg (78 %)
$R_f$ (I, 10:1) = 0,33
MS (EI):m/z = 331 ($M^+$)
$^1$H-NMR ([$D_6$]DMSO): δ = 8,25 (bt, 1H, NHCO), 7,70 (d, 1H, Benzoxazolin 7-H), 7,25 (dd, 1H, Benzoxazolin 5-H), 7,15 (d, 1H, Benzoxazolin 4-H), 5,80-6,05 (m, 1H, C=CH), 5,10-5,70 (m, 2H, C=$CH_2$), 4,80 (m, 1H, 5-H), 4,45 (d, 2H, $CH_2$C=C), 4,10 (t, 1H, 4-H), 3,70 (dd, 1H, 4-H), 3,40 (bt, 2H, $CH_2$N), 1,80 (s, 3H, $COCH_3$).

In Analogie zum Beispiel 80 wurden die in Tabelle 15 aufgeführten Verbindungen dargestellt.

Tabelle 15

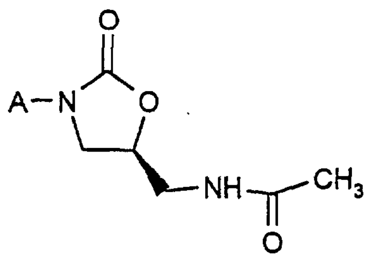

| Bsp.-Nr. | A | Ausbeute (% d.Th.) | $R_f$ (Laufmittel, Verhältnis) | MS (DCI) m/z ($M^+$+H) |
|---|---|---|---|---|
| 81 | 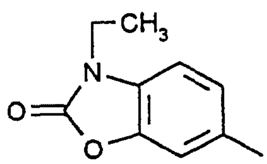 | 49 | 0,37 (I, 10:1) | 320 |
| 82 | 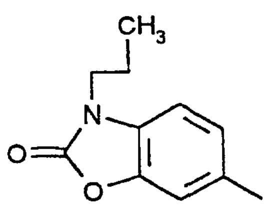 | 69 | 0,23 (I, 10:1) | 334 |
| 83 | 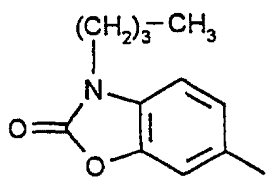 | 50 | 0,26 (I, 10:1) | 348 |
| 84 | 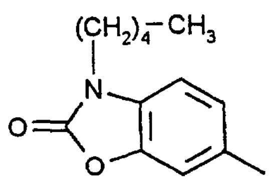 | 59 | 0,28 (I, 10:1) | 362 |
| 85 | 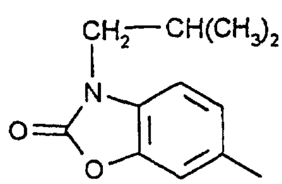 | 24 | 0,25 (I, 10:1) | 348 |

| Bsp.-Nr. | A | Ausbeute (% d.Th.) | $R_f$ (Laufmittel, Verhältnis) | MS (DCl) m/z ($M^+$+H) |
|---|---|---|---|---|
| 86 | 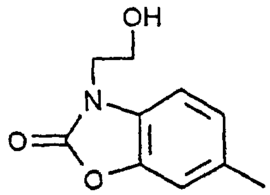 | 51 | 0,10 (I, 10:1) | 336 |

**Beispiel 87**

(5S)-3-(3-Dimethylaminomethyl-2-benzoxazolinon-6-yl)-5-(acetylaminomethyl)oxazolidin-2-on

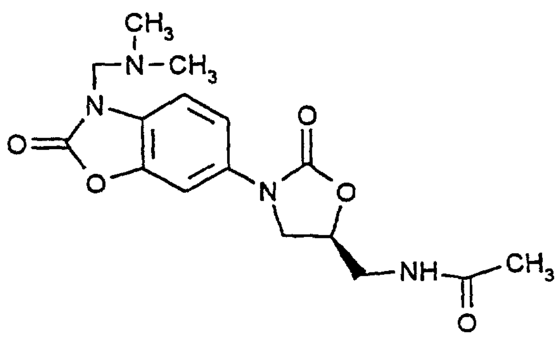

Eine Mischung aus 290 mg (1,0 mmol) der Verbindung aus Beispiel 23, 140 µl einer 30 % Formaldehyd-Lösung in Wasser, 150 µl einer 51 % Dimethylamin-Lösung in Wasser und 10 ml Ethanol wird 16 h bei 80°C gerührt. Der Niederschlag wird bei Raumtemperatur abfiltriert, mit Petrolether gewaschen und im Hochvakuum getrocknet.
Ausbeute: 86 %
$R_f$ (II, 5:1) = 0,24
MS (DCl, $NH_3$): m/z = 349 ($M^+$ +H)
$^1$H-NMR ([$D_6$]DMSO): δ = 8,25 (bt, 1H, NHCO), 7,65 (d, 1H, Ar 7-H), 7,40 (d, 1H, Ar 4-H); 7,25 (dd, 1H, Ar 5-H), 4,70 (m, 1H, 5-H), 4,60 (s, 2H, $NCH_2N$), 4,75 (t, 1H, 4-H), 3,75 (dd, 1H, 4-H), 3,40 (t, 2H, $CH_2N$), 2,30 (s, 6H, $NCH_3$), 1,80 (s, 3H, $COCH_3$).

Analog Beispiel 31 werden die in Tabelle 16 aufgeführten Verbindung dargestellt.

Tabelle 16

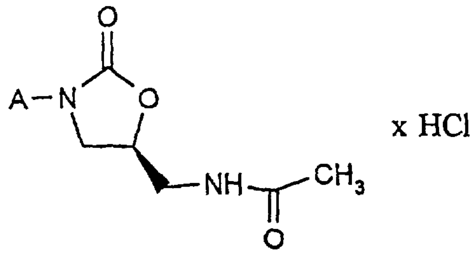

| Bsp.-Nr. | A | Ausbeute (% d. Th.) |
|---|---|---|
| 88 | 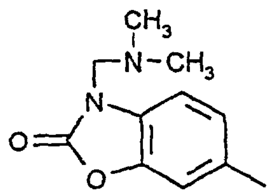 | 97 |

**Beispiel 89**

(5S)-3-(2-Benzothiazolinon-6-yl)-5-(acetylaminomethyl)-oxazolidin-2-on

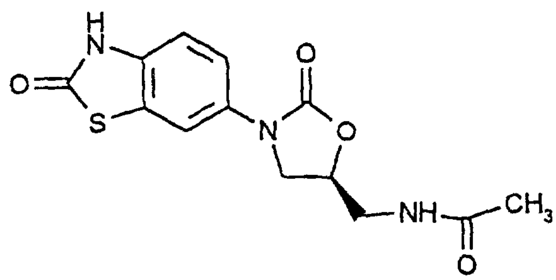

Eine Mischung aus 95 g (0,28 mol) der Verbindung aus Beispiel XXV und 200 g (0,37 mol) Oxone® (Kaliummonopersulfat Tripelsalz) in 5 l Wasser wird 24 h bei Raumtemperatur gerührt. Nach Zugabe von 1 l 2-Propanol wird der Niederschlag abgesaugt und der Rückstand durch Chromatographie gereinigt. Man erhält 84,6 g (81 %) (5S)-3-(2-Methylsulfonyl-2-benzothiazolinon-6-yl)-5-(acetylaminomethyl)-oxazolidinon. 2 g (5,4 mmol) dieser Verbindung werden in 50 ml Wasser und 10 ml Triethylamin 14 h zum Rückfluß erhitzt. Nach dem Abziehen der flüchtigen Bestandteile wird der Rückstand durch Chromatographie gereinigt.
Ausbeute: 1,15 g (69 %)
Schmp.: 223°C
MS (Cl), m/z = 325 ($M^+NH_4^+$)

**Beispiel 90**

(5S)-3-(3-Hydroxymethyl-2-benzothiazolinon-6-yl)-5-(acetylaminomethyl)oxazolidin-2-on

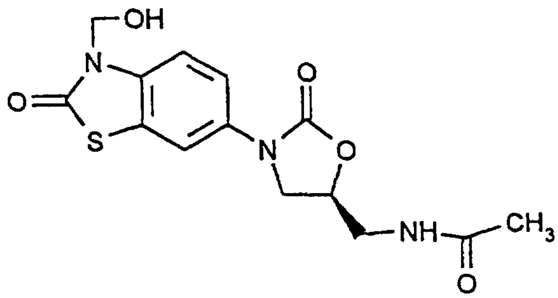

Eine Mischung aus 308 mg (1,0 mmol) der Verbindung aus Beispiel 89 und 0,13 ml 37 % Formaldehyd-Lösung in 1 ml Wasser wird 14 h bei 70-80°C gerührt. Der entstandene Niederschlag wird abgesaugt, mit Wasser gewaschen und getrocknet.
Ausbeute: 280 mg (83 %)
Schmp.: 192°C

**Beispiel 91**

(5S)-3-(3-Fluormethyl-2-benzoxazolinon-6-yl)-5-(acetylaminomethyl)oxazolidin-2-on

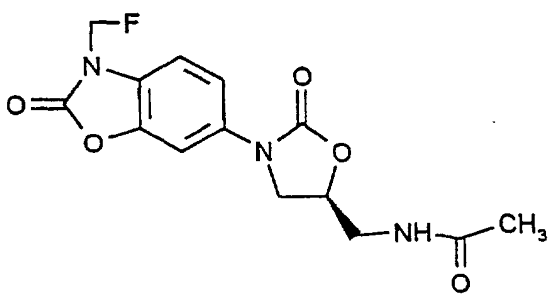

Zu einer Suspension von 100 ml (311 mmol) der Verbindung aus Beispiel 42 und 10 ml Dichlormethan werden bei -50°C 61 µl (466 mmol) Diethylaminoschwefeltrifluorid (DAST) gegeben. Man läßt auf Raumtemperatur kommen, rührt weitere 52 h, versetzt mit 5 ml gesättigter $NaHCO_3$-Lösung, rührt 10 min und wäscht dann die organische Phase mit Wasser. Der dabei ausgefallene Niederschlag wird abgesaugt, die organische Phase getrocknet ($Na_2SO_4$) und eingedampft.
Ausbeute: 25 mg (25 %)
$R_f$ = 0,22 (VII, 5:1)
MS (EI): m/z = 323 ($M^+$)
$^1$H-NMR (200 MHz, [$D_6$]DMSO): δ = 8,25 (bs, 1H, NHCO), 7,72 (d, 1H, Ar-H-2), 7,55 (d, 1H, Ar-H-4), 7,32 (dd, 1H, Ar-H-5), 6,05 (d, 2H, $CH_2F$), 4,70 (m, 1H, H-5), 4,10 (t, 1H, H-4), 3,75 (d, 1H, H-4), 3,40 (m, 2H, $CH_2N$), 1,85 (s, 3H, $COCH_3$).
In Analogie zur Vorschrift des Beispiels 91 erhält man die in der Tabelle 17 aufgeführten Verbindungen.

**Tabelle 17**

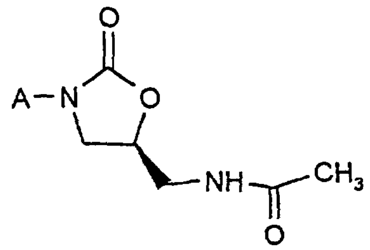

| Bsp.-Nr. | A | Ausbeute (% d. Th.) | Schmp. (°C) | $R_f$ (Laufmittel, Verhältnis |
|---|---|---|---|---|
| 92 | 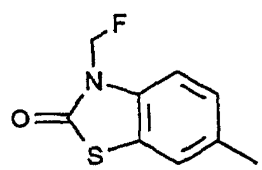 | 74 | 185 | 0,54 (I, 9:1) |

**Beispiel 93**

(5S)-3-(2-(Allylimino)-3-methyl-2,3-dihydrobenzoxazol-6-yl)-5-(acetylaminomethyl)oxazolidin-2-on

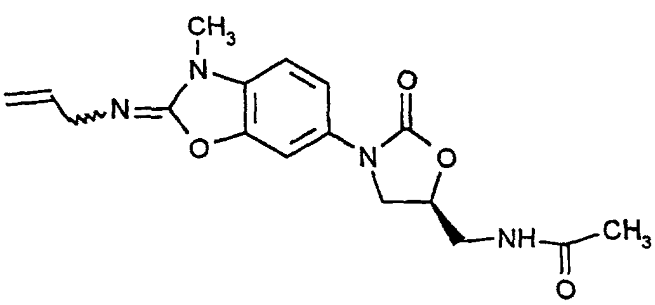

Eine Mischung aus 0,30 g (0,694 mol) der Verbindung aus Beispiel XXXV, 0,29 g (3,33 mol) Allylbromid und 0,38 g (2,77 mol) wasserfreies Kaliumcarbonat in 4 ml Ethanol wird 11 h unter Rückfluß erhitzt. Zur Aufarbeitung wird abgesaugt, das Filtrat im Vakuum eingeengt, getrocknet und durch Chromatographie gereinigt. Das so erhaltene Öl wird in Essigester gelöst und mit Petrolether das Produkt gefällt.
Ausbeute: 0,015 g (6 %)
$R_f$ (VII, 1:1) = 0,47
MS (EI): m/z = 344 ($M^+$)
$^1$H-NMR ([$D_6$]DMSO): δ = 8,24 (t, 1H, NHCO), 7,52 (d, 1H, Ar 7-H), 7,15 (dd, 1H, Ar 5-H), 7,03 (d, 1H, Ar 4-H), 5,72-6,07 (m, 1H, HC=C), 5,22 (dq, 1H $H_2C$=C), 5,03 (dq, 1H, $H_E$C=C), 4,70 (m, 1H, 5-H), 4,10 (t, 1H, 4-H), 3,98 (m, 2H, $CH_2N$), 3,72 (dd, 1H, 4-H), 3,40 (t, 2H, $CH_2N$), 3,33 (s, 3H, $NCH_3$), 1,82 (s, 3H, $COCH_3$).

**Beispiel 94**

(5S)-3-(2-Cyclopropylcarbonylimino-3-methyl-2,3-dihydrobenzoxazol-6-yl)-5-(acetylaminomethyl)-oxazolidin-2-on

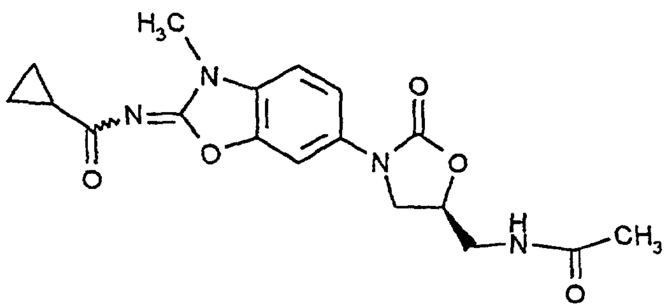

Zu einer Suspension von 304 mg (0,703 mmol) der Verbindung aus Beispiel XXXV in 10 ml THF werden 310 µl (2,2 mmol) Triethylamin gegeben und anschließend bei 0°C 100 µl (11 mmol) Cyclopropancarbonsäurechlorid zugetropft. Nach 1h gibt man die Mischung auf Eiswasser, sättigt die wäßrige Phase mit Natriumchlorid, extrahiert dreimal mit Essigester, trocknet ($Na_2SO_4$), zieht die Lösungsmittel ab und kristallisiert aus Dichlormethan.
Ausbeute: 196 mg (75 %)
$R_f$ = 0,45 (VII, 1:1)
MS (DCI/$NH_3$): m/z = 373 ($M^+$+H)
$^1$H-NMR (200 MHz, [$D_6$]DMSO): δ = 8,25 (bt, 1H, NHCO), 7,75 (s, 1H, Ar), 7,40 (bs, 2H, Ar), 4,75 (m, 1H, H-5), 4,15 (t, 1H, H-4), 3,40 (m, 5H, $CH_2N$, $CH_3$), 1:90 (s, 3H, $COCH_3$), 1,70 (m, 1H, Cpr-H), 0,70-0,95 (m, 4H, Cpr-H).

In Analogie zur Vorschrift des Beispiels 94 werden die in der Tabelle 18 aufgeführten Verbindungen dargestellt.

**Tabelle 18**

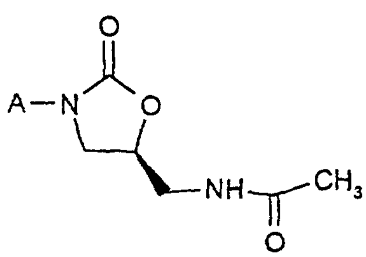

| Bsp.-Nr. | A | Acylierungs-mittel | Aus-beute (% d. Th.) | $R_f$ (Lauf-mittel, Verhält-nis) | MS (CI) m/z ($M^+$+H) |
|---|---|---|---|---|---|
| 95 | 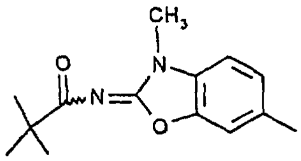 | 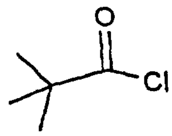 | 79 | 0,53 (VII, 1:1) | 389 |
| 96 | 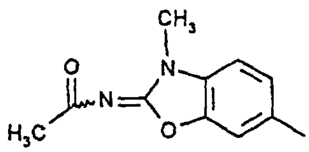 | 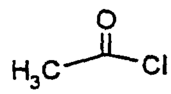 | 36 | 0,35 (VII, 1:1) | 347 |
| 97 | 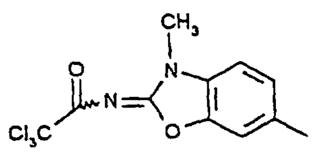 | 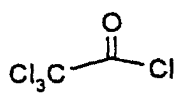 | 38 | 0,53 (VII, 1:1) | 449 |
| 98 | 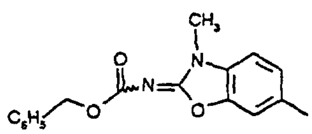 | 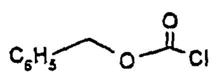 | 32 | 0,43 (VII, 1:1) | 439 |
| 99 | 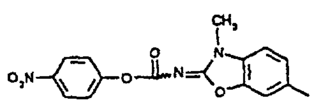 | 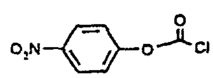 | 62 | 0,44 (VII, 1:1) | 470 |
| 100 | 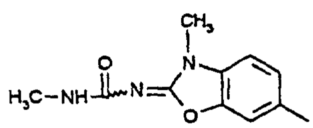 | $H_3C$-NCO | 46 | 0,26 (VII, 1:1) | 362 |
| 101 | 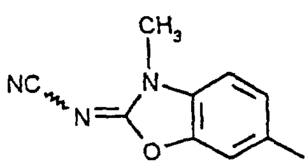 | BrCN | 44 | 0,37 (VII, 1:1) | 330 |

**Beispiel 102**

(5S)-3-(3-Aza-1-oxa-2-thiaindan-2-dioxid-6-yl)-5-(acetylaminomethyl)ozazolidin-2-on

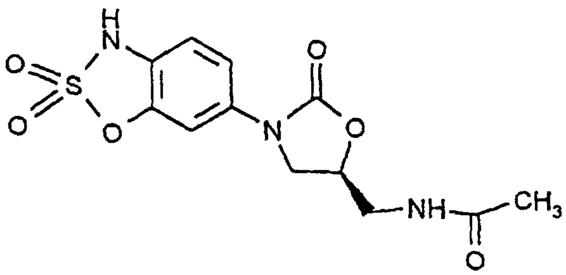

Zu einer Mischung aus 0,5 g (1,88 mmol) der Verbindung aus Beispiel 23, 0,63 ml (4,52 mmol) Triethylamin und 20 ml wasserfreiem Dichlormethan wird bei -5°C tropfenweise eine Lösung aus 0,17 ml (2,07 mmol) Sulforylchlorid in 5 ml Dichlormethan gegeben. Man rührt weitere I h bei -5°C, anschließend 14 h bei Raumtemperatur und versetzt dann mit Wasser. Die organische Phase wird dreimal mit Dichlormethan gewaschen, die vereinigten wäßrigen Phasen mit Natriumchlorid gesättigt und viermal mit Essigester extrahiert. Die Essigester-Phasen werden getrocknet ($Na_2SO_4$) und im Vakuum das Lösemittel abgezogen. Ausbeute: 98 mg (16 %)
$R_f$ (VII, 1:1) = 0,17
MS (FAB): mlz = 326 ($M^+$-H)
$^1$H-NMR ([$D_6$]DMSO): $\delta$ = 8,25 (t, 1H, NHCO), 7,50 (d, 1H, Ar 7-H), 7,27 (dd, 1H, Ar 5-H), 7,05 (d, 1H, Ar 4-H), 4,70 (m, 1H, 5-H), 4,05 (t, 1H, 4-H), 3,65 (dd, 1H, 4-H), 3,40 (t, 1H, $CH_2N$), 1,80 (s, 3H, $COCH_3$).

In Analogie zur Vorschrift des Beispiels 1 werden die in der Tabelle 19 aufgeführten Verbindungen dargestellt:

Tabelle 19

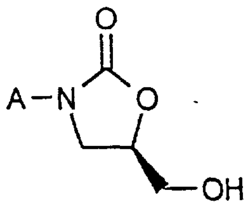

| Bsp.-Nr. | A | Ausbeute (% d. Th.) | Schmp. (°C) | $R_f$ (Laufmittel, Verhältnis) | MS m/z |
|---|---|---|---|---|---|
| 103 | 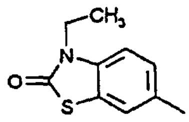 | 76 | 156 | 0,32 (I, 100:5) | 312 ($M+NH_4^+$) |
| 104 | 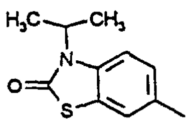 | 62 | 157 | 0,33 (I, 100:5) | 326 ($M+NH_4^+$) |
| 105 | 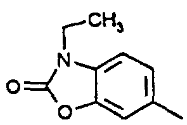 | 50 | - | 0,12 (II, 1:1) | 296 ($M+NH_4^+$) |

In Analogie zur Vorschrift des Beispiels 5 werden die in der Tabelle 20 aufgeführten Verbindungen dargestellt:

## Tabelle 20

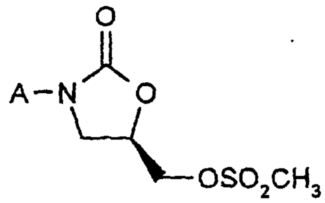

| Bsp.-Nr. | A | Ausbeute (% d. Th.) | Schmp. (°C) | $R_f$ (Laufmittel, Verhältnis) | MS m/z |
|---|---|---|---|---|---|
| 106 | 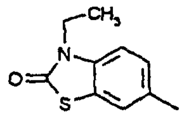 | 86 | 150 | 0,52 (I, 100:5) | - |
| 107 | 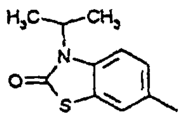 | quant. | - | 0,58 (I, 100:5) | - |
| 108 | 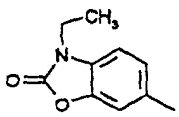 | 95 | - | 0,31 (VII, 5:1) | 357 (M+H$^+$) |

In Analogie zur Vorschrift des Beispiels 9 werden die in der Tabelle 21 aufgeführten Verbindungen dargestellt:

## Tabelle 21

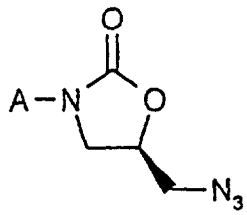

| Bsp.-Nr. | A | Ausbeute (% d. Th.) | Schmp. (°C) | $R_f$ (Laufmittel, Verhältnis) | MS m/z |
|---|---|---|---|---|---|
| 109 | 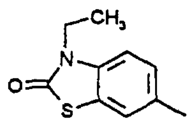 | 93 | 180-183 | 0,69 (I, 100:5) | - |
| 110 | 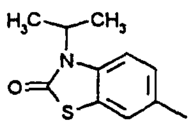 | 91 | - | 0,69 (I, 100:5) | - |
| 111 | 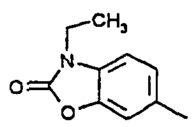 | 88 | - | 0,27 (VII, 5:1) | 304 ($M+H^+$) |

In Analogie zur Vorschrift des Beispiels 13 werden die in der Tabelle 22 aufgeführten Verbindungen dargestellt:

Tabelle 22

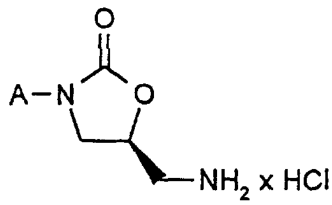

| Bsp.-Nr. | A | Ausbeute (% d. Th.) | Schmp. (°C) | $R_f$ (Laufmittel, Verhältnis) | MS m/z |
|---|---|---|---|---|---|
| 112 | 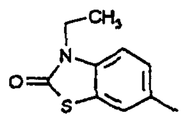 | 91 | 258 (Z) | 0,25 (I, 9:1) | 311 ($M+NH_4^+$) |
| 113 | 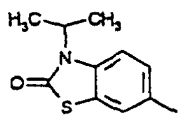 | 90 | 231 (Z) | 0,19 (I, 9:1) | 325 ($M+NH_4^+$) |
| 114[a] | 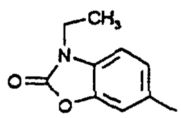 | quant. | - | 0,21 (I, 10:1) | 295 ($M+NH_4^+$) |

[a] isoliert als freies Amin in Analogie zur Vorschrift des Beispiels XXVII

In Analogie zur Vorschrift des Beispiels 54 werden die in der Tabelle 23 aufgeführten Verbindungen dargestellt:

## Tabelle 23:

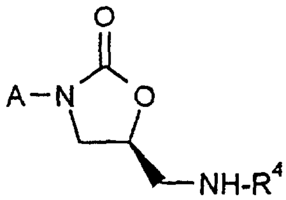

| Bsp.-Nr. | A | $R^4$ | Ausbeute (%) | Schmp. (°C) | $R_f$ (Laufmittel, Verhältnis) | MS m/z |
|---|---|---|---|---|---|---|
| 115 | 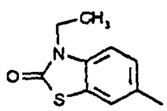 | 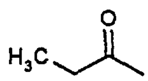 | 73 | 175 | 0,26 (I, 100:5) | 350 $(M+H^+)$ |
| 116 | 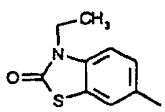 | 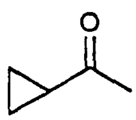 | 74 | 191 | 0,28 (I, 100:5) | 362 $(M+H^+)$ |
| 117 | 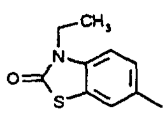 | 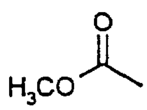 | 55 | 142 | 0,50 (I, 100:5) | 352 $(M+H^+)$ |
| 118 | 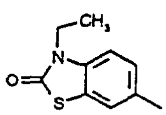 | 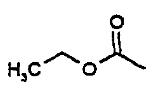 | 51 | 129 | 0,45 (I, 100:5) | 383 $(M+H^+)$ |
| 119 | 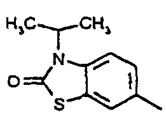 | 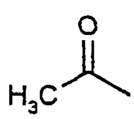 | 64 | 132 | 0,20 (I, 100:5) | 367 $(M+H^+)$ |
| 120 | 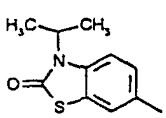 | 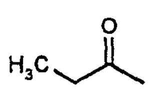 | 69 | 143 | 0,35 (I, 100:5) | 363 $(M^+)$ |

| Bsp.-Nr. | A | $R^4$ | Ausbeute (%) | Schmp. (°C) | $R_f$ (Laufmittel, Verhältnis) | MS m/z |
|---|---|---|---|---|---|---|
| 121 | 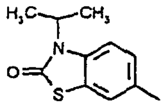 | 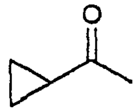 | 57 | 143 | 0,38 (I, 100:5) | 375 ($M^+$) |
| 122 | 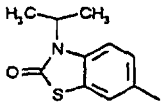 | 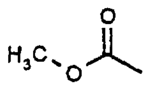 | 64 | 151 | 0,39 (I, 100:5) | 383 ($M+NH_4^+$) |
| 123 | 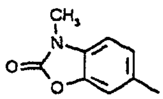 | 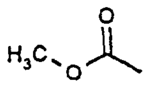 | 53 | - | 0,40 (I, 10:1) | 339 ($M+H^+$) |
| 124 | 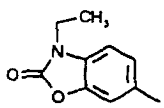 | 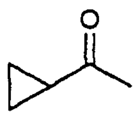 | 69 | - | 0,46 (I, 10:1) | 346 ($M+H^+$) |
| 125 | 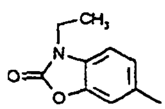 | 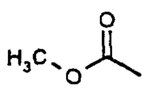 | 48 | - | 0,43 (I, 10:1) | 353 ($M+H^+$) |
| 126 | 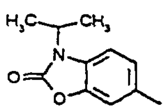 | 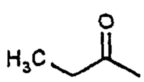 | 82 | - | 0,44 (I, 10:1) | 334 ($M+H^+$) |
| 127 | 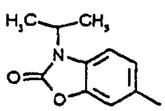 | 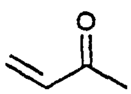 | 90 | - | 0,45 (I, 10:1) | 348 ($M+H^+$) |
| 128 | 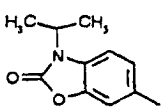 | 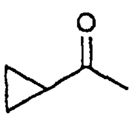 | 86 | - | 0,45 (I, 10:1) | 360 ($M+H^+$) |
| 129 | 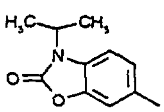 | 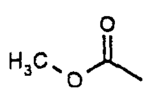 | 85 | - | 0,49 (I, 10:1) | 350 ($M+H^+$) |

| Bsp.-Nr. | A | $R^4$ | Ausbeute (%) | Schmp. (°C) | $R_f$ (Laufmittel, Verhältnis) | MS m/z |
|---|---|---|---|---|---|---|
| 130 | 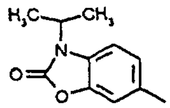 | 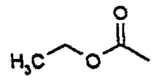 | 48 | - | 0,71 (I, 10:1) | 443 ($M+NH_4^+$) |
| 131[a] | 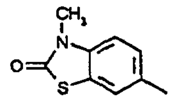 | 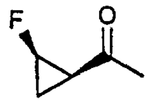 (rac) | 57 | - | 0,26 (I, 100:5) | 366 ($M+H^+$) |
| 132[a] | 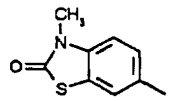 | 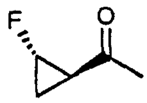 (rac) | 56 | - | 0,31 (I, 100:5) | 366 ($M+H^+$) |
| 133[a] | 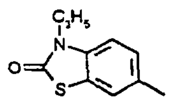 | 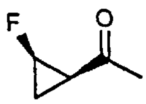 (rac) | 37 | 209 | 0,37 (I, 100:5) | 397 ($M+NH_4^+$) |
| 134[a] | 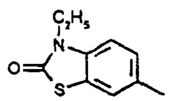 | 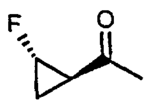 (rac) | 72 | 182 | 0,34 (I, 100:5) | 397 ($M+NH_4^+$) |

[a] dargestellt aus den entsprechenden Carbonsäuren mit 1-Hydroxybenzotriazol (HOBT), N-Ethyl-N'-(3-dimethylamino)carbodiimid (EDC)

In Analogie zur Vorschrift des Beispiels 24 werden die in der Tabelle 24 aufgeführten Verbindungen dargestellt:

**Tabelle 24:**

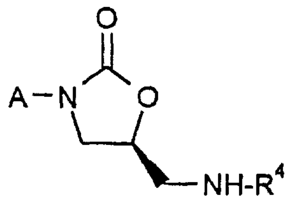

| Bsp.-Nr. | A | $R^4$ | Ausbeute (% d.Th.) | $R_f$ (Laufmittel, Verhältnis) | MS m/z |
|---|---|---|---|---|---|
| 135 | 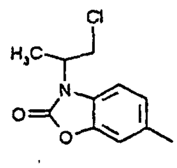 | 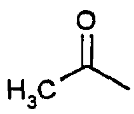 | 65 | 0,29 (I, 10:1) | 368 $(M+H^+)$ |
| 136 | 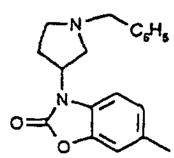 | 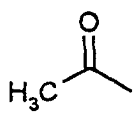 | 93 | 0,44 (I, 10:1) | - |
| 137 | 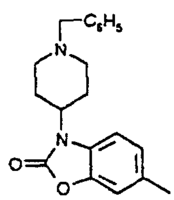 | 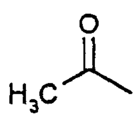 | quant. | 0,13 (I, 10:1) | 465 $(M+H^+)$ |
| 138 | 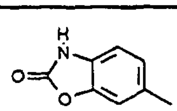 | 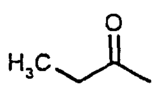 | 79 | 0,26 (I, 10:1) | 323 $(M+NH_4^+)$ |
| 139 | 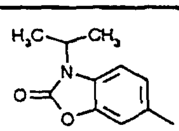 | 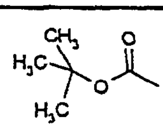 | 96 | 0,71 (I, 10:1) | 409 $(M+NH_4^+)$ |

| Bsp.-Nr. | A | $R^4$ | Ausbeute (% d.Th.) | $R_f$ (Laufmittel, Verhältnis | MS m/z |
|---|---|---|---|---|---|
| 140 | 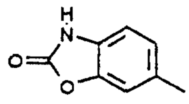 | 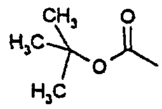 | 49 | 0,34 (I, 10:1) | 367 ($M+NH_4^+$) |
| 141 | 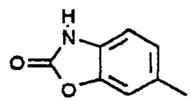 | 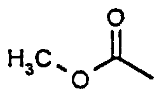 | 66 | - | 325 ($M+NH_4^+$) |
| 142 | 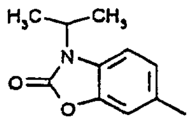 | 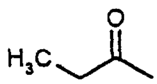 | 82 | 0,38 (I, 10:1) | 348 ($M+H^+$) |

In Analogie zur Vorschrift des Beispiels 80 werden die in der Tabelle 25 aufgeführten Verbindungen dargestellt:

## Tabelle 25:

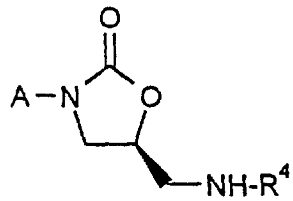

| Bsp.-Nr. | A | $R^4$ | Ausbeute (% d.Th.) | $R_f$ (Laufmittel, Verhältnis) | MS m/z |
|---|---|---|---|---|---|
| 143 | 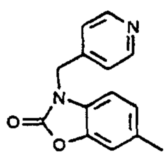 | 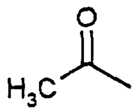 | 65 | 0,32 (I, 10:1) | 397 ($M+H^+$) |
| 144 | 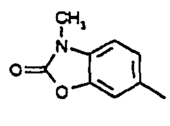 | 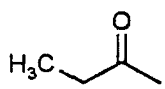 | 74 | 0,52 (I, 10:1) | 337 ($M+H^+$) |
| 145 | 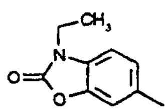 | 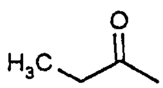 | 64 | 0,37 (I, 10:1) | 351 ($M+NH_4^+$) |
| 146 | 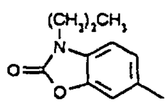 | 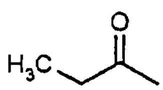 | 85 | 0,32 (I, 10:1) | 365 ($M+NH_4^+$) |
| 147 | 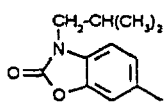 | 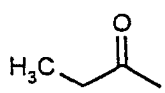 | 36 | - | 379 ($M+NH_4^+$) |
| 148 | 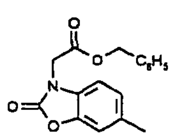 | 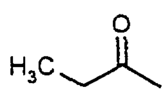 | 76 | 0,81 (I, 10:1) | 454 ($M+H^+$) |

| Bsp.-Nr. | A | $R^4$ | Ausbeute (% d.Th.) | $R_f$ (Laufmittel, Verhältnis) | MS m/z |
|---|---|---|---|---|---|
| 149 | 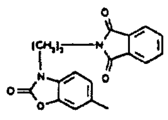 | 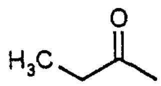 | 41 | 0,31 (I, 10:1) | 496 ($M+NH_4^+$) |
| 150 | 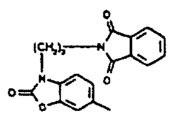 | 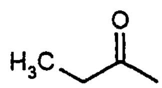 | 73 | 0,30 (I, 10:1) | 493 ($M+H^+$) |
| 151 | 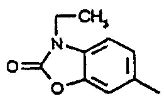 | 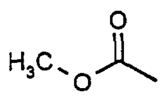 | 90 | 0,43 (I, 10:1) | 353 ($M+H^+$) |
| 152 | 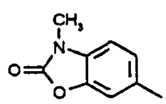 | 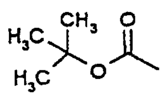 | 23 | 0,49 (I, 10:1) | 381 ($M+H^+$) |
| 153 | 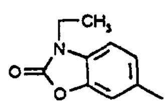 | 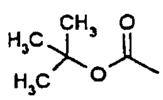 | 63 | 0,48 (I, 10:1) | 395 ($M+NH_4^+$) |

In Analogie zur Vorschrift des Beispiels V werden die in der Tabelle 26 aufgeführten Verbindungen dargestellt:

## Tabelle 26:

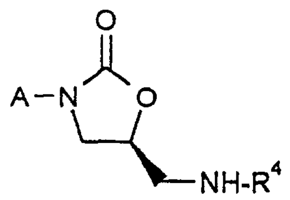

| Bsp.-Nr. | A | $R^4$ | Ausbeute (% d. Th.) | Schmp. (°C) | $R_f$ (Laufmittel, Verhältnis) | MS m/z |
|---|---|---|---|---|---|---|
| 154 | 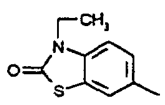 | 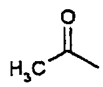 | 31 | 166 | 0,61 (I, 9:1) | 353 ($M+NH_4^+$) |
| 155 | 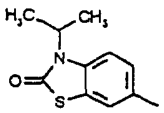 | 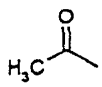 | 63 | 120 | 0,65 (I, 9:1) | 350 ($M+H^+$) |

In Analogie zur Vorschrift des Beispiels 31 werden unter Verwendung von 4 N HCl in Dioxan die in Tabelle 27 aufgeführten Verbindungen dargestellt:

**Tabelle 27:**

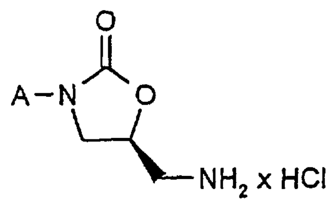

| Bsp.-Nr. | A | Ausbeute (% d. Th.) | MS m/z |
|---|---|---|---|
| 156 | 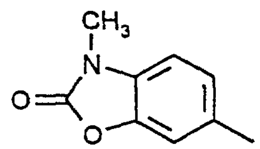 | 70 | - |
| 157 | 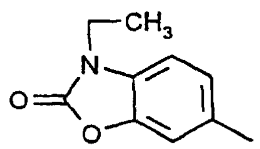 | 93 | 278 ($M^+$-Cl) |
| 158 | 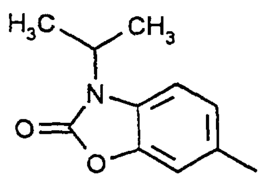 | 97 | 309 ($M+NH_4^+$-Cl) |

In Analogie zur Vorschrift des Beispiels 9 werden die in der Tabelle 28 aufgeführten Verbindungen dargestellt:

**Tabelle 28:**

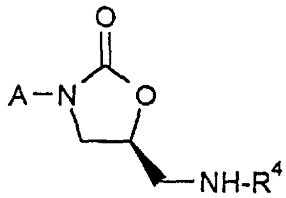

| Bsp.-Nr. | A | $R^4$ | Ausbeute (% d.Th.) | $R_f$ (Laufmittel, Verhältnis | MS · m/z |
|---|---|---|---|---|---|
| 159 | 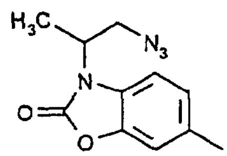 | 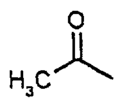 | 95 | 0,29 (I, 10:1) | 392 ($M+NH_4^+$) |

**Beispiel 160**

(5S)-3-(3-(3-Aminopropyl)-2-benzoxazolinon-6-yl)-5-(propionylaminomethyl)-2-oxazolidinon

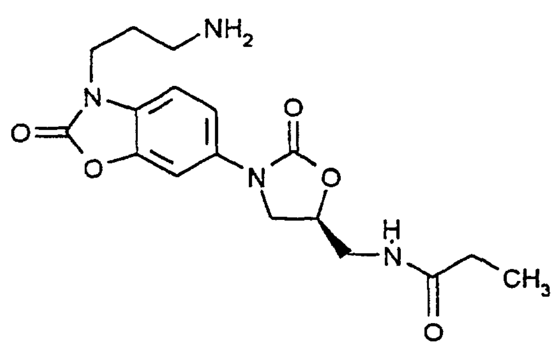

Eine Suspension der Verbindung aus Beispiel 150 (328 mg, 0,67 mmol) in Ethanol (20 ml) wird mit 40 % Methylamin (in $H_2O$, 320 µl, 4,1 mmol) versetzt und 3 Stunden bei 70°C gerührt, anschließend 1 Stunde unter Rückfluß erhitzt. Der ausgefallene Niederschlag wird abgesaugt und getrocknet.
Ausbeute: 123 mg (51 %)
$R_f$ (I, 10:1) = 0,21
$^1$H-NMR (200 MHz, [$D_6$]DMSO): $\delta$ = 8,10 (bt, 1H, NH), 7,18 (d, 1H, Ar-H), 7,08 (d, 1H, Ar-H), 6,81 (dd, 1H, Ar-H), 6,60 (bs, 2H, $NH_2$), 4,70 (m, 1H, 5-H), 4,10 (t, 1H, 4-H), 3,70 (dd, 1H, 4-H), 3,40 (m, 4H, $CH_2N$), 3,20 (m, 2H, $CH_2N$), 2,10 (q, 2H, $COCH_2$), 190 (m, 2H, $CH_2$), 0,95 (t, 3H, CH).

In Analogie zur Vorschrift des Beispiels 160 werden die in der Tabelle 29 aufgeführten Verbindungen dargestellt:

**Tabelle 29:**

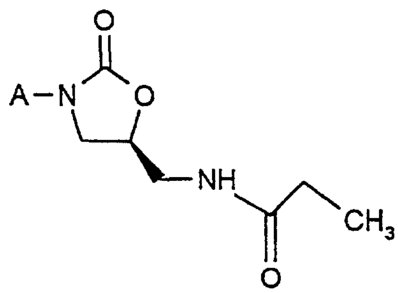

| Bsp.-Nr. | A | Ausbeute (% d.Th.) | $R_f$ (Laufmittel, Verhältnis) | MS m/z |
|---|---|---|---|---|
| 161 | 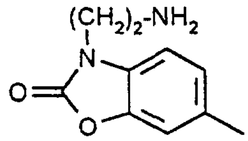 | 17 | 0,30 (I, 10:1) | 347 $(M+H^+)$ |

In Analogie zur Vorschrift des Beispiels XXXVII werden die in der Tabelle 30 aufgeführten Verbindungen dargestellt:

## Tabelle 30:

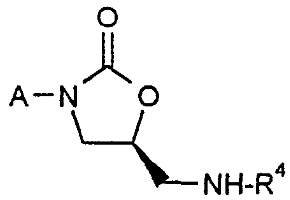

| Bsp.-Nr. | A | $R^4$ | Ausbeute (% d. Th.) | $R_f$ (Laufmittel, Verhältnis) | MS m/z |
|---|---|---|---|---|---|
| 162 | 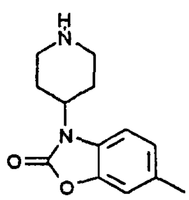 | 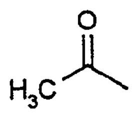 | 13 | 0,01 (I, 10:1) | 375 ($M+H^+$) |
| 163 | 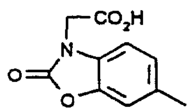 | 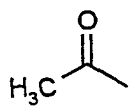 | 32 | 0,01 (I, 10:1) | 364 ($M+H^+$) |
| | 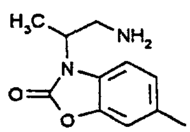 | 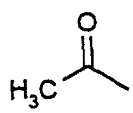 | quant. | 0,11 (I, 100:1) | - |

In Analogie zur Vorschrift des Beispiels 31 werden die in der Tabelle 31 aufgeführten Verbindungen dargestellt:

**Tabelle 31:**

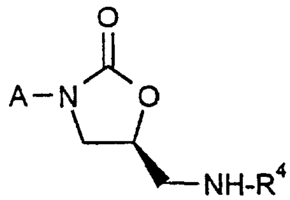

| Bsp.-Nr. | A | $R^4$ | Ausbeute (% d. Th.) | MS m/z |
|---|---|---|---|---|
| 165 | 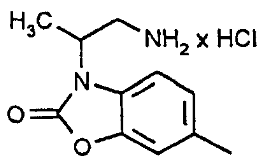 | 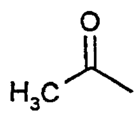 | 25 | - |
| 166 | 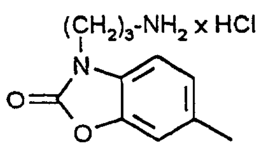 | 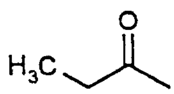 | 60 | 363 ($M+H^+$) |
| 167 | 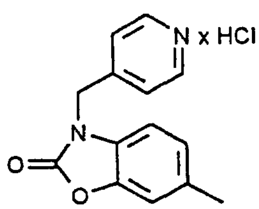 | 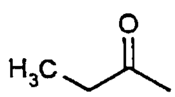 | 31 | - |

In Analogie zur Vorschrift des Beispiels 33 werden die in der Tabelle 32 aufgeführten Verbindungen dargestellt:

Tabelle 32:

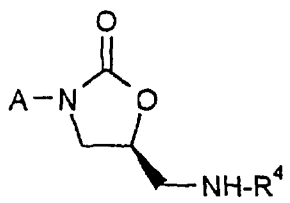

| Bsp.-Nr. | A | $R^4$ | Ausbeute (% d. Th.) | $R_f$ (Laufmittel, Verhältnis) | MS m/z |
|---|---|---|---|---|---|
| 168 | 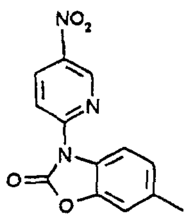 | 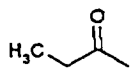 | 43 | 0,55 (I, 10:1) | 445 $(M+NH_4^+)$ |

**Patentansprüche**

1. Verbindungen der allgemeinen Formel (1)

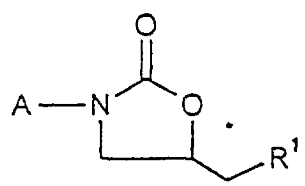

(I)

in welcher

$R^1$ für Azido, Hydroxy oder für eine Gruppe der Formel $-OR^2$, $O-SO_2R^3$ oder $-NR^4R^5$ steht, worin

$R^2$ geradketriges oder verzweigtes Acyl mit bis zu 8 Kohlenstoffatomen oder eine Hydroxyschutzgruppe bedeutet,

$R^3$ geradkettiges oder verzweigtes Alkyl mit bis zu 4 Kohlenstoffatomen oder Phenyl bedeutet, das gegebenenfalls durch geradkettiges oder verzweigtes Alkyl mit bis zu 4 Kohlenstoffatomen substituiert ist,

$R^4$ und $R^5$ gleich oder verschieden sind und Cycloalkyl mit 3 bis 6 Kohlenstoffatomen, Wasserstoff, Phenyl oder geradkettiges oder verzweigtes Alkyl oder Alkoxy mit jeweils bis zu 8 Kohlenstoffatomen oder eine Aminoschutzgruppe bedeuten,
oder

$R^4$ oder $R^5$ eine Gruppe der Formel -CO-$R^6$, P(O)($OR^7$)($OR^8$) oder -$SO_2$-$R^9$ bedeutet,
worin

$R^6$ Cycloalkyl oder Halogen-substituiertes Cycloalkyl mit jeweils 3 bis 6 Kohlenstoffatomen, Trifluormethyl, geradkettiges oder verzweigtes Alkoxy mit bis zu 8 Kohlenstoffatomen, Phenyl, Benzyloxy oder Wasserstoff bedeutet, oder

$R^6$ geradkettiges oder verzweigtes Alkyl oder Alkenyl mit jeweils bis zu 8 Kohlenstoffatomen bedeutet, die gegebenenfalls durch Cyano, Halogen oder Trifluormethyl substituiert sind,
oder
geradkettiges oder verzweigtes Thioalkyl oder Acyl mit jeweils bis zu 6 Kohlenstoffatomen bedeutet,
oder
eine Gruppe der Formel -$NR^{10}R^{11}$ bedeutet,
worin

$R^{10}$ und $R^{11}$ gleich oder verschieden sind und Wasserstoff, Phenyl oder geradkettiges oder verzweigtes Alkyl mit bis zu 6 Kohlenstoffatomen bedeuten,

oder

$R^6$ einen 5-gliedrigen aromatischen Heterocyclus mit bis zu 3 Heteroatomen aus der Reihe S, N und/oder 0 bedeutet, der gegebenenfalls durchgeradkettiges oder verzweigtes Alkyl mit bis zu 3 Kohlenstoffatomen substituiert ist,

$R^7$ und $R^8$ gleich oder verschieden sind und Wasserstoff oder geradkettiges oder verzweigtes Alkyl mit bis zu 4 Kohlenstoffatomen bedeuten,

$R^9$ geradkettiges oder verzweigtes Alkyl mit bis zu 4 Kohlenstoffatomen oder Phenyl bedeutet

A für einen Rest der Formel

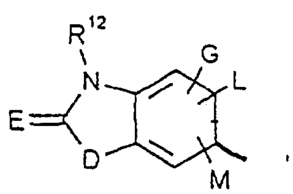

steht,
worin

G, L und M gleich oder verschieden sind und für Wasserstoff, Carboxy, Halogen, Cyano, Formyl, Trifluormethyl, Nitro, für geradkettiges oder verzweigtes Alkyl mit bis zu 6 Kohlenstoffatomen oder für eine Gruppe der Formel -CO-$NR^{17}R^{18}$ stehen,
worin

$R^{17}$ und $R^{18}$ gleich oder verschieden sind und Wasserstoff, geradkettiges oder verzweigtes Alkyl mit bis zu 4 Kohlenstoffatomen oder Phenyl bedeuten,

$R^{12}$ Wasserstoff, Cycloalkylcarbonyl oder Cycloalkyl mit jeweils 3 bis 6 Kohlenstoffatomen, oder geradkettiges oder verzweigtes Alkoxycarbonyl mit bis zu 6 Kohlenstoffatomen bedeutet, oder geradkettiges oder verzweigtes Alkyl oder Alkenyl mit jeweils bis zu 10 Kohlenstoffatomen bedeutet, die gegebenenfalls durch Cyano, Azido, Trifluormethyl, Pyridyl, Halogen, Hydroxy, Carboxyl, geradkettiges oder verzweigtes Alkoxycarbonyl mit bis zu 6 Kohlenstoffatomen, Benzyloxycarbonyl, Aryl mit 6 bis 10 Kohlenstoffatomen. Cycloalkyl mit 3 bis 6 Kohlenstoffatomen und/oder durch eine Gruppe der Formel $-(CO)_c-NR^{19}R^{20}$, $R^{21}-N-SO_2-R^{22}$, $R^{23}R^{24}-N-SO_2-$, $R^{25}-S(O)_d-$ oder

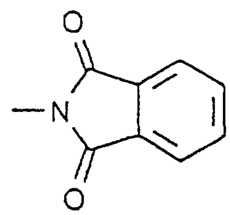

substituiert sind,
worin

c eine Zahl 0 oder 1 bedeutet,

$R^{19}$, $R^{20}$ und $R^{21}$ die oben angegebene Bedeutung von $R^{17}$ und $R^{18}$ haben und mit dieser gleich oder verschieden sind,
oder gemeinsam mit dem Stickstoffatom einen 5- bis 6-gliedrigen, gesättigten Heterocyclus mit gegebenenfalls einem weiteren Heteroatom aus der Serie N, S und/oder O bilden, der seinerseits gegebenenfalls, auch an einem weiteren Stickstoffatom, durch geradkettiges oder verzweigtes Alkyl oder Acyl mit bis zu 3 Kohlenstoffatomen substituiert sein kann,

$R^{23}$ und $R^{24}$ die oben angegebene Bedeutung von $R^{17}$ und $R^{18}$ haben und mit dieser gleich oder verschieden sind,

d eine Zahl 0, 1 oder 2 bedeutet,

$R^{22}$ und $R^{25}$ gleich oder verschieden sind und geradkettiges oder verzweigtes Alkyl mit bis zu 4 Kohlenstoffatomen, Benzyl, Phenyl oder Tolyl bedeuten,
oder

$R^{12}$ einen Rest der Formeln

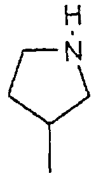 , 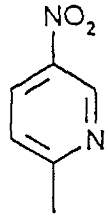 , 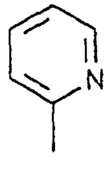 , 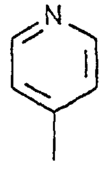 .

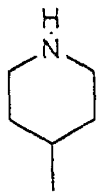, 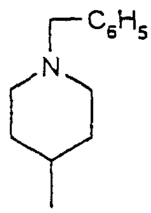 oder 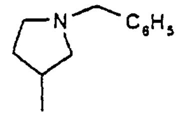.

bedeutet oder
eine Gruppe der Formel $-COCCl_3$ oder geradkettiges oder verzweigtes Acyl mit bis zu 6 Kohlenstoffatomen bedeutet, das gegebenenfalls Trifluormethyl, Trichlormethyl oder durch eine Gruppe der Formel $-OR^{26}$ substituiert ist, worin

$R^{26}$ Wasserstoff oder geradkettiges oder verzweigtes Alkyl mit bis zu 6 Kohlenstoffatomen bedeutet, das gegebenenfalls durch Aryl mit bis zu 10 Kohlenstoffatomen substituiert ist,
oder

$R^{12}$ eine Gruppe der Formel $-(CO)_c-NR^{27}R^{28}$, $-NR^{29}-SO_2R^{30}$, $R^{31}R^{32}-N-SO_2-$ oder $R^{33}-S(O)_f$ bedeutet,
worin

e die oben angegebene Bedeutung von c hat und mit dieser gleich oder verschieden ist,

$R^{27}$ und $R^{28}$ und $R^{29}$ jeweils die oben angegebene Bedeutung von $R^{19}$, $R^{20}$ und $R^{21}$ haben und mit dieser gleich oder verschieden sind,

$R^{31}$ und $R^{32}$ die oben angegebene Bedeutung von $R^{17}$ und $R^{18}$ haben und mit dieser gleich oder verschieden sind,

f die oben angegebene Bedeutung von d hat und mit dieser gleich oder verschieden ist,

$R^{30}$ und $R^{33}$ die jeweils oben angegebene Bedeutungen von $R^{22}$ und $R^{25}$ haben und mit dieser gleich oder verschieden sind,

D ein Sauerstoff oder Schwefelatom bedeutet,

E ein Sauerstoff- oder Schwefelatom oder eine Gruppe der Formel NH bedeutet,
oder im Fall, daß $R^{12}$ nicht für Wasserstoff steht, E eine Gruppe der Formel $NR^{34}$ bedeutet, worin $R^{34}$ mit Ausnahme von Wasserstoff die oben angegebene Bedeutung von $R^{12}$ hat und mit dieser gleich oder verschieden ist,
oder

$R^{34}$ Cyano oder eine Gruppe der Formel $-CO_2R^{35}$ bedeutet,
worin

$R^{35}$ Benzyl oder Phenyl bedeutet, die gegebenenfalls durch Nitro oder Halogen substituiert sind,

mit der Maßgabe, daß $R^1$ nicht für Hydroxy steht, wenn E NH bedeutet und gleichzeitig $R^{12}$ Wasserstoff bedeutet,
und deren tautomeren Formen, Isomere und Salze.

**2.** Verbindungen nach Anspruch 1, **dadurch gekennzeichnet, daß**

$R^1$ für Azido, Hydroxy oder für eine Gruppe der Formel $-OR^2$, $O-SO_2R^3$ oder $-NR^4R^5$ steht, worin

$R^2$ geradkettiges oder verzweigtes Acyl mit bis zu 6 Kohlenstoffatomen oder Benzyl bedeutet,

$R^3$ geradkettiges oder verzweigtes Alkyl mit bis zu 3 Kohlenstoffatomen, Phenyl oder Toluolyl bedeutet

$R^4$ und $R^5$ gleich oder verschieden sind und Cyclopropyl, Cyclobutyl, Cyclopentyl, Cyclohexyl, Wasserstoff, Phenyl oder geradkettiges oder verzweigtes Alkyl oder Alkoxy mit jeweils bis zu 6 Kohlenstoffatomen, tert.Butoxycarbonyl oder Benzyloxycarbonyl bedeuten,
oder

$R^4$ oder $R^5$ eine Gruppe der Formel $-CO-R^6$, $P(O)(OR^7)(OR^8)$ oder $-SO_2-R^9$ bedeutet, worin

$R^6$ Cyclopropyl, Fluor-substituiertes Cyclpropyl, Cyclobutyl, Cyclopentyl, Cyclohexyl, Trifluormethyl oder geradkettiges oder verzweigtes Alkoxy mit bis zu 6 Kohlenstoffatomen, Phenyl, Benzyloxy oder Wasserstoff bedeutet, oder

$R^6$ geradkettiges oder verzweigtes Alkyl oder Alkenyl mit jeweils bis zu 6 Kohlenstoffatomen bedeutet, die gegebenenfalls durch Cyano, Fluor, Chlor, Brom oder Trifluormethyl substituiert sind, oder
geradkettiges oder verzweigtes Thioalkyl oder Acyl mit jeweils bis zu 5 Kohlenstoffatomen bedeutet, oder
eine Gruppe der Formel $-NR^{10}R^{11}$ bedeutet,
worin

$R^{10}$ und $R^{11}$ gleich oder verschieden sind und Wasserstoff, Phenyl oder geradkettiges oder verzweigtes Alkyl mit bis zu 4 Kohlenstoffatomen bedeuten, oder

$R^6$ Isoxazolyl, Furyl, Thienyl, Pyrryl, Oxazolyl oder Imidazolyl bedeutet, die gegebenenfalls durch Methyl

substituiert sind,

$R^7$ und $R^8$ gleich oder verschieden sind und Wasserstoff oder geradkettiges oder verzweigtes Alkyl mit bis zu 3 Kohlenstoffatomen bedeuten,

$R^9$ geradkettiges oder verzweigtes Alkyl mit bis zu 3 Kohlenstoffatomen oder Phenyl bedeutet,

A für einen Rest der Formel

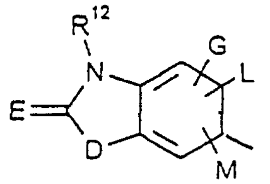

,

steht,
worin

G, L und M gleich oder verschieden sind und für Wasserstoff, Carboxy, Fluor, Chlor, Brom, Jod, Cyano, Trifluormethyl, Formyl, Nitro, für geradkettiges oder verzweigtes Alkyl mit bis zu 4 Kohlenstoffatomen oder für eine Gruppe der Formel -CO-NR$^{17}$R$^{18}$ stehen,
worin

$R^{17}$ und $R^{18}$ gleich oder verschieden sind und Wasserstoff, geradkettiges oder verzweigtes Alkyl mit bis zu 3 Kohlenstoffatomen oder Phenyl bedeuten,

$R^{12}$ Wasserstoff, Cyclopropylcarbonyl, Cyclopropyl, Cyclobutyl, Cyclopentyl, Cyclohexyl, oder
geradkettiges oder verzweigtes Alkoxycarbonyl mit bis zu 4 Kohlenstoffatomen, oder
geradkettiges oder verzweigtes Alkyl oder Alkenyl mit jeweils bis zu 9 Kohlenstoffatomen bedeutet, die gegebenenfalls durch Cyano, Azido, Trifluormethyl, Pyridyl, Fluor, Chlor, Brom, Hydroxy, Carboxyl, geradkettiges oder verzweigtes Alkoxycarbonyl mit bis zu 5 Kohlenstoffatomen, Phenyl, Naphthyl, Benzyloxycarbonyl, Cyclopropyl, Cyclopentyl, Cyclohexyl und/oder durch eine Gruppe der Formel-$(CO)_c$-NR$^{19}$R$^{20}$, R$^{21}$-N-$SO_2$-R$^{22}$, R$^{23}$R$^{24}$-N-$SO_2$-, R$^{25}$-S$(O)_d$- oder

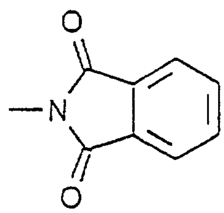

substituiert sind,
worin

c eine Zahl 0 oder 1 bedeutet,
$R^{19}$, $R^{20}$ und $R^{21}$ die oben angegebene Bedeutung von $R^{17}$ und $R^{18}$ haben und mit dieser gleich oder verschieden sind, oder gemeisan mit dem Stickstoffatom einen Morpholinyl-, Pyrrolidinyl-, Piperazinyl- oder Piperidylring bilden, die gegebenenfalls, auch über die freie N-Funktion, durch Methyl, Ethyl oder Acetyl substituiert sind,
$R^{23}$ und $R^{24}$ die oben angegebene Bedeutung von $R^{17}$ und $R^{18}$ haben und mit dieser gleich oder verschieden sind,
d eine Zahl 0, 1 oder 2 bedeutet,
$R^{22}$ und $R^{25}$ gleich oder verschieden sind und geradkettiges oder verweigtes Alkyl mit bis zu 3 Kohlenstoffatomen, Benzyl, Phenyl oder Tolyl bedeuten,

$R^{12}$ einen Rest der Formeln

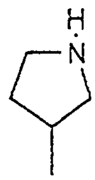 , 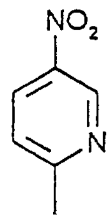 , 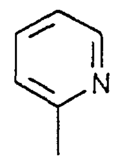 , 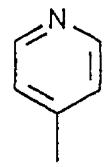 ,

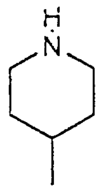 , 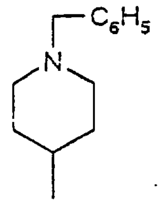 oder 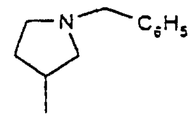

bedeutet oder
eine Gruppe der Formel $-COCCl_3$ oder geradkettiges oder verzweigtes Acyl mit bis zu 5 Kohlenstoffatomen bedeutet, das gegebenenfalls durch Trifluormethyl, Trichlormethyl oder eine Gruppe der Formel $-OR^{26}$ substituiert ist,
worin

$R^{26}$ Wasserstoff oder geradkettiges oder verzweigtes Alkyl mit bis zu 5 Kohlenstoffatomen bedeutet, das gegebenenfalls durch Phenyl oder Naphthyl substituiert ist,
oder

$R^{12}$ eine Gruppe der Formel $-(CO)_e-NR^{27}R^{28}$, $-NR^{29}-SO_2R^{30}$, $R^{31}R^{32}-N-SO_2-$ oder $R^{33}-S(O)_f$ bedeutet,
worin

e die oben angegebene Bedeutung von c hat und mit dieser gleich oder verschieden ist,

$R^{27}$, $R^{28}$ und $R^{29}$ die jeweils oben angegebene Bedeutung von $R^{19}$, $R^{20}$ und $R^{21}$ haben und mit dieser gleich oder verschieden sind,

$R^{31}$ und $R^{32}$ die oben angegebene Bedeutung von $R^{17}$ und $R^{18}$ haben und mit dieser gleich oder verschieden sind,

f die oben angegebene Bedeutung von d hat und mit dieser gleich oder verschieden ist,

$R^{30}$ und $R^{33}$ die jeweils oben angegebene Bedeutungen von $R^{22}$ und $R^{25}$ haben und mit dieser gleich oder verschieden sind,

D ein Sauerstoff oder Schwefelatom bedeutet,

E ein Sauerstoff- oder Schwefelatom oder eine Gruppe der Formel NH bedeutet,
oder im Fall, **daß** $R^{12}$ nicht für Wasserstoff steht, E eine Gruppe der Formel $NR^{34}$ bedeutet, worin $R^{34}$ mit Ausnahme von Wasserstoff die oben angegebene Bedeutung von $R^{12}$ hat und mit dieser gleich oder verschieden ist, oder

$R^{34}$ Cyano oder eine Gruppe der Formel $-CO_2R^{35}$ bedeutet, worin

$R^{35}$ Benzyl oder Phenyl bedeutet, die gegebenenfalls durch Nitro, Fluor, Chlor oder Brom substituiert sind,

mit der Maßgabe, **daß** $R^1$ nicht für Hydroxy steht, wenn E NH bedeutet und gleichzeitig $R^{12}$ Wasserstoff bedeutet, und deren tautomeren Formen. Isomere und Salze.

**3.** Verbindungen nach Anspruch 1, **dadurch gekennzeichnet, daß**

$R^1$ für Azido, Hydroxy oder für eine Gruppe der Formel $-OR^2$, $O-SO_2R^3$ oder $-NR^4R^5$ steht, worin

$R^2$ geradkettiges oder verzweigtes Acyl mit bis zu 5 Kohlenstoffatomen oder Benzyl bedeutet,

$R^3$ Methyl, Ethyl, Phenyl oder Toluolyl bedeutet,

$R^4$ und $R^5$ gleich oder verschieden sind und Cyclopropyl, Cyclobutyl, Cyclopentyl, Cyclohexyl, Wasserstoff, Phenyl oder geradkettiges oder verzweigtes Alkyl oder Alkoxy mit jeweils bis zu 5 Kohlenstoffatomen, tert.Butoxycarbonyl oder Benzyloxycarbonyl bedeuten,
oder

$R^4$ oder $R^5$ eine Gruppe der Formel $-CO-R^6$, $P(O)(OR^7)(OR^8)$ oder $-SO_2R^9$ bedeutet, worin

$R^6$ Cyclopropyl, Fluor-substituiertes Cyclopropyl, Cyclobutyl, Cyclopentyl, Cyclohexyl, Trifluomethyl oder geradkettiges oder verzweigtes Alkoxy mit bis zu 5 Kohlenstoffatomen, Phenyl, Benzyloxy oder Wasserstoff bedeutet,

$R^6$ geradkettiges oder verzweigtes Alkyl oder Alkenyl mit jeweils bis zu 5 Kohlenstoffatomen bedeutet, die gegebenenfalls durch Cyano, Fluor, Chlor, Brom oder Trifluormethyl substituiert sind, oder
geradkettiges oder verzweigtes Thioalkyl- oder Acyl mit jeweils bis zu 4 Kohlenstoffatomen bedeutet, oder
eine Gruppe der Formel $-NR^{10}R^{11}$ bedeutet, worin

$R^{10}$ und $R^{11}$ gleich oder verschieden sind und Wasserstoff, Phenyl oder geradkettiges oder verzweigtes Alkyl mit bis zu 3 Kohlenstoffatomen bedeuten,
oder

$R^6$ Isoxazolyl, Furyl, Oxazolyl oder Imidazolyl bedeutet, die gegebenenfalls durch Methyl substituiert sind,

$R^7$ und $R^8$ gleich oder verschieden sind und Wasserstoff, Methyl oder Ethyl bedeuten,

$R^9$ Methyl oder Phenyl bedeutet,

A für einen Rest der Formel

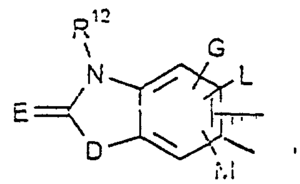

steht,
worin

G, L und M gleich oder verschieden sind und für Wasserstoff, Carboxy, Fluor, Chlor, Brom, Jod, Cyano, Formyl, Nitro, für geradkettiges oder verzweigtes Alkyl mit bis zu 3 Kohlenstoffatomen oder für eine Gruppe $-CO-NH_2$ stehen,

$R^{12}$ Wasserstoff, Cyclopropylcarbonyl, Cyclopropyl, Cyclobutyl, Cyclopentyl, Cyclohexyl, oder
geradkettiges oder verzweigtes Alkoxycarbonyl mit bis zu 3 Kohlenstoffatomen, oder
geradkettiges oder verzweigtes Alkyl oder Alkenyl mit jeweils bis zu 8 Kohlenstoffatomen bedeutet, die gegebenenfalls durch Cyano, Azido, Trifluormethyl, Pyridyl, Fluor, Chlor, Brom, Hydroxy, Carboxyl, geradkettiges oder verzweigtes Alkoxycarbonyl mit bis zu 4 Kohlenstoffatomen, Phenyl, Benzyloxycarbonyl, Cyclopropyl, Cyclopentyl, Cyclohexyl und/oder durch eine Gruppe der Formel $-(CO)_c-NR^{19}R^{20}$, $R^{21}-N-SO_2-R^{22}$, $R^{23}R^{24}-N-SO_2-$, $R^{25}-S(O)_d-$ oder

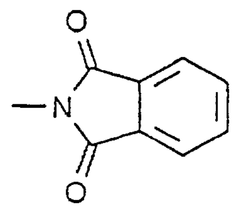

substituiert ist,
worin

c eine Zahl 0 oder 1 bedeutet,

$R^{19}$, $R^{20}$, $R^{21}$, $R^{23}$ und $R^{24}$ gleich oder verschieden sind und Wasserstoff, Methyl oder Ethyl bedeuten,

d eine Zahl 0, 1 oder 2 bedeutet,

$R^{22}$ und $R^{25}$ gleich oder verschieden sind und geradkettiges oder verzweigtes Alkyl mit bis zu 4 Kohlenstoffatomen, Benzyl, Phenyl oder Tolyl bedeuten, oder

$R^{12}$ einen Rest der Formeln

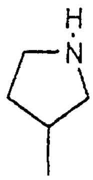 , 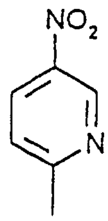 , 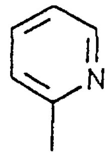 , 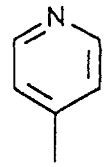 ,

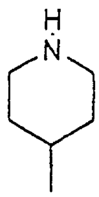 , 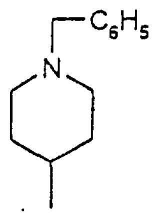 oder 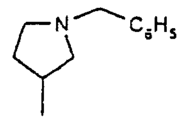

bedeutet oder
eine Gruppe der Formel $-COCCl_3$ oder geradkettiges oder verzweigtes Acyl mit bis zu 4 Kohlenstoffatomen bedeutet, das gegebenenfalls durch Trifluormethyl, Trichlormethyl, eine Gruppe der Formel $-OR^{26}$ substituiert ist,
worin

$R^{26}$ Wasserstoff oder geradkettiges oder verzweigtes Alkyl mit bis zu 4 Kohlenstoffatomen bedeutet, das gegebenenfalls durch Phenyl substituiert ist,
oder

$R^{12}$ eine Gruppe der Formel $-(CO)_e-NR^{27}R^{28}$ oder $R^{33}-S(O)_f$ bedeutet,
worin

e die Zahl 1 bedeutet,

$R^{27}$ und $R^{28}$ gleich oder verschieden sind und Wasserstoff; Methyl oder Ethyl bedeuten,

f die oben angegebene Bedeutung von d hat und mit dieser gleich oder verschieden ist,

$R^{33}$ Methyl, Phenyl, Tolyl oder Benzyl bedeutet,

D ein Sauerstoff oder Schwefelatom bedeutet,

E ein Sauerstoff- oder Schwefelatom oder eine Gruppe der Formel NH bedeutet,
oder im Fall, **daß** $R^{12}$ nicht für Wasserstoff steht, E eine Gruppe der Formel $NR^{34}$ bedeutet, worin $R^{34}$ mit Ausnahme von Wasserstoff die oben angegebene Bedeutung von $R^{12}$ hat und mit dieser gleich oder verschieden ist, oder

$R^{34}$ Cyano oder eine Gruppe der Formel $-CO_2R^{35}$ bedeutet,
worin

$R^{35}$ Benzyl oder Phenyl bedeutet, die gegebenenfalls durch Nitro substituiert sind,

mit der Maßgabe, **daß** $R^1$ nicht für Hydroxy steht, wean E NH bedeutet und gleichzeitig $R^{12}$ Wasserstoff beteutet, und deren tautomere Formen, Isomere und Salze.

**4.** Verbindungen nach Anspruch 1, **dadurch gekennzeichnet, daß**

G, L und M für Wasserstoff stehen,

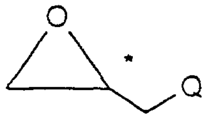

(IV)

**5.** Verfahren zur Herstellung von Verbindungen der Ansprüche 1 bis 4, **dadurch gekennzeichnet, daß** man

[A] Verbindungen der allgemeinen Formeln (II) oder (III)

A-N=C=O (II)

oder

$A\text{-}CO\text{-}N_3$ (III)

in welchen

A die oben angegebene Bedeutungen hat,

mit Lithiumbromid/$(C_4H_9)_3$ P(O) und Epoxiden der allgemeinen Formel (IV)

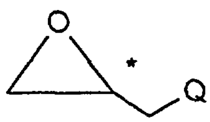

(IV)

in welcher

Q für $C_1$-$C_6$-Acyloxy steht,

in inerten Lösemitteln, gegebenenfalls in Anwesenheit einer Base
umsetzt,
und im Fall $R^1$ = OH durch eine typische Esterverseifung oder durch eine typische Umesterung die Hydroxyfunktion freisetzt,
oder

[B] Verbindungen der allgemeinen Formel (V)

$A\text{-}NH\text{-}CO_2\text{-}X$ (V)

in welcher

A die oben angegebene Bedeutung hat
und

X für eine typische Schutzgruppe, vorzugsweise Benzyl steht,

in inerten Lösemitteln und in Anwesenheit einer Base, beispielsweise Lithiumalkylen oder Lithium-N-alkyl- oder Lithium-N-silylalkylamiden, vorzugsweise N-Butyllithium, mit Epoxiden der allgemeinen Formel (IV) umsetzt,
oder

[C] im Fall $R^1$ = OH, zunächst Verbindungen der allgemeinen Formel (III) durch Abspaltung von Stickstoff in Alkoholen in die Verbindungen der allgemeinen Formel (Va)

$$A\text{-}NH\text{-}CO_2\text{-}Y \qquad (Va)$$

in welcher

A die oben angegebene Bedeutung hat
und

Y für geradkettiges oder verzweigtes $C_2$-$C_6$-Alkyl, vorzugsweise n-Butyl steht,

überführt,
und in einem zweiten Schritt wie unter [A] beschrieben in inerten Lösemitteln und in Anwesenheit einer Base, vorzugsweise Lithium-N-alkyl-oder N-Silylalkylamiden oder n-Butyllithium und Epoxiden der allgemeinen Formel (IV) umsetzt, oder

[D] Verbindungen der allgemeinen Formel (VI)

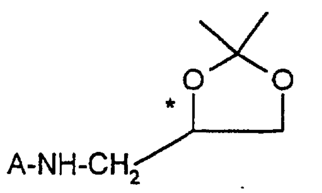

(VI)

in welcher

A die oben angegebene Bedeutung hat,

entweder direkt mit Säuren und Kohlensäurediethylester
umsetzt,
oder zunächst durch Umsetzung der Verbindungen der allgemeinen Formel (VI) mit Säuren die Verbindungen der allgemeinen Formel (VII)

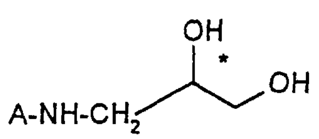

(VII)

in welcher

A die oben angegebene Bedeutung hat,

herstellt,
und anschließend in Anwesenheit eines Hilfsmittels in inerten Lösemitteln cyclisiert,

oder

[E] zunächst Verbindungen der allgemeinen Formel (Ia)

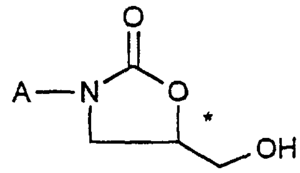

(Ia)

in welcher

A die oben angegebene Bedeutung hat,

durch Umsetzung mit ($C_1$-$C_4$)-Alkyl- oder Phenylsulfonsäurechloriden in inerten Lösemitteln und in Anwesenheit einer Base in die entsprechenden Verbindungen der allgemeinen Formel (Ib)

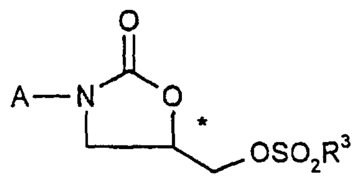

(Ib)

in welcher

A und $R^3$ die oben angegebene Bedeutung haben,

überführt,
anschließend mit Natriumazid in inerten Lösemitteln die Azide der allgemeinen Formel (Ic)

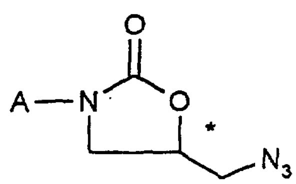

(Ic)

in welcher

A die oben angegebene Bedeutung hat,

herstellt,
in einem weiteren Schritt durch Umsetzung mit $(C_1\text{-}C_4\text{-}O)_3$-P oder $PPh_3$, vorzugsweise $(CH_3O)_3P$ in inerten Lösemitteln und mit Säuren in die Amine der allgemeinen Formel (Id)

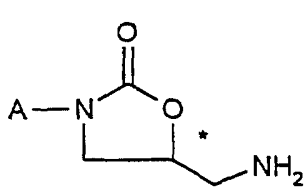

(Id)

in welcher

A die oben angegebene Bedeutung hat,

überführt,
und durch Umsetzung mit Acetanhydrid oder anderen Acylierungsmitteln der allgemeinen Formel (VIII)

$$R^{36}\text{-CO-}R^{6} \qquad \text{(VIII)}$$

in welcher

$R^6$ die oben angegebene Bedeutung hat
und
$R^{36}$ für Halogen, vorzugsweise für Chlor oder für den Rest -OCOR$^6$ steht,

in inerten Lösemitteln die Verbindungen der allgemeinen Formel (Ie)

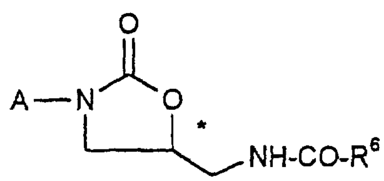

(Ie)

in welcher

A und $R^6$ die oben angegebene Bedeutung haben,

herstellt,
oder

[F] im Fall

A = 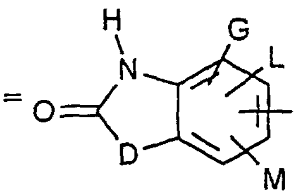 oder 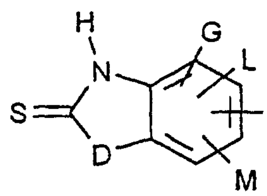

Verbindungen der allgemeinen Formel (IX)

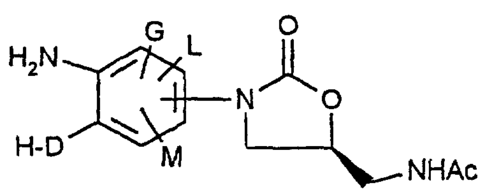

(IX)

in welcher

G, L, M und D die oben angegebene Bedeutung haben,

entweder mit Carbonyldiimidazol bzw. Thiocarbonyldiimidazol in Dimethylformamid oder durch Umsetzung mit $KS-CO_2-C_2H_5$ / $CH_3OH$ und anschließender Zugabe von Wasser cyclisiert,
in Fall

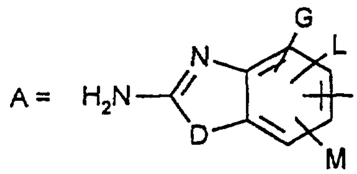

+ die Verbindungen der allgemeinen Formel (IX) mit BrCN / $H_2O$ / $CH_3OH$ umsetzt,
oder

[G] im Fall $R^{12} \neq H$, ausgehend von den Verbindungen mit $R^1$ = NH-$COCH_3$ eine Acylierung oder eine Alkylierung unter Doppelbindungsverschiebung durchführt, oder
Verbindungen der allgemeinen Formel (I) mit dem Rest

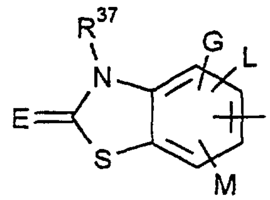

worin

$R^{37}$ $C_1$-$C_{10}$-Alkyl, vorzugsweise $C_1$-$C_3$-Alkyl bedeutet,

und E = O,
Verbindungen der allgemeinen Formel (X)

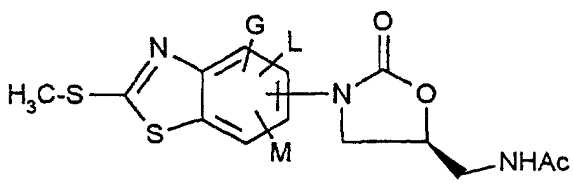

(X)

in welcher

G, L und M die oben angegebene Bedeutung haben,

zunächst durch Umsetzung mit $C_1$-$C_{10}$-Alkylhalogeniden, bevorzugt $C_1$-$C_3$-Alkyljodiden, in inerten Lösemitteln in die Salze der Verbindungen der allgemeinen Formel (XI)

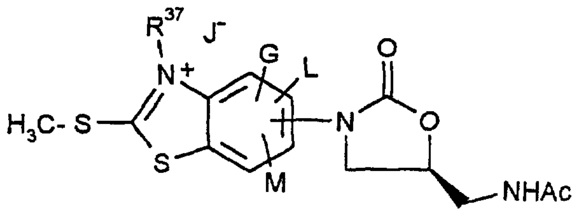

(XI)

in welcher

$R^{37}$ für $C_1$-$C_{10}$-Alkyl, vorzugsweise für $C_1$-$C_3$-Alkyl steht, und

G, L und M die oben angegebene Bedeutung haben,

überführt,
und in einem letzten Schritt mit Methanol zur Reaktion bringt,
und im Fall E = S Verbindungen der allgemeinen Formel (XI) einer Thermolyse unterzieht,
und im Fall der S-Oxide eine Oxidation nach üblicher Methode durchführt,
und gegebenenfalls weitere Substituenten oder bereits vorhandene funktionelle Gruppen nach üblichen Methoden, wie beispielsweise Alkylierung, Redoxreaktionen, Substitutionsreaktionen und/oder Verseifungen oder Ein- und Abbau von Schutzgruppen, einführt bzw. derivatisiert.

**6.** Verwendung der Verbindungen nach den Ansprüchen 1 bis 4 zur Herstellung von Arzneimitteln.

**7.** Arzneimittel, enthaltend Verbindungen gemäß den Ansprüchen 1 bis 4.

## Claims

**1.** Compounds of the general formula (I)

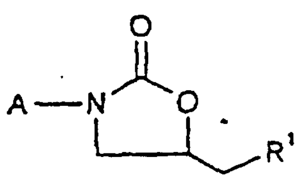

(I)

in which

$R^1$ represents azido, hydroxyl or represents a group of the formula $-OR^2$, $O-SO_2R^3$ or $-NR^4R^5$, in which

$R^2$ denotes straight-chain or branched acyl having up to 8 carbon atoms, or a hydroxy-protecting group,

$R^3$ denotes straight-chain or branched alkyl having up to 4 carbon atoms or phenyl which is optionally substituted by straight-chain or branched alkyl having up to 4 carbon atoms,

$R^4$ and $R^5$ are identical or different and denote cycloalkyl having 3 to 6 carbon atoms, hydrogen, phenyl or straight-chain or branched alkyl or alkoxy having in each case up to 8 carbon atoms, or an amino-protecting group,
or

$R^4$ or $R^5$ denotes a group of the formula $-CO-R^6$, $P(O)(OR^7)(OR^8)$ or $-SO_2-R^9$, in which

$R^6$ denotes cycloalkyl or halogen-substituted cycloalkyl having in each case 3 to 6 carbon atoms, trifluoromethyl, straight-chain or branched alkoxy having up to 8 carbon atoms, phenyl, benzyloxy or hydrogen, or

$R^6$ denotes straight-chain or branched alkyl or alkenyl having in each case up to 8 carbon atoms which are optionally substituted by cyano, halogen or trifluoromethyl,
or
denotes straight-chain or branched thioalkyl or acyl having in each case up to 6 carbon atoms,
or
denotes a group of the formula $-NR^{10}R^{11}$, in which

$R^{10}$ and $R^{11}$ are identical or different and denote hydrogen, phenyl or straight-chain or branched alkyl having up to 6 carbon atoms,
or

$R^6$ denotes a 5-membered aromatic heterocycle having up to 3 heteroatoms from the series S, N and/or O, which is optionally substituted by straight-chain or branched alkyl having up to 3 carbon atoms,

$R^7$ and $R^8$ are identical or different and denote hydrogen or straight-chain or branched alkyl having up to 4 carbon atoms,

$R^9$ denotes straight-chain or branched alkyl having up to 4 carbon atoms, or phenyl,

A represents a radical of the formula

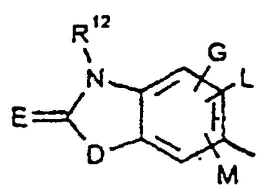

in which

G, L and M are identical or different and represent hydrogen, carboxyl, halogen, cyano, formyl, trifluoromethyl, nitro, represent straight-chain or branched alkyl having up to 6 carbon atoms, or represent a group of the formula $-CO-NR^{17}R^{18}$, in which

$R^{17}$ and $R^{18}$ are identical or different and denote hydrogen, straight-chain or branched alkyl having up to 4 carbon atoms, or phenyl,

$R^{12}$ denotes hydrogen, cycloalkylcarbonyl or cycloalkyl having in each case 3 to 6 carbon atoms, or straight-chain or branched alkoxycarbonyl having up to 6 carbon atoms, or denotes straight-chain or branched alkyl or alkenyl having in each case up to 10 carbon atoms, which is optionally substituted by cyano, azido, trifluoromethyl, pyridyl, halogen, hydroxyl, carboxyl, straight-chain or branched alkoxycarbonyl having up to 6 carbon atoms, benzyloxycarbonyl, aryl having 6 to 10 carbon atoms, cycloalkyl having 3 to 6 carbon atoms and/or by a group of the formula $-(CO)_c-NR^{19}R^{20}$, $R^{21}-N-SO_2-R^{22}$, $R^{23}R^{24}-N-SO_2-$, $R^{25}-S(O)_d$ or

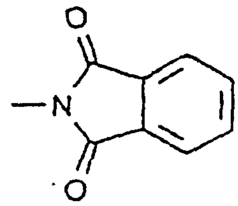

in which

c — is a number 0 or 1,

$R^{19}$, $R^{20}$ and $R^{21}$ — have the meaning given above of $R^{17}$ and $R^{18}$ and are identical to or different from it,
or together with the nitrogen atom form a 5- to 6-membered, saturated heterocycle having optionally a further heteroatom from the series N, S and/or 0, which can in turn optionally be substituted, even on a further nitrogen atom, by straight-chain or branched alkyl or acyl having up to 3 carbon atoms,

$R^{23}$ and $R^{24}$ — have the meaning given above of $R^{17}$ and $R^{18}$ and are identical to or different from it,

d — denotes a number 0, 1 or 2,

$R^{22}$ and $R^{25}$ — are identical or different and denote straight-chain or branched alkyl having up to 4 carbon atoms, benzyl, phenyl or tolyl,
or

$R^{12}$ — denotes a radical of the formulae

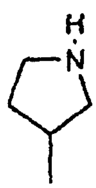 , 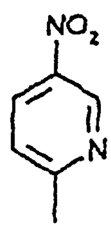 , 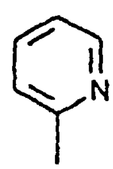 , 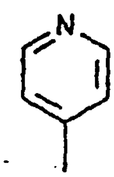 ,

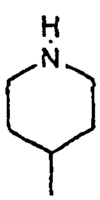 , 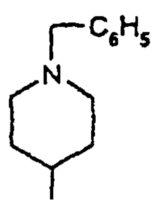 or 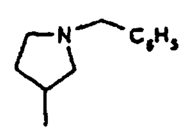 .

or
denotes a group of the formula $-COCCl_3$ or straight-chain or branched acyl having up to 6 carbon atoms which is optionally substituted by trifluoromethyl, trichloromethyl or by a group of the formula $-OR^{26}$,
in which

$R^{26}$ — denotes hydrogen or straight-chain or branched alkyl having up to 6 carbon atoms which is optionally substituted by aryl having

up to 10 carbon atoms,
or

$R^{12}$ denotes a group of the formula - $(CO)_e$-$NR^{27}R^{28}$, -$NR^{29}$-$SO_2R^{30}$, $R^{31}R^{32}$-N-$SO_2$- or $R^{33}$-$S(O)_f$,
in which

e has the meaning given above of c and is identical to or different from it,

$R^{27}$ and $R^{28}$ and $R^{29}$ each have the meaning given above of $R^{19}$, $R^{20}$ and $R^{21}$ and are identical to or different from it,

$R^{31}$ and $R^{32}$ have the meaning given above of $R^{17}$ and $R^{18}$ and are identical to or different from it,

f has the meaning given above of d and is identical to or different from it,

$R^{30}$ and $R^{33}$ have the meanings in each case given above of $R^{22}$ and $R^{25}$ and are identical to or different from these,

D denotes an oxygen atom or sulphur atom,

E denotes an oxygen or sulphur atom or a group of the formula NH,
or in the case where $R^{12}$ does not represent hydrogen, E denotes a group of the formula $NR^{34}$ in which $R^{34}$ with the exception of hydrogen has the meaning given above of $R^{12}$ and is identical to or different from it,
or

$R^{34}$ denotes cyano or a group of the formula -$CO_2R^{35}$,
in which

$R^{35}$ denotes benzyl or phenyl which are optionally substituted by nitro or halogen,

with the proviso that $R^1$ does not represent hydroxyl if E denotes NH and at the same time $R^{12}$ denotes hydrogen,

and the tautomeric forms, isomers and salts thereof.

**2.** Compounds according to Claim 1, **characterized in that**

$R^1$ represents azido, hydroxyl or represents a group of the formula -$OR^2$, O-$SO_2R^3$ or -$NR^4R^5$,
in which

$R^2$ denotes straight-chain or branched acyl having up to 6 carbon atoms, or benzyl,

$R^3$ denotes straight-chain or branched alkyl having up to 3 carbon atoms, phenyl or tolyl,

$R^4$ and $R^5$ are identical or different and denote cyclopropyl, cyclobutyl, cyclopentyl, cyclohexyl, hydrogen, phenyl or straight-chain or branched alkyl or alkoxy having in each case up to 6 carbon atoms, tert-butoxycarbonyl or benzyloxycarbonyl,
or

$R^4$ or $R^5$ denotes a group of the formula -CO-$R^6$, P(O) $(OR^7)$ $(OR^8)$ or -$SO_2$-$R^9$,
in which

$R^6$ denotes cyclopropyl, fluorine-substituted cyclopropyl, cyclobutyl, cyclopentyl, cyclohexyl, trifluoromethyl or straight-chain or branched alkoxy having up to 6 carbon atoms, phenyl, benzyloxy or hydrogen, or

$R^6$ denotes straight-chain or branched alkyl or alkenyl having in each case up to 6 carbon atoms which are optionally substituted by cyano, fluorine, chlorine, bromine or trifluoromethyl, or denotes straight-chain or branched thioalkyl or acyl having in each case up to 5 carbon atoms, or
denotes a group of the formula $-NR^{10}R^{11}$,
in which

$R^{10}$ and $R^{11}$ are identical or different and denote hydrogen, phenyl or straight-chain or branched alkyl having up to 4 carbon atoms, or

$R^6$ denotes isoxazolyl, furyl, thienyl, pyrryl, oxazolyl or imidazolyl which are optionally substituted by methyl,

$R^7$ and $R^8$ are identical or different and denote hydrogen or straight-chain or branched alkyl having up to 3 carbon atoms,

$R^9$ denotes straight-chain or branched alkyl having up to 3 carbon atoms, or phenyl,

A represents a radical of the formula

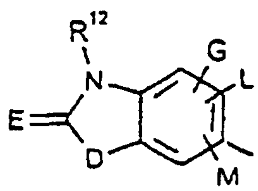

in which

G, L and M are identical or different and represent hydrogen, carboxyl, fluorine, chlorine, bromine, iodine, cyano, trifluoromethyl, formyl, nitro, represent straight-chain or branched alkyl having up to 4 carbon atoms, or represent a group of the formula $-CO-NR^{17}R^{18}$,
in which

$R^{17}$ and $R^{18}$ are identical or different and denote
hydrogen, straight-chain or branched alkyl having up to 3 carbon atoms, or phenyl,

$R^{12}$ denotes hydrogen, cyclopropylcarbonyl or cyclopropyl, cyclobutyl, cyclopentyl, cyclohexyl, or straight-chain or branched alkoxycarbonyl having up to 4 carbon atoms, or
straight-chain or branched alkyl or alkenyl having in each case up to 9 carbon atoms which are optionally substituted by cyano, azido, trifluoromethyl, pyridyl, fluorine, chlorine, bromine, hydroxyl, carboxyl, straight-chain or branched alkoxycarbonyl having up to 5 carbon atoms, naphthyl, benzyloxycarbonyl, cyclopropyl, cyclopentyl, cyclohexyl and/or by a group of the formula $-(CO)_c-NR^{19}R^{20}$, $R^{21}-N-SO_2-R^{22}$, $R^{23}R^{24}-N-SO_2-$, $R^{25}-S(O)_d-$ or

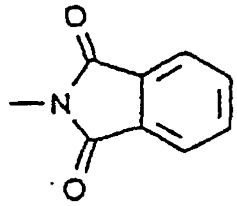

in which

| | |
|---|---|
| C | denotes a number 0 or 1, |
| $R^{19}$, $R^{20}$ and $R^{21}$ | have the meaning given above of $R^{17}$ and $R^{18}$ and are identical to or different from it, or together with the nitrogen atom form a morpholinyl, pyrrolidinyl, piperazinyl or piperidyl ring which are optionally substituted, even via the free N function, by methyl, ethyl or acetyl, |
| $R^{23}$ and $R^{24}$ | have the meaning given above of $R^{17}$ and $R^{18}$ and are identical to or different from it, |
| d | denotes a number 0, 1 or 2, |
| $R^{22}$ and $R^{25}$ | are identical or different and denote straight-chain or branched alkyl having up to 3 carbon atoms, benzyl, phenyl or tolyl, or |
| $R^{12}$ | denotes a radical of the formulae |

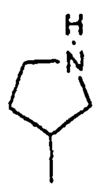 ,
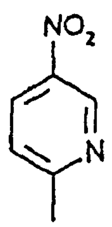 ,
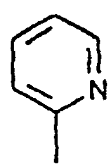 ,
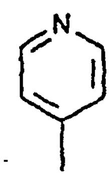 ,

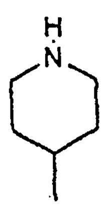 ,
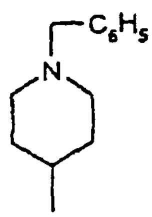 or
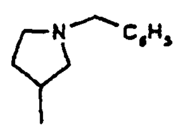 .

or
denotes a group of the formula $-COCCl_3$ or straight-chain or branched acyl having up to 5 carbon atoms which is optionally substituted by trifluoromethyl, trichloromethyl or a group of the formula $-OR^{26}$,
in which

$R^{26}$ denotes hydrogen or straight-chain or branched alkyl having up to 5 carbon atoms which is optionally substituted by phenyl or naphthyl,
or

$R^{12}$ denotes a group of the formula $-(CO)_e-NR^{27}R^{28}$, $-NR^{29}-SO_2R^{30}$, $R^{31}R^{32}-N-SO_2-$ or $R^{33}-S(O)_f$,
in which

e has the meaning given above of c and is identical to or different from it,

$R^{27}$, $R^{28}$ and $R^{29}$ have the meaning in each case given above of $R^{19}$, $R^{20}$ and $R^{21}$ and are identical to or different from it,

$R^{31}$ and $R^{32}$ have the meaning given above of $R^{17}$ and $R^{18}$ and are identical to or different from it,

f has the meaning given above of d and is identical to or different from it,

$R^{30}$ and $R^{33}$ have the meanings in each case given above of $R^{22}$ and $R^{25}$ and are identical to or different from these,

D denotes an oxygen or sulphur atom,

E denotes an oxygen or sulphur atom or a group of the formula NH,
or, in the case where $R^{12}$ does not represent hydrogen, E denotes a group of the formula $NR^{34}$ in which $R^{34}$ with the exception of hydrogen has the meaning given above of $R^{12}$ and is identical to or different from it, or

$R^{34}$ denotes cyano or a group of the formula $-CO_2R^{35}$,
in which

$R^{35}$ denotes benzyl or phenyl which are optionally substituted by nitro, fluorine, chlorine or bromine,

and the tautomeric forms, isomers and salts thereof.

**3.** Compounds according to Claim 1, **characterized in that**

$R^1$ represents azido, hydroxyl or represents a group of the formula $-OR^2$, $O-SO_2R^3$ or $-NR^4R^5$,
in which

$R^2$ denotes straight-chain or branched acyl having up to 5 carbon atoms, or benzyl,

$R^3$ denotes methyl, ethyl, phenyl or tolyl,

$R^4$ and $R^5$ are identical or different and denote cyclopropyl, cyclobutyl, cyclopentyl, cyclohexyl, hydrogen, phenyl or straight-chain or branched alkyl or alkoxy having in each case up to 5 carbon atoms, tert-butoxycarbonyl or benzyloxycarbonyl,
or

$R^4$ or $R^5$ denotes a group of the formula $-CO-R^6$, $P(O)(OR^7)(OR^8)$ or $-SO_2-R^9$,
in which

$R^6$ denotes cyclopropyl, fluorine-substituted cyclopropyl, cyclobutyl, cyclopentyl, cyclohexyl, trifluoromethyl or straight-chain or branched alkoxy having up to 5 carbon atoms, phenyl, benzyloxy or hydrogen,

$R^6$ denotes straight-chain or branched alkyl or alkenyl having in each case up to 5 carbon

atoms which are optionally substituted by cyano, fluorine, chlorine, bromine or trifluoromethyl, or
denotes straight-chain or branched thioalkyl or acyl having in each case up to 4 carbon atoms, or
denotes a group of the formula $-NR^{10}R^{11}$ in which

$R^{10}$ and $R^{11}$ are identical or different and denote hydrogen, phenyl or straight-chain or branched alkyl having up to 3 carbon atoms, or

$R^6$ denotes isoxazoly, furyl, oxazolyl or imidazolyl which are optionally substituted by methyl,

$R^7$ and $R^8$ are identical or different and denote hydrogen, methyl or ethyl,

$R^9$ denotes methyl or phenyl,

A represents a radical of the formula

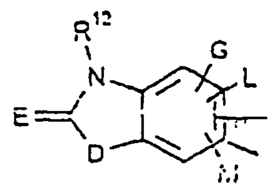

in which

G, L and M are identical or different and represent hydrogen, carboxyl, fluorine, chlorine, bromine, iodine, cyano, formyl, nitro, represent straight-chain or branched alkyl having up to 3 carbon atoms or represent a group $-CO-NH_2$,

$R^{12}$ denotes hydrogen, cyclopropylcarbonyl, cyclopropyl, cyclobutyl, cyclopentyl, cyclohexyl, or straight-chain or branched alkoxycarbonyl having up to 3 carbon atoms, or
straight-chain or branched alkyl or alkenyl having in each case up to 8 carbon atoms which is optionally substituted by cyano, azido, trifluoromethyl, pyridyl, fluorine, chlorine, bromine, hydroxyl, carboxyl, straight-chain or branched alkoxycarbonyl having up to 4 carbon atoms, phenyl, benzyloxycarbonyl, cyclopropyl, cyclopentyl, cyclohexyl and/or by a group of the formula $-(CO)_c-NR^{19}R^{20}$, $R^{21}-N-SO_2-R^{22}$, $R^{23}R^{24}-N-SO_2-$, r $R^{25}-S(O)_d-$ or

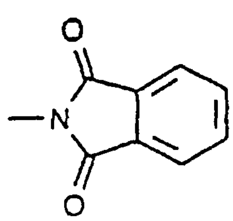

in which

C denotes a number 0 or 1,

$R^{19}$, $R^{20}$, $R^{21}$, $R^{23}$ and $R^{24}$ are identical or different and denote hydrogen, methyl or ethyl,

d denotes a number 0, 1 or 2,

$R^{22}$ and $R^{25}$ are identical or different and denote straight-chain or branched alkyl having up to 4 carbon atoms, benzyl, phenyl or tolyl,
or

$R^{12}$ denotes a radical of the formulae

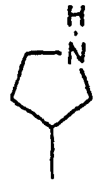 , 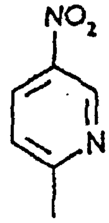 , 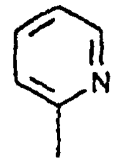 , 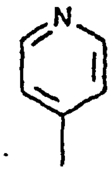 ,

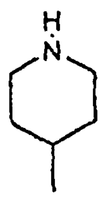 , 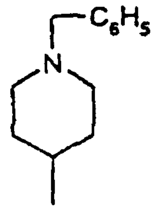 or 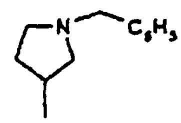 .

or
denotes a group of the formula $-COCCl_3$ or straight-chain or branched acyl having up to 4 carbon atoms which is optionally substituted by trifluoromethyl, trichloromethyl, a group of the formula $-OR^{26}$,
in which

$R^{26}$ denotes hydrogen or straight-chain or branched alkyl having up to 4 carbon atoms which is optionally substituted by phenyl,
or

$R^{12}$ denotes a group of the formula $-(CO)_e-NR^{27}R^{28}$ or $R^{33}-S(O)_f$,

e denotes the number 1,

$R^{27}$ and $R^{28}$ are identical or different and denote hydrogen, methyl or ethyl,

f has the meaning given above of d and is identical to or different from it,

$R^{33}$ denotes methyl, phenyl, tolyl or benzyl,

D denotes an oxygen or sulphur atom,

E denotes an oxygen or sulphur atom or a group of the formula NH,
or, in the case where $R^{12}$ does not represent hydrogen, E denotes a group of the formula $NR^{34}$ in which $R^{34}$ with the exception of hydrogen has the meaning given above of $R^{12}$ and is identical to or different from it, or

$R^{34}$ denotes cyano or a group of the formula $-CO_2R^{35}$, in which

$R^{35}$ denotes benzyl or phenyl which are optionally substituted by nitro,

and the tautomeric forms, isomers and salts thereof.

**4.** Compounds according to Claim 1, **characterized in that**

G, L and M represent hydrogen.

**5.** Process for the preparation of compounds of Claims 1 to 4, **characterized in that**

[A] compounds of the general formulae (II) or (III)

A-N=C=O (II)

or

$A-CO-N_3$ (III)

in which

A has the meaning given above

are reacted with lithium bromide/$(C_4H_9)_3P(O)$ and epoxides of the general formula (IV)

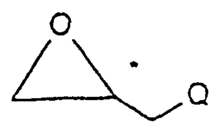

(IV)

in which

Q represents $C_1$-$C_6$-acyloxy

in inert solvents, optionally in the presence of a base,
and, in the case $R^1$ = OH, the hydroxyl function is liberated by a typical ester hydrolysis or by a typical transesterification,
or

[B] compounds of the general formula (V)

$A-NH-CO_2-X$ (V)

in which

A has the meaning given above
and

X represents a typical protecting group, preferably benzyl,

are reacted with epoxides of the general formula (IV), in inert solvents and in the presence of a base, for example lithium alkyls or lithium N-alkyl- or lithium N-silylalkylamides, preferably n-butyllithium,
or

[C] in the case $R^1$ = OH, compounds of the general formula (III) are first of all converted by elimination of nitrogen in alcohols into the compounds of the general formula (Va)

$$A\text{-}NH\text{-}CO_2\text{-}Y \qquad (VA)$$

in which

A has the meaning given above
and

Y represents straight-chain or branched $C_2$-$C_6$-alkyl, preferably n-butyl,

and in a second step as described under [A], are reacted in inert solvents and in the presence of a base, preferably lithium N-alkyl- or N-silylalkyl amides or n-butyllithium and epoxides of the general formula (IV),
or

[D] compounds of the general formula (VI)

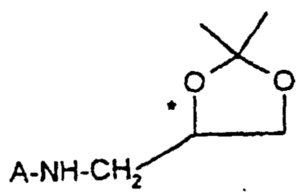
(VI)

in which

A has the meaning given above

are either reacted directly with acids and diethyl carbonate,
or first of all, by reacting the compounds of the general formula (VI) with acids, the compounds of the general formula (VII)

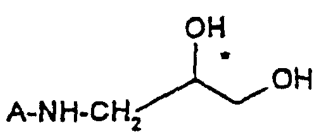
(VII)

in which

A has the meaning given above

are prepared
and are subsequently cyclized in the presence of an auxiliary in inert solvents,
or

[E] compounds of the general formula (Ia)

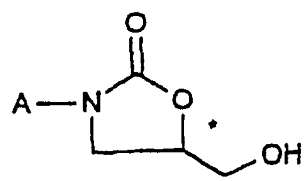

(Ia)

in which

A has the meaning given above

are first of all converted, by reaction with ($C_1$-$C_4$)-alkyl- or phenylsulphonyl chlorides in inert solvents and in the presence of a base, into the corresponding compounds of the general formula (Ib)

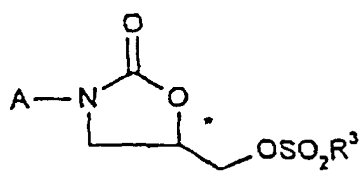

(Ib)

in which

A and $R^3$ have the meaning shown above,

subsequently, using sodium azide in inert solvents, the azides of the general formula (Ic)

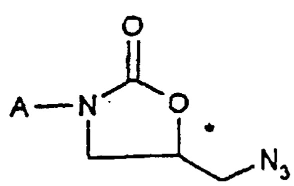

(Ic)

in which

A has the meaning given above

are prepared,
in a further step by reaction with ($C_1$-$C_4$-O)$_3$-P or $PPh_3$, preferably $(CH_3O)_3P$, in inert solvents and with acids, are converted into the amines of the general formula (Id)

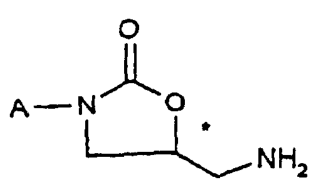

(Id)

in which

A has the meaning given above,

and, by reaction with acetic anhydride or other acylating agents of the general formula (VIII)

$$R^{36}\text{-CO-}R^{6} \qquad \text{(VIII)}$$

in which

$R^6$ has the meaning given above and

$R^{36}$ represents halogen, preferably represents chlorine, or represents the radical $-OCOR^6$

in inert solvents the compounds of the general formula (Ie)

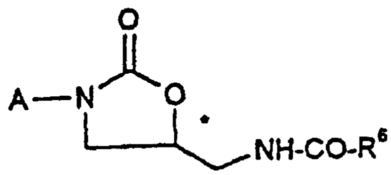

(Ie)

in which

A and $R^6$ have the meaning given above

are prepared,
or

[F]

where A =

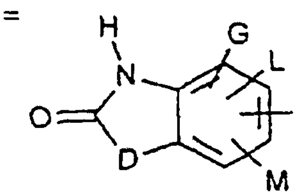

or

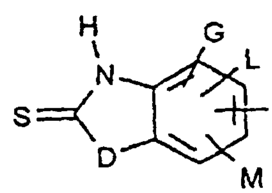

compounds of the general formula (IX)

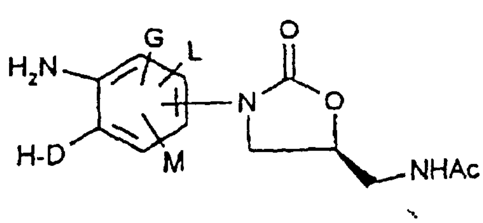

(IX)

in which

G, L, M and D have the meaning given above

are cyclized either using carbonyldiimidazole or thiocarbonyldiimidazole in dimethylformamide or by reaction with $KS\text{-}CO_2\text{-}C_2H_5/CH_3OH$ and subsequent addition of water, where A =

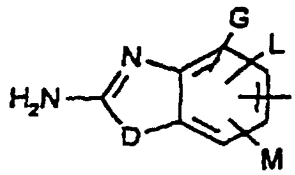

the compounds of the general formula (IX) are reacted with $BrCN/H_2O/CH_3OH$, or

[G) where $R^{12} \neq H$, starting from the compounds where $R^1 = NH\text{-}COCH_3$, an acylation or alkylation with double bond displacement is carried out, or compounds of the general formula (I) having the radical

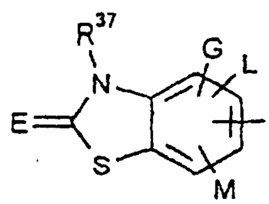

in which

$R^{37}$ denotes $C_1$-$C_{10}$-alkyl, preferably $C_1$-$C_3$-alkyl,

and E = O, compounds of the general formula (X)

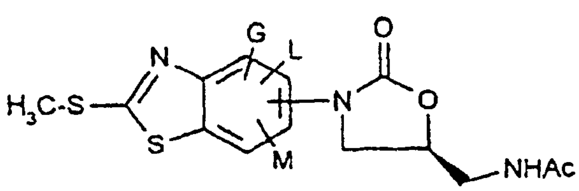

(X)

in which

G, L and M have the meaning given above,

are first of all converted, by reaction with $C_1$-$C_{10}$-alkyl halides, preferably $C_1$-$C_3$-alkyl iodides, in inert solvents into the salts of the compounds of the general formula (XI)

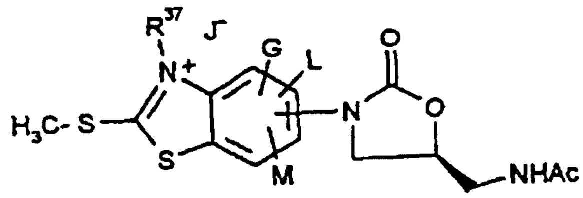

(XI)

in which

| | |
|---|---|
| $R^{37}$ | represents $C_1$-$C_{10}$-alkyl, preferably represents $C_1$-$C_3$-alkyl, and |
| G, L and M | have the meaning given above, |

and in a last step are reacted with methanol,
and where E = S compounds of the general formula (XI) are subjected to thermolysis,
and, in the case of the S-oxides, oxidation is carried out by a customary method,
and, if desired, further substituents or functional groups which are already present are introduced and/or derivatized by customary methods, for example alkylation, redox reactions, substitution reactions and/or hydrolyses or introduction and elimination of protecting groups.

**6.** Use of compounds according to Claims 1 to 4 for the preparation of medicaments.

**7.** Medicaments comprising compounds according to Claims 1 to 4.

## Revendications

**1.** Composés de formule générale (I)

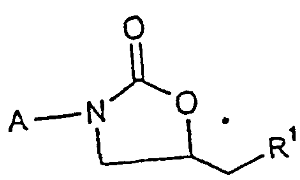

(I)

dans laquelle

$R^1$ est un groupe azido, hydroxy ou un groupe de formule $-OR^2$, $-O-SO_2R^3$ ou $-NR^4R^5$,
où

| | |
|---|---|
| $R^2$ | représente un groupe acyle linéaire ou ramifié ayant jusqu'à 8 atomes de carbone ou un groupe protégeant la fonction hydroxy, |
| $R^3$ | est un groupe alkyle linéaire ou ramifié ayant jusqu'à 4 atomes de carbone ou un groupe phényle qui est substitué, le cas échéant par un radical alkyle linéaire ou ramifié ayant jusqu'à 4 atomes de carbone, |
| $R^4$ et $R^5$ | sont identiques ou différents et représentent un groupe cycloalkyle ayant 3 à 6 atomes de carbone, l'hydrogène, un groupe phényle ou un groupe alkyle ou alkoxy linéaire ou ramifié ayant chacun jusqu'à 8 atomes de carbone ou un groupe protégeant la fonction amino, ou bien |
| $R^4$ et $R^5$ | représentent un groupe de formule $-CO-R^6$, $P(O)(OR^7)(OR^8)$ ou $-SO_2-R^9$, où |
| $R^6$ | est un groupe cycloalkyle ou un groupe cycloalkyle à substituant halogéno ayant chacun jusqu'à |

3 à 6 atomes de carbone, un groupe trifluorométhyle, un groupe alkoxy linéaire ou ramifié ayant jusqu'à 8 atomes de carbone, un groupe phényle, benzyloxy ou l'hydrogène, ou bien

$R^6$ est un groupe alkyle ou un groupe alcényle linéaire ou ramifié dont chacun a jusqu'à 8 atomes de carbone et qui sont éventuellement substitués par un radical cyano, halogéno ou trifluorométhyle,

ou bien

un groupe thioalkyle ou acyle linéaire ou ramifié ayant chacun jusqu'à 6 atomes de carbone,

ou bien

un groupe de formule $-NR^{10}R^{11}$,

dans laquelle

$R^{10}$ et $R^{11}$ sont identiques ou différents et représentent l'hydrogène, un groupe phényle ou un groupe alkyle linéaire ou ramifié ayant jusqu'à 6 atomes de carbone,

ou bien

$R^6$ est un hétérocycle aromatique pentagonal ayant jusqu'à 3 hétéroatomes de la série S, N et/ou O, qui est substitué, le cas échéant par un radical alkyle linéaire ou ramifié ayant jusqu'à 3 atomes de carbone,

$R^7$ et $R^8$ sont identiques ou différents et représentent l'hydrogène ou un groupe alkyle linéaire ou ramifié ayant jusqu'à 4 atomes de carbone,

$R^9$ est un groupe alkyle linéaire ou ramifié ayant jusqu'à 4 atomes de carbone ou un groupe phényle,

A représente un reste de formule

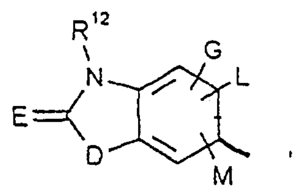

dans laquelle

G, L et M sont identiques ou différents et représentent l'hydrogène, un groupe carboxy, un halogène, un groupe cyano, formyle, trifluorométhyle, nitro, un groupe alkyle linéaire ou ramifié ayant jusqu'à 6 atomes de carbone ou un groupe de formule $-CO-NR^{17}R^{18}$,

dans laquelle

$R^{17}$ et $R^{18}$ sont identiques ou différents et représentent l'hydrogène, un groupe alkyle linéaire ou ramifié ayant jusqu'à 4 atomes de carbone, ou un groupe phényle,

$R^{12}$ représente l'hydrogène, un groupe cycloalkylcarbonyle ou un groupe cycloalkyle ayant chacun 3 à 6 atomes de carbone ou un groupe alkoxycarbonyle linéaire ou ramifié ayant jusqu'à 6 atomes de carbone, ou un groupe alkyle ou alcényle linéaire ou ramifié ayant chacun jusqu'à 10 atomes de carbone, qui sont substitués, le cas échéant par un radical cyano, azido, trifluorométhyle, pyridyle, halogéno, hydroxy, carboxyle, un radical alkoxycarbonyle linéaire ou ramifié ayant jusqu'à 6 atomes de carbone, un radical benzyloxycarbonyle, aryle ayant 6 à 10 atomes de carbone, cycloalkyle ayant 3 à 6 atomes de carbone et/ou par un groupe de formule

$$-(CO)_c-NR^{19}R^{20},\ R^{21}-N-SO_2-R^{22},$$

$R^{23}R^{24}$-N-$SO_2$-, $R^{25}$-S(O)$_d$-

ou

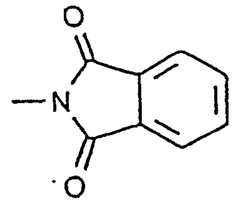

dans laquelle

c représente le nombre 0 ou 1,

$R^{19}$, $R^{20}$ et $R^{21}$ ont la définition indiquée ci-dessus pour $R^{17}$ et $R^{18}$ et y sont identiques ou en sont différents, ou forment conjointement avec l'atome d'azote un hétérocycle saturé pentagonal ou hexagonal ayant, le cas échéant, un autre hétéroatome de la série N, S et/ou 0, qui peut être substitué de son côté, le cas échéant, également sur un autre atome d'azote, par un reste alkyle ou acyle linéaire ou ramifié ayant jusqu'à 3 atomes de carbone,

$R^{23}$ et $R^{24}$ ont la définition indiquée ci-dessus pour $R^{17}$ et $R^{18}$ et y sont identiques ou en sont différents,

d représente le nombre 0, 1 ou 2,

$R^{22}$ et $R^{25}$ sont identiques ou différents et représentent un groupe alkyle linéaire ou ramifié ayant jusqu'à 4 atomes de carbone, un groupe benzyle, phényle ou tolyle,
ou bien

$R^{12}$ représente un reste des formules ou

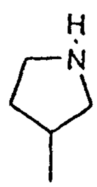 , 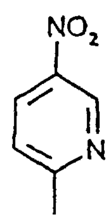 , 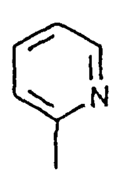 , 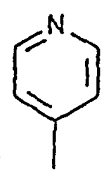 ,

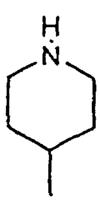 , 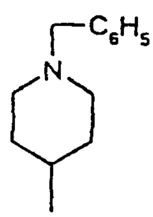 ou 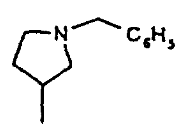 ,

un groupe de formule -$COCCl_3$ ou un groupe acyle linéaire ou ramifié ayant jusqu'à 6 atomes de carbone, qui est substitué, le cas échéant par un reste trifluorométhyle, trichlorométhyle ou par un groupe de formule -$OR^{26}$,
dans laquelle

$R^{26}$ représente l'hydrogène ou un radical alkyle linéaire ou ramifié ayant jusqu'à 6 atomes de carbone, qui est substitué, le cas échéant par un reste aryle ayant jusqu'à 10 atomes de carbone,
ou bien

$R^{12}$ représente un groupe de formule $-(CO)_e-NR^{27}R^{28}$, $-R^{29}-SO_2R^{30}$, $R^{31}R^{32}-N-SO_2-$ ou $R^{33}-S(O)_f$,
où

e a la définition indiquée ci-dessus pour c et y est identique ou en est différent,

$R^{27}$, $R^{28}$ et $R^{29}$ ont chacun la définition indiquée ci-dessus pour $R^{19}$, $R^{20}$ et $R^{21}$ et y sont identiques ou en sont différents,

$R^{31}$ et $R^{32}$ ont la définition indiquée ci-dessus pour $R^{17}$ et $R^{18}$ et y sont identiques ou en sont différents,

f a la définition indiquée ci-dessus pour d et y est identique ou en est différent,

$R^{30}$ et $R^{33}$ ont chacun les définitions indiquées ci-dessus pour $R^{22}$ et $R^{25}$ et y sont identiques ou en sont différents,

D représente un atome d'oxygène ou un atome de soufre,

E représente un atome d'oxygène ou un atome de soufre ou un groupe de formule NH,
ou bien au cas où $R^{12}$ ne représente pas de l'hydrogène, E est un groupe de formule $NR^{34}$ dans laquelle $R^{34}$, à l'exception de l'hydrogène, a la définition indiquée ci-dessus pour $R^{12}$ et y est identique ou en est différent
ou bien

$R^{34}$ est un groupe cyano ou un groupe de formule $-CO_2R^{35}$,
dans laquelle

$R^{35}$ est un groupe benzyle ou un groupe phényle qui sont substitués, le cas échéant par un radical nitro ou halogéno,

sous réserve que $R^1$ ne représente pas un groupe hydroxy lorsque E désigne un groupe NH en même temps que $R^{12}$ représente de l'hydrogène, et leurs formes tautomères, leurs isomères et leurs sels.

**2.** Composés suivant la revendication 1, **caractérisés en ce que**

$R^1$ est un groupe azido, hydroxy ou un groupe de formule $-OR^2$, $-O-SO_2R^3$ ou $-NR^4R^5$,
où

$R^2$ est un groupe acyle linéaire ou ramifié ayant jusqu'à 6 atomes de carbone ou un groupe benzyle,

$R^3$ est un groupe alkyle linéaire ou ramifié ayant jusqu'à 3 atomes de carbone, un groupe phényle ou toluoyle,

$R^4$ et $R^5$ sont identiques ou différents et représentent un groupe cyclopropyle, cyclobutyle, cyclopentyle, cyclohexyle, l'hydrogène, un groupe phényle ou un groupe alkyle ou alkoxy linéaire ou ramifié ayant chacun jusqu'à 6 atomes de carbone, un groupe tertio-butoxycarbonyle ou un groupe benzyloxycarbonyle,
ou bien

$R^4$ et $R^5$ représentent un groupe de benzyle $-CO-R^6$, $P(O)(OR^7)(OR^8)$ ou $-SO_2-R^9$,
où

$R^6$ est un groupe cyclopropyle, cyclopropyle à substituant fluoro, cyclobutyle, cyclopentyle, cyclohexyle, trifluorométhyle ou un groupe alkoxy linéaire ou ramifié ayant jusqu'à 6 atomes de carbone, un groupe phényle, benzyloxy ou l'hydrogène, ou bien

$R^6$ est un groupe alkyle ou un groupe alcényle linéaire ou ramifié ayant chacun jusqu'à 6 atomes de carbone, qui sont éventuellement substitués par un radical cyano, fluoro, chloro, bromo ou trifluorométhyle,
ou bien
un groupe thioalkyle ou acyle linéaire ou ramifié ayant chacun jusqu'à 5 atomes de carbone,
ou bien
un groupe de formule $-NR^{10}R^{11}$,
dans laquelle

$R^{10}$ et $R^{11}$ sont identiques ou différents et représentent l'hydrogène, un groupe phényle ou un groupe alkyle linéaire ou ramifié ayant jusqu'à 4 atomes de carbone,

ou bien

$R^6$ est un groupe isoxazolyle, furyle, thiényle, pyrryle, oxazolyle ou imidazolyle, pouvant éventuellement porter un substituant méthyle,

$R^7$ et $R^8$ sont identiques ou différents et représentent l'hydrogène ou un groupe alkyle linéaire ou ramifié ayant jusqu'à 3 atomes de carbone,

$R^9$ est un groupe alkyle linéaire ou ramifié ayant jusqu'à 3 atomes de carbone ou un groupe phényle,

A représente un reste de formule

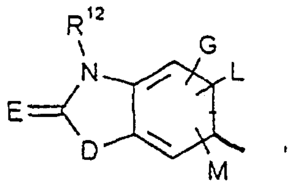

dans laquelle

G, L et M sont identiques ou différents et représentent l'hydrogène, un groupe carboxy, lé fluor, le chlore, le brome, l'iode, un groupe cyano, trifluorométhyle, formyle, nitro ou un groupe alkyle linéaire ou ramifié ayant jusqu'à 4 atomes de carbone ou un groupe de formule $-CO-NR^{17}R^{18}$,

où

$R^{17}$ et $R^{18}$ sont identiques ou différents et représentent l'hydrogène, un groupe alkyle linéaire ou ramifié ayant jusqu'à 3 atomes de carbone ou un groupe phényle,

$R^{12}$ représente l'hydrogène, un groupe cyclopropylcarbonyle, cyclopropyle, cyclobutyle, cyclopentyle, cyclohexyle, ou un groupe alkoxycarbonyle linéaire ou ramifié ayant jusqu'à 4 atomes de carbone, ou bien un groupe alkyle ou un groupe alcényle linéaire ou ramifié ayant chacun jusqu'à 9 atomes de carbone, qui sont substitués, le cas échéant par un radical cyano, azido, trifluorométhyle, pyridyle, fluoro, chloro, bromo, hydroxy, carboxyle, alkoxycarbonyle linéaire ou ramifié ayant jusqu'à 5 atomes de carbone, phényle, naphtyle, benzyloxycarbonyle, cyclopropyle, cyclopentyle, cyclohexyle et/ou par un groupe de formule

$$(CO)_c\text{-}NR^{19}R^{20},\ R^{21}\text{-}N\text{-}SO_2\text{-}R^{22},\ R^{23}R^{24}\text{-}N\text{-}SO_2\text{-},\ R^{25}\text{-}S(O)_d\text{-}$$

ou

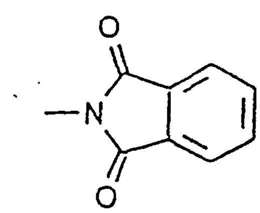

dans laquelle

c est le nombre 0 ou 1,

$R^{19}$, $R^{20}$ et $R^{21}$ ont la définition indiquée ci-dessus pour $R^{17}$ et

$R^{18}$ et y sont identiques ou en sont différents, ou forment conjointement avec l'atome d'azote un noyau morpholinyle, pyrrolidinyle, pipérazinyle ou pipéridyle, ces noyaux étant éventuellement substitués, même par l'intermédiaire de la fonction N libre, par un reste méthyle, éthyle ou acétyle,

$R^{23}$ et $R^{24}$ ont la définition indiquée ci-dessus pour $R^{17}$ et $R^{18}$ et y sont identiques ou en sont différents,

d représente le nombre 0, 1 ou 2,

$R^{22}$ et $R^{25}$ sont identiques ou différents et représentent un groupe alkyle linéaire ou ramifié ayant jusqu'à 3 atomes de carbone, un groupe benzyle, phényle ou tolyle,

ou bien

$R^{12}$ est un reste des formules

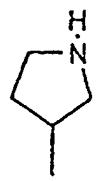
,
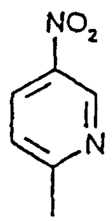
,
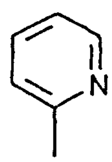
,
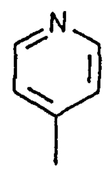
,

ou

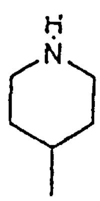
,
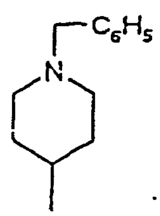

ou

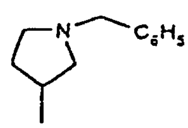

un groupe de formule $-COCCl_3$ ou un groupe acyle linéaire ou ramifié ayant jusqu'à 5 atomes de carbone, qui est substitué, le cas échéant par un reste trifluorométhyle, trichlorométhyle ou un groupe de formule $-OR^{26}$,
dans laquelle

$R^{26}$ représente l'hydrogène ou un radical alkyle linéaire ou ramifié ayant jusqu'à 5 atomes de carbone, qui est substitué, le cas échéant par un reste phényle ou naphtyle,
ou bien

$R^{12}$ est un groupe de formule - $(CO)_e$-$NR^{27}R^{28}$, -$R^{29}$-$SO_2R^{30}$, $R^{31}R^{32}$-N-$SO_2$- ou $R^{33}$-S $(O)_f$,
dans laquelle

e a la définition indiquée ci-dessus pour c et y est identique ou en est différent, y est identique ou en est different,

$R^{27}$, $R^{28}$ et $R^{29}$ ont les définitions données ci-dessus pour

chacun $R^{19}$, $R^{20}$ et $R^{21}$ et y sont identiques ou en sont différents,

$R^{31}$ et $R^{32}$ ont la définition indiquée ci-dessus pour $R^{17}$ et $R^{18}$ et y sont identiques ou en sont différents,

f a la définition indiquée ci-dessus pour d et y est identique ou en est différent,

$R^{30}$ et $R^{33}$ ont chacun les définitions indiquées ci-dessus pour $R^{22}$ et $R^{25}$ et y sont identiques ou en sont différents,

D représente un atome d'oxygène ou de soufre,

E représente un atome d'oxygène ou de soufre ou un groupe de formule NH,

ou bien au cas où $R^{12}$ ne représente pas l'hydrogène, E est un groupe de formule $NR^{34}$ dans laquelle $R^{34}$ a, excepté l'hydrogène, la définition indiquée ci-dessus pour $R^{12}$ et y est identique ou en est différent, ou bien

$R^{34}$ est un groupe cyano ou un groupe de formule $-CO_2R^{35}$, dans laquelle

$R^{35}$ désigne un groupe benzyle ou phényle, portant éventuellement un substituant nitro, fluoro, chloro ou bromo,

sous réserve que $R^1$ ne représente pas un groupe hydroxy lorsque E désigne un groupe NH en même temps que $R^{12}$ représente l'hydrogène, et leurs formes tautomères, leurs isomères et leurs sels.

3. Composés suivant la revendication 1, **caractérisés en ce que**

$R^1$ est un groupe azido, hydroxy ou un groupe de formule $-OR^2$, $-O-SO_2R^3$ ou $-NR^4R^5$, où

$R^2$ est un groupe acyle linéaire ou ramifié ayant jusqu'à 5 atomes de carbone ou un groupe benzyle,

$R^3$ est un groupe méthyle, éthyle, phényle ou toluoyle,

$R^4$ et $R^5$ sont identiques ou différents et représentent un groupe cyclopropyle, cyclobutyle, cyclopentyle, cyclohexyle, l'hydrogène, un groupe phényle ou un groupe alkyle ou alkoxy linéaire ou ramifié ayant chacun jusqu'à 5 atomes de carbone, un groupe tertio-butoxycarbonyle ou benzyloxycarbonyle, ou bien

$R^4$ ou $R^5$ représentent un groupe de formule $-CO-R^6$, P (O) ($OR^7$) ($OR^8$) ou $-SO_2-R^9$, où

$R^6$ est un groupe cyclopropyle, cyclopropyle à substituant fluoro, cyclobutyle, cyclopentyle, cyclohexyle, trifluorométhyle ou un groupe alkoxy linéaire ou ramifié ayant jusqu'à 5 atomes de carbone, un groupe phényle, benzyloxy ou l'hydrogène,

$R^6$ est un groupe alkyle ou un groupe alcényle linéaire ou ramifié ayant chacun jusqu'à 5 atomes de carbone, portant éventuellement un substituant cyano, fluoro, chloro, bromo ou trifluorométhyle,
ou bien
un groupe thioalkyle ou acyle linéaire ou ramifié ayant chacun jusqu'à 4 atomes de carbone, ou bien
un groupe de formule $-NR^{10}R^{11}$, dans laquelle

$R^{10}$ et $R^{11}$ sont identiques ou différents et représentent l'hydrogène, un groupe phényle ou un groupe alkyle linéaire ou ramifié ayant jusqu'à 3 atomes de carbone, ou bien

$R^6$ est un groupe isoxazolyle, furyle, oxazolyle ou imidazolyle, portant éventuellement un substituant méthyle,

$R^7$ et $R^8$ sont identiques ou différents et représentent l'hydrogène, un groupe méthyle ou éthyle,

$R^9$ est un groupe méthyle ou phényle,

A représente un reste de formule

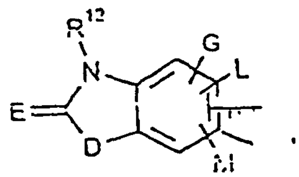

dans laquelle

G, L et M sont identiques ou différents et représentent l'hydrogène, un groupe carboxy, le fluor, le chlore, le brome, l'iode, un groupe cyano, formyle, nitro, un groupe alkyle linéaire ou ramifié ayant jusqu'à 3 atomes de carbone ou un groupe de formule $-CO-NH_2$,

$R^{12}$ représente l'hydrogène, un groupe cyclopropylcarbonyle, cyclopropyle, cyclobutyle, cyclopentyle, cyclohexyle, ou bien
un groupe alkoxycarbonyle linéaire ou ramifié ayant jusqu'à 3 atomes de carbone, ou bien
un groupe alkyle ou un groupe alcényle linéaire ou ramifié ayant chacun jusqu'à 8 atomes de carbone, portant éventuellement un substituant cyano, azido, trifluorométhyle, pyridyle, fluoro, chloro, bromo, hydroxy, carboxyle, alkoxycarbonyle linéaire ou ramifié ayant jusqu'à 4 atomes de carbone, phényle, benzyloxycarbonyle, cyclopropyle, cyclopentyle, cyclohexyle et/ou un groupe de formule $-(CO)_c-NR^{19}R^{20}$, $R^{21}-N-SO_2-R^{22}$, $R^{23}R^{24}-N-SO_2-$, $R^{25}-S(O)_d-$ ou

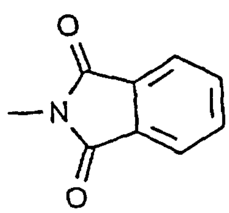

dans laquelle

c représente le nombre 0 ou 1,

$R^{19}$, $R^{20}$, $R^{21}$, $R^{23}$ et $R^{24}$ sont identiques ou différents et représentent l'hydrogène, un groupe méthyle ou éthyle,

d est le nombre 0, 1 ou 2,

$R^{22}$ et $R^{25}$ sont identiques ou différents et. représentent un groupe alkyle linéaire ou ramifié ayant jusqu'à 4 atomes de carbone, un groupe benzyle, phényle ou tolyle,
ou bien

$R^{12}$ représente un reste des formules

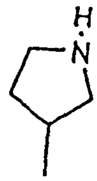 , 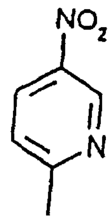 , 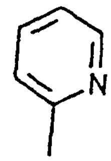 , 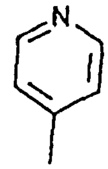 ,

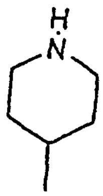 , 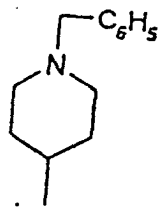 ou 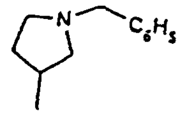

ou bien
un groupe de formule $-COCCl_3$ ou un groupe acyle linéaire ou ramifié ayant jusqu'à 4 atomes de carbone, qui est substitué, le cas échéant par un radical trifluorométhyle, trichlorométhyle, un groupe de formule $-OR^{26}$,
où

$R^{26}$ représente l'hydrogène ou un radical alkyle linéaire ou ramifié ayant jusqu'à 4 atomes de carbone, qui est substitué, le cas échéant par un radical phényle,
ou bien

$R^{12}$ est un groupe de formule - $(CO)_e$-$NR^{27}R^{28}$, ou $R^{33}$-$S(O)_f$, dans laquelle

| | |
|---|---|
| e | représente le nombre 1, |
| $R^{27}$, $R^{28}$ et $R^{29}$ s | ont identiques ou différents et représentent l'hydrogène, un groupe méthyle ou éthyle, |
| f | a la définition indiquée ci-dessus pour d et y est identique ou en est différent, |
| $R^{33}$ | est un groupe méthyle, phényle, tolyle ou benzyle, |

| | |
|---|---|
| D | représente un atome d'oxygène ou de soufre, |
| E | représente un atome d'oxygène ou de soufre ou un groupe de formule NH, |

ou bien au cas où $R^{12}$ ne représente pas l'hydrogène, E désigne un groupe de formule $NR^{34}$ dans laquelle, $R^{34}$, à l'exception de l'hydrogène, a la définition indiquée ci-dessus pour $R^{12}$ et y est identique ou en est différent, ou bien

$R^{34}$ est un groupe cyano ou un groupe de formule $-CO_2R^{35}$, dans laquelle

$R^{35}$ désigne un groupe benzyle ou phényle, portant éventuellement un substituant nitro,

sous réserve que $R^1$ ne représente pas un groupe hydroxy lorsque E désigne un groupe NH en même temps que $R^{12}$ représente l'hydrogène, et leurs formes tautomères, leurs isomères et leurs sels.

**4.** Composés suivant la revendication 1, **caractérisés en ce que**

G, L et M représentent l'hydrogène.

**5.** Procédé de production de composés suivant les revendications 1 à 4, **caractérisé en ce que**

[A] on fait réagir des composés de formule générale (II) ou (III)

$$A\text{-}N{=}C{=}O \qquad (II)$$

ou

$$A\text{-}CO\text{-}N_3 \qquad (III)$$

dans lesquelles

A a les définitions indiquées ci-dessus,

avec le système bromure de lithium/$(C_4H_9)_3P(O)$ et des époxydes de formule générale (IV)

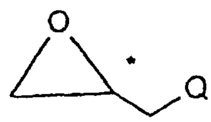

(IV)

dans laquelle

Q est un groupe acyloxy en $C_1$ à $C_6$,

dans des solvants inertes, éventuellement en présence d'une base,

et au cas où $R^1$ est un groupe OH, on libère la fonction hydroxy par une saponification typique de l'ester ou par une transestérification typique,

ou bien

[B] on fait réagir des composés de formule générale (V)

$$A\text{-}NH\text{-}CO_2\text{-}X \qquad (V)$$

dans laquelle

A a la définition indiquée ci-dessus
et
X est un groupe protecteur typique, de préférence le groupe benzyle,

dans des solvants inertes et en présence d'une base, par exemple de lithium-alkyles ou de N-alkyl- ou -N-silylalkylamidures de lithium, de préférence le N-butyl-lithium, avec des époxydes de formule générale (IV),

ou bien

[C] au cas où $R^1$ représente un groupe OH, on transforme tout d'abord des composés de formule générale (III) par élimination d'azote dans des alcools en les composés de formule générale (Va)

$$A\text{-}NH\text{-}CO_2\text{-}Y \qquad (Va)$$

dans laquelle

A a la définition indiquée ci-dessus et
et
Y est un groupe alkyle linéaire ou ramifié en $C_2$ à $C_6$, de préférence le groupe n-butyle,

et dans une seconde étape, on conduit une réaction comme en [A] dans des solvants inertes et en présence d'une base, de préférence des N-alkyl- ou -N-silylalkylamidures de lithium ou le n-butyl-lithium et d'époxydes de formule générale (IV),

ou bien

[D] on fait réagir des composés de formule générale (VI)

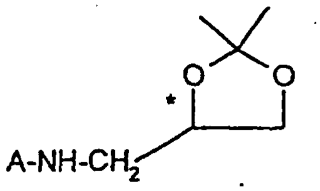
(VI)

dans laquelle

A a la définition indiquée ci-dessus,

directement avec des acides et l'ester diéthylique de l'acide carbonique,

ou bien on prépare tout d'abord par réaction des composés de formule générale (VI) avec des acides, les composés de formule générale (VII)

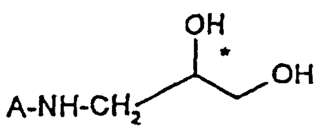
(VII)

dans laquelle

A a la définition indiquée ci-dessus,

puis on effectue une cyclisation en présence d'une substance auxiliaire dans des solvants inertes,

ou bien

[E] on transforme tout d'abord des composés de formule générale (Ia)

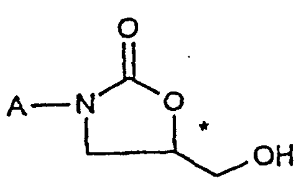
(Ia)

dans laquelle

A a la définition indiquée ci-dessus,

par réaction avec des chlorures d'acides (alkyle en $C_1$ à $C_4$) - ou phénylsulfoniques dans des solvants inertes et en présence d'une base en les composés correspondants de formule générale (Ib)

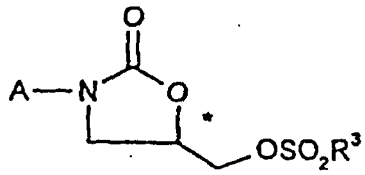

(Ib)

dans laquelle

A et $R^3$ ont la définition indiquée ci-dessus,

puis on prépare avec l'azoture de sodium dans des solvants inertes les azides de formule générale (Ic)

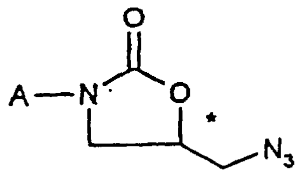

(Ic)

dans laquelle

A a la définition indiquée ci-dessus,

on les transforme dans une autre étape par réaction avec $(C_1\text{-}C_4\text{-}O)_3\text{-}P$ ou $PPh_3$, de préférence $(CH_3O)_3P$ dans des solvants inertes et avec des acides, en les amines de formule générale (Id)

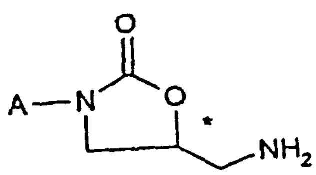

(Id)

dans laquelle

A a la définition indiquée ci-dessus,

et on prépare par réaction avec l'acétanhydride ou d'autres agents d'acylation de formule générale (VIII)

$$R^{36}\text{-CO-}R^6 \qquad \text{(VIII)}$$

dans laquelle

$R^6$ a la définition indiquée ci-dessus
et
$R^{36}$ représente un halogène, de préférence le chlore ou le reste $-OCOR^6$,

dans des solvants inertes, les composés de formule générale (Ie)

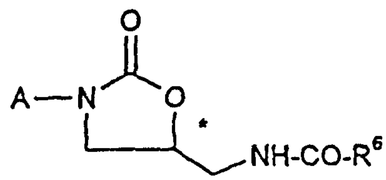

(Ie)

dans laquelle

A et $R^6$ ont la définition indiquée ci-dessus,

ou bien
[F] au cas où A représente

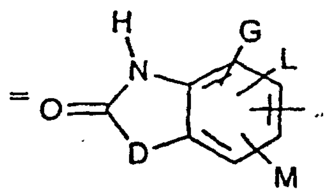

ou

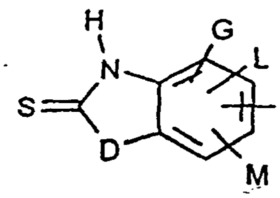

on cyclise des composés de formule générale (IX)

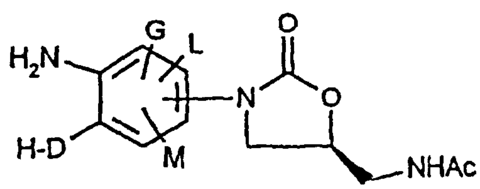

(IX)

dans laquelle

G, L, M et D ont la définition indiquée ci-dessus,

soit avec le carbonyldiimidazole ou le thiocarbonyldiimidazole dans le diméthylformamide ou par réaction avec $KS\text{-}CO_2\text{-}C_2H_5/CH_3OH$, puis addition d'eau,
au cas où A représente

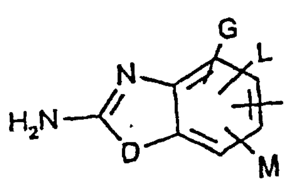

on fait réagir les composés de formule générale (IX) avec $BrCN/H_2O/CH_3OH$,
ou bien
[G] au cas où $R^{12}$ ne représente pas l'hydrogène, on conduit à partir des composés dans lesquels $R^1$ représente $NH\text{-}COCH_3$ une acylation ou une alkylation avec déplacement de la double liaison, ou bien
on transforme des composés de formule générale (I) portant le reste

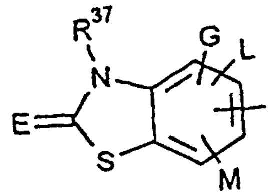

où

$R^{37}$ est un groupe alkyle en $C_1$ à $C_{10}$, de préférence un groupe alkyle en $C_1$ à $C_3$ et
E représente O,
des composés de formule générale (X)

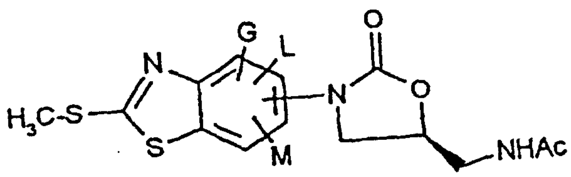

(X)

dans laquelle

G, L et M ont la définition indiquée ci-dessus,

tout d'abord par réaction avec des halogénures d'alkyle en $C_1$ à $C_{10}$, de préférence des iodures d'alkyle en $C_1$ à $C_3$, dans des solvants inertes en les sels des composés de formule générale (XI)

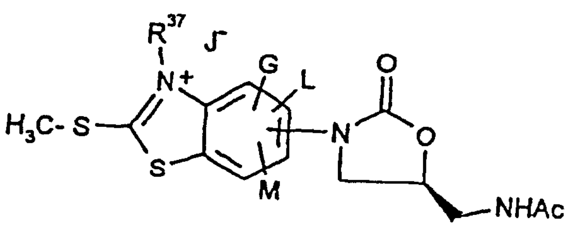

(XI)

dans laquelle

$R^{37}$ est un groupe alkyle en $C_1$ à $C_{10}$, de préférence un groupe alkyle en $C_1$ à $C_3$, et
G, L et M ont la définition indiquée ci-dessus,

et dans une dernière étape, on conduit une réaction avec le méthanol,
et au cas où E représente S, on soumet des composés de formule générale (XI) à une thermolyse,
et dans le cas des S-oxydes, on effectue une oxydation selon un mode opératoire classique,
et le cas échéant, on introduit ou on transforme en dérivés d'autres substituants ou des groupes fonctionnels déjà présents, selon des modes opératoires classiques, par exemple alkylation, réactions d'oxydo-réduction, réactions de substitution et/ou saponifications, ou bien introduction et élimination de groupes protecteurs.

**6.** Utilisation des composés suivant les revendications 1 à 4 pour la préparation de médicaments.

**7.** Médicaments contenant des composés suivant les revendications 1 à 4.